# EUROPEAN PATENT APPLICATION

(11) **EP 4 803 094 A2**
(43) Date of publication of application: **09.09.2026**
(21) Application number: 26193474.9
(22) Date of filing: 07.06.2024
(51) Int. Cl.: A61K 38/00

(54) **GLP-1/GIP DUAL, GLP-1/GCG DUAL AND GLP-1/GIP/GCG TRIPLE RECEPTOR AGONISTS**

(30) Priority: 09.06.2023 IN 202321039646
(62) Document number(s) of the earlier application(s) in accordance with Art. 76 EPC: 24734138.1 / 4 724 471
(71) Applicant: Sun Pharmaceutical Industries Limited, Mumbai Maharashtra 400 063 (IN)
(72) Inventor: THENNATI, Rajamannar, 390020 Vadodara (IN); BURADE, Vinod Sampatrao, 390020 Vadodara (IN); NATARAJAN, Muthukumaran, Mandi (IN); JOSHI, Dhiren Rameshchandra, 390024 Vadodara (IN); GANDHI, Manish Harendraprasad, 390019 Vadodara (IN); JIVANI, Chandulal Thakarshiibhai, 390011 Vadodara (IN); TIWARI, Abhishek, 324005 Kota (IN); SONI, Krunal Harishbhai, 390019 Vadodara (IN); MARU, Alpeshbhai Balabhai, 364505 Bhavnagar (IN); DIXIT, Pankaj Vinodrao, 452012 Indore (IN); PATELIYA, Bharatbhai Balabhai, 391410 Vadodara (IN); NAGARAJA, Ravishankara Madavati, 390012 Vadodara (IN)
(74) Representative: Nederlandsch Octrooibureau

(57) **Abstract**

The present invention relates to GLP-1/GIP/GCG triple receptor agonists and use thereof in the treatment or prevention of Type 2 diabetes mellitus (T2DM), hyperlipidemia/dyslipidemia, metabolic syndromes, metabolic dysfunction-associated steatotic liver disease (MASLD), metabolic dysfunction-associated steatohepatitis (MASH), neurodegenerative disorders, fibro-EP1sis, cardiovascular risks, and/or obesity.

## Description

### CROSS-REFERENCE TO RELATED APPLICATIONS

This application claims the benefit of priority of Indian Application No. 202321039646, filed June 9, 2023, which is incorporated herein by reference in its entirety.

### REFERENCE TO AN ELECTRONIC SEQUENCE LISTING

This application contains a Sequence Listing which has been submitted electronically and is hereby incorporated by reference in its entirety. The Sequence Listing was created on June 7, 2024, is named "24-0750-WO_Sequence-Listing.xml" and is 143,360 bytes in size.

### FIELD

The present disclosure relates to GLP-1/GLP dual, GLP-1/GCG dual and GLP-1/GIP/GCG triple receptor agonists. In particular, the present disclosure relates to GLP-1/GIP/GCG triple receptor agonists comprising incretin analog polypeptides. The polypeptides as described herein have structural features that provide balanced activity and an extended duration of action at each of these receptors. The polypeptides according to the present disclosure may be useful for treating Type 2 diabetes mellitus (T2DM), hyperlipidemia/dyslipidemia, metabolic syndromes, metabolic dysfunction-associated steatotic liver disease (MASLD), metabolic dysfunction-associated steatohepatitis (MASH), neurodegenerative disorders, fibrosis, obesity, and reducing cardiovascular risks.

### BACKGROUND

The prevalence of diabetes has continued to rise over the past several decades. T2DM is the most prevalent form of diabetes, which is characterized by high blood glucose levels caused by insulin resistance. A person suffering from T2DM is more likely to develop comorbidities such as hyperlipidemia/dyslipidemia, metabolic syndromes, metabolic dysfunction-associated steatotic liver disease (MASLD), metabolic dysfunction-associated steatohepatitis (MASH), neurodegenerative disorders, fibrosis, cardiovascular risks and/or obesity.

The current therapies for T2DM include diet and exercise as well as treatment with oral medications and injectable glucose-lowering drugs including incretin-based therapies, such as GLP-1 mono receptor agonists and/or GLP-1/GIP dual receptor agonists. As the emerging approaches, new therapies are being studied wherein the compounds are not only active at GLP-1 mono or GLP-1/GIP dual receptors, but also at GCG receptors. Certain compounds have been described as having GLP-1/GCG dual and/or GLP-1/GIP/GCG triple agonist activity.

For example, Cotadutide, MK-1462 and Mazdutide are peptides which act as GLP-1/GCG dual receptor agonists. Similarly, Retatrutide (SEQ ID NO: 7) is another clinical candidate, which acts as a GLP-1/GIP/GCG triple receptor agonist. WIPO publication numbers WO2019/193576, WO2006/097537 and WO1998/008871 disclose GLP-1 receptor agonist compounds. WIPO publication numbers WO2022/079639, WO2021/260530, WO2017/74714A1, WO2020/23386, WO2020/023388, WO2015/067715, WO2016/111971, WO2014/192284, WO2011/119657 and WO2013/164483 disclose GLP-1/GIP dual receptor agonist compounds. WIPO publication numbers WO2011/075393, WO2012/177444, WO2014/091316 and WO2017/153575 disclose GLP-1/GCG dual receptor agonist compounds. WIPO publication numbers WO2015/067716, WO2016/198624, WO2014/049610 and WO2017/116204 disclose GLP-1/GIP/GCG triple receptor agonist compounds.

Recent studies on GLP-1/GCG dual and/or GLP-1/GIP/GCG triple receptor agonists have also highlighted the importance of understanding the contribution of individual hormone action and divergent effects by varying the GLP-1: GCG activity and the ratios in GLP-1/GCG dual receptor agonists and GLP-1/GIP/GCG triple receptor agonists. Hope et al. Front. Endocrinol., 08 September 2021, Vol 12-2021*.*

Glucagon Receptor (GCGR) agonism, besides being diabetogenic, is known to increase heart rate and contractility, which might lead to adverse cardiovascular outcomes. Further chronic excess of glucagon also leads to catabolism of amino acids and proteins, which lead to a loss of lean body mass.

Retatrutide, for example, despite showing increased weight loss in the treatment of obesity, is associated with several side effects such as an increase in heart rate. These adverse effects may be attributed to the GCGR agonism component of drug action. Current research in Diabetes & Obesity Journal, July 26, 2023*.*

Therefore, while the broad metabolic benefits of GLP-1, GIP and GCG receptor agonist compounds have been established in the treatment paradigm, there remains a need for treatments, especially for T2DM and associated comorbidities such as cardiovascular diseases and/or obesity, that are capable of providing effective glucose control with weight loss benefits and reduced adverse effect profile such as an increase in heart rate, cardiac arrythmias and a loss of lean mass. There is also a need for therapeutic agents available for use with sufficiently extended duration of action to allow for dosing as infrequently as twice-weekly or once a week.

It has been surprisingly found that the modification of fatty acid side chains and/or the substitution of branched side chains containing amino acids with straight side chain amino acids in the sequences of GLP-1/GCG dual and/or GLP-1/GIP/GCG triple receptor agonists can lead to substantial GLP-1 dominance, which leads to enhanced weight loss without acute reduction in food consumption along with reduced side effects.

### SUMMARY

In one aspect, the present disclosure relates to a polypeptide or a pharmaceutically acceptable salt thereof comprising an amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 1)
 wherein:
X1 is Y;
X2 is Aib;
X3 is Q or N;
X10 is Y;
X12 is I;
X13 is L or D isomer of an amino acid of the formula 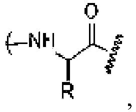 wherein " 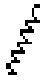 " represents the point of attachment to Leu, and wherein R is selected from C₁-C₆ alkyl, C₃-C₆ cycloalkylmethyl and C₃-C₆ cycloalkyl;
X16 is K;
X17 is I or K;
X18 is A;
X19 is Q;
X20 is, K, Aib, or L or D isomer of an amino acid of the formula 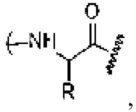 wherein " 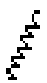 " represents the point of attachment to Leu, and and wherein R is selected from C₁-C₆ alkyl, C₃-C₆ cycloalkylmethyl and C₃-C₆ cycloalkyl;
X21 is A;
X23 is V or I;
X24 is Q or E;
X25 is W or Y;
X27 is I or L;
X28 is A or E;
X29 is G;
X30 is G;
X31 is P;
X32 is S;
X33 is S;
X34 is G;
X35 is A;
X36 is P;
X37 is P;
X38 is P; and
X39 is S;

wherein the acid group of the C-terminal amino acid is a free carboxylic acid group or is amidated at C-terminal primary amide; and
with a proviso that at least one of X17 and X20 is K and that at least one of said K is conjugated to a C₁₆-C₂₂ fatty acid.

In another aspect, the present disclosure relates to a polypeptide or a pharmaceutically acceptable salt thereof comprising an amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 2)
 wherein:
is Y;
X2 is Aib;
X3 is Q or N;
X10 is Y;
X12 is I;
X13 is αMe-L;
X16 is K;
X17 is I or K;
X18 is A;
X19 is Q;
X20 is K, Aib, or L or D isomer of an amino acid of the formula 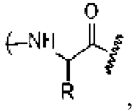 wherein " 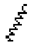 " represents the point of attachment to Leu, and wherein R is selected from C₁-C₆ alkyl, C₃-C₆ cycloalkylmethyl and C₃-C₆ cycloalkyl;
X21 is A;
X23 is V or I;
X24 is Q or E;
X25 is W or Y;
X27 is I or L;
X28 is A, or E;
X29 is G
X30 is G;
X31 is P;
X32 is S;
X33 is S;
X34 is G;
X35 is A;
X36 is P;
X37 is P;
X38 is P; and
X39 is S;

wherein the acid group of the C-terminal amino acid is a free carboxylic acid group or is amidated as C-terminal primary amide; and
with a proviso that at least one of X17 and X20 is K, and further provided that at least one of said K comprises a side chain amino (ε amino) group acylated with a moiety of the formula selected from:
   aminoethoxyethoxyacetic acid-Aib-Glu-C₁₆-C₂₂ fatty acid chain;
   aminoethoxyethoxyacetic acid-C(O)-diaminobutane-Glu-C₁₆-C₂₂ fatty acid chain; Glu-C₁₆-C₂₂ fatty acid chain;
   aminoethoxyethoxyacetic acid- aminoethoxyethoxyacetic acid-Glu-C₁₆-C₂₂ fatty acid chain; and
   aminoethoxyethoxyacetic acid-Glu-C₁₆-C₂₂ fatty acid chain.
   with a proviso that when X20 is Aib, the side chain amino group is not acylated with aminoethoxyethoxyacetic acid-aminoethoxyethoxyacetic acid-Glu-C₁₆-C₂₂ fatty acid chain or aminoethoxyethoxyacetic acid-Glu-C₁₆-C₂₂ fatty acid chain.

In another aspect, the present disclosure relates to a polypeptide or a pharmaceutically acceptable salt thereof comprising an amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 3)
 wherein:
X1 is Y;
X2 is Aib;
X3 is Q or N;
X10 is Y;
X12 is I;
X13 is Aib, Ser(OMe), nor-V, nor-L, or αMe-L;
X16 is K;
X17 is I or K;
X18 is A;
X19 is Q;
X20 is K, Aib, Ser(OMe), nor-V, or nor-L;
X21 is A;
X23 is V or I;
X24 is Q or E;
X25 is W or Y;
X27 is E, I or L;
X28 is A or E;
X29 is G;
X30 is G;
X31 is P;
X32 is S;
X33 is S;
X34 is G;
X35 is A;
X36 is P;
X37 is P;
X38 is P; and
X39 is S;
wherein the acid group of the C-terminal amino acid is a free carboxylic acid group or is amidated as C-terminal primary amide; and
with a proviso that at least one of X17 and X20 is K, and further provided that at least one of said K comprises a side chain amino (ε amino) group acylated with a moiety of the formula selected from:

| | |
|---|---|
| Moiety A | 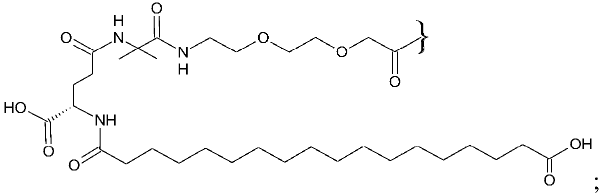 |
| Moiety B | 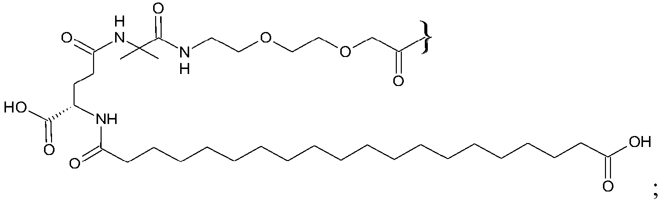 |
| Moiety C | 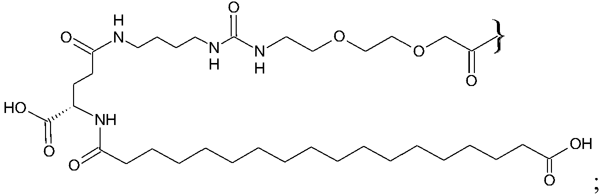 |
| Moiety D | 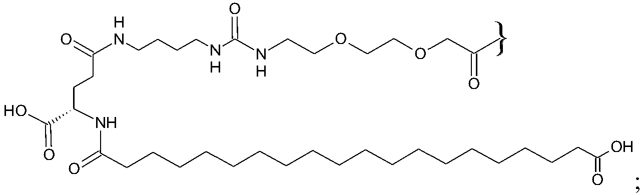 |
| Moiety E | 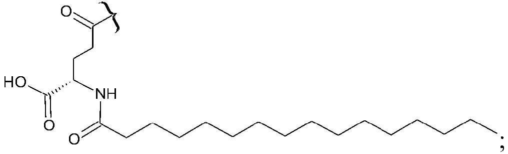 |
| Moiety F | 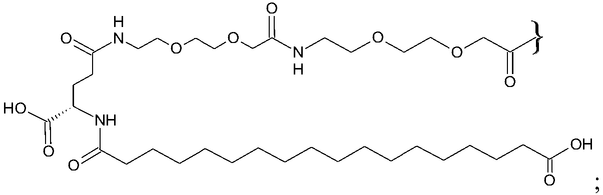 |
| Moiety G | 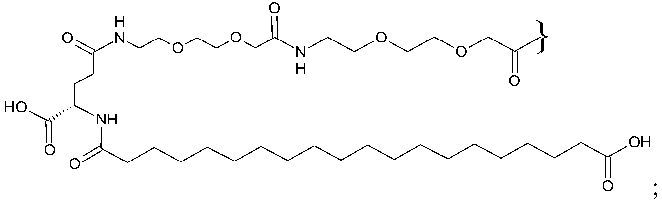 |
| Moiety H | 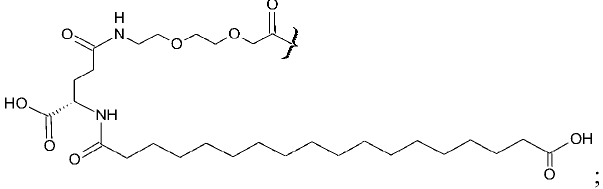 and |
| Moiety I | 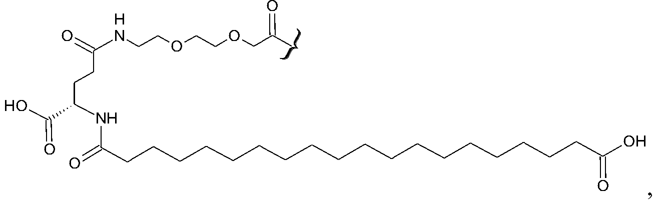 |

wherein the polypeptide is not SEQ ID NO: 7, SEQ ID NO: 23 or SEQ ID NO: 30.

In another aspect, the present disclosure relates to a polypeptide or a pharmaceutically acceptable salt thereof comprising an amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 4)
 wherein:
X1 is Y;
X2 is Aib;
X3 is Q or N;
X10 is Y;
X12 is I;
X13 is nor-V, nor-L or αMe-L;
X16 is K;
X17 is I or K;
X18 is A;
X19 is Q;
X20 is K or Aib;
X21 is A;
X23 is V or I;
X24 is Q or E;
X25 is W or Y;
X27 is I or L;
X28 is A or E;
X29 is G;
X30 is G;
X31 is P;
X32 is S;
X33 is S;
X34 is G;
X35 is A;
each of X36, X37 and X38 is independently P; and
X39 is S;
with a proviso that at least one of X17 and X20 is K, and further provided that said K comprises a side chain amino (ε amino) group acylated with a moiety of the formula selected from:

| | |
|---|---|
| Moiety A | 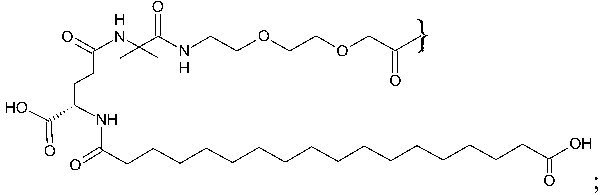 |
| Moiety B | 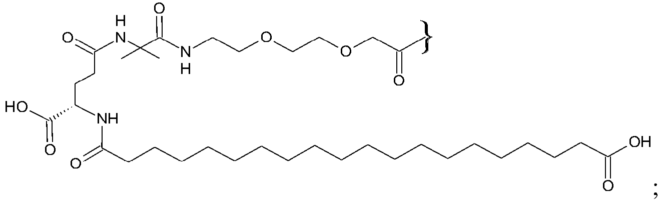 |
| Moiety C | 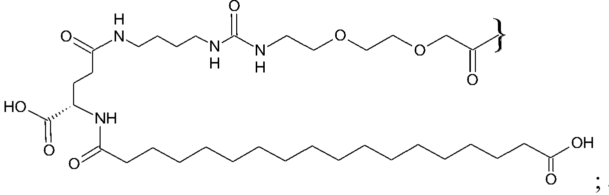 and |
| Moiety D | 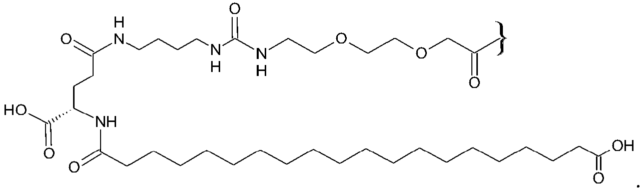 |

In another aspect, the present disclosure relates to a polypeptide or a pharmaceutically acceptable salt thereof comprising an amino acid sequence: 
Y-Aib-X3-G-T-F-T-S-D-Y-S-I-X13-L-D-K-X17-A-Q-X20-A-F-X23-X24-X25-L-X27-
X28-G-G-P-S-S-G-A-P-P-P-S (SEQ ID NO: 5)
 wherein;
X3 is Q or N;
X13 is nor-V, nor-L or αMe-L;
X17 is I or K;
X20 is K or Aib;
X23 is V or I;
X24 is Q or E;
X25 is W or Y;
X27 is I or L; and
X28 is A or E;
wherein the acid group of the C-terminal amino acid is a free carboxylic acid group or is amidated as C-terminal primary amide; and
with a proviso that at least one of X17 and X20 is K, and further provided that said K comprises a side chain amino (ε amino) group acylated with a moiety of the formula selected from:

| | |
|---|---|
| Moiety A | 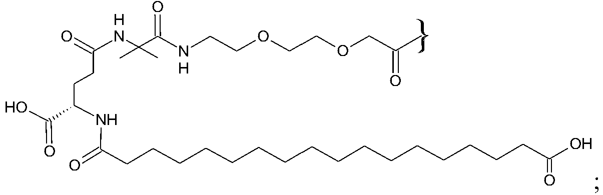 |
| Moiety B | 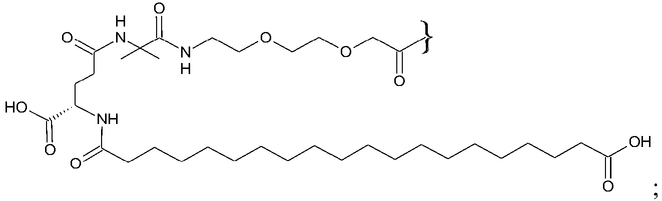 |
| Moiety C | 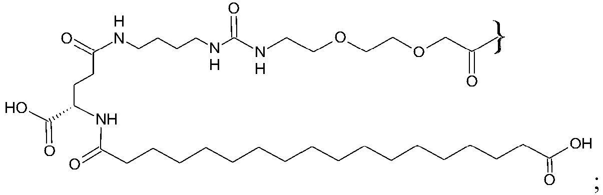 |
| Moiety D | 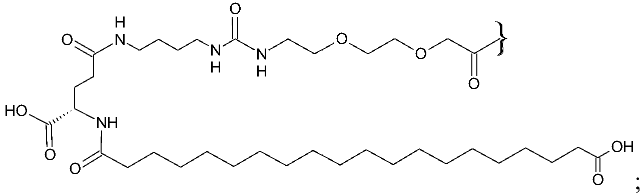 |
| Moiety G | 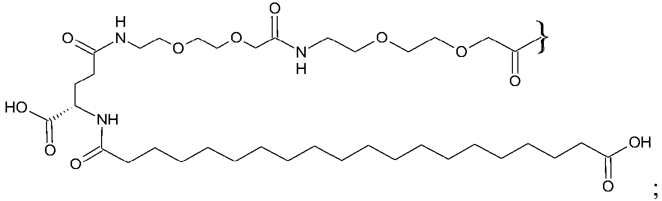 |
| Moiety H | 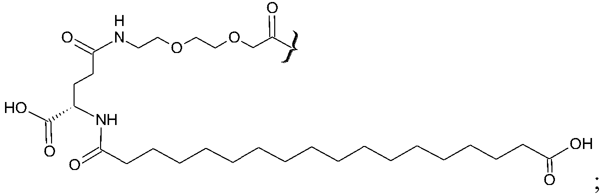 and |
| Moiety I | 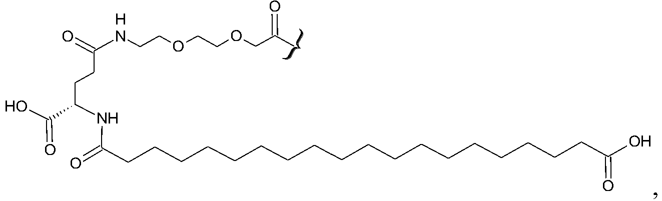 |

wherein the polypeptide is not SEQ ID NO: 7.

In another aspect, the present disclosure relates to a polypeptide or a pharmaceutically acceptable salt thereof comprising an amino acid sequence: 
Y-Aib-X3-G-T-F-T-S-D-Y-S-I-X13-L-D-K-X17-A-Q-X20-A-F-I-E-Y-L-L-E-G-G-P-S-
S-G-A-P-P-P-S (SEQ ID NO: 6)
 wherein:
X3 is Q or N;
X13 is nor-V, nor-L or αMe-L;
X17 is K; and
X20 is Aib, nor-L or nor-V;
wherein the acid group of the C-terminal amino acid is a free carboxylic acid group or is amidated as C-terminal primary amide; and
wherein the side chain amino (ε amino) group of K at position X17 is acylated with a moiety of the formula selected from:

| | |
|---|---|
| Moiety A | 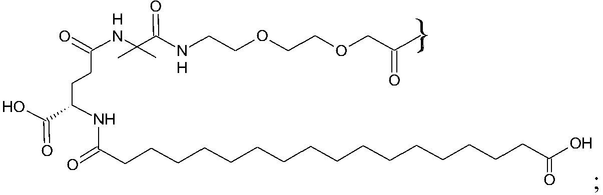 |
| Moiety H | 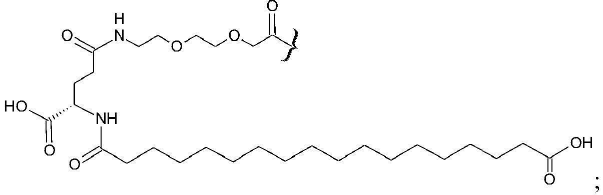 and |
| Moiety I | 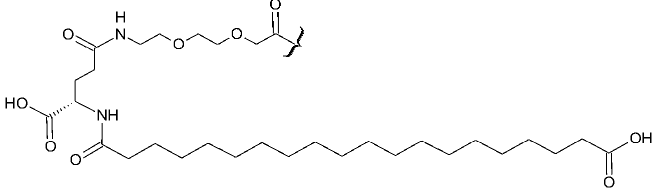 |

wherein said polypeptide is not SEQ ID NO: 7.

In another aspect, the present disclosure relates to an incretin analog polypeptide comprising:
a lysine residue comprising a fatty acid protracting group attached to the lysine ε-nitrogen;
a peptide residue comprising the sequence Gly-Thr-Phe-Thr-Ser-Asp (SEQ ID NO:31) attached indirectly via its carboxy terminus to the lysine residue;
a peptide residue having the sequence Gly-Gly-Pro-Ser-Ser-Gly-Ala-Pro-Pro-Pro-Ser-CONH₂ (SEQ ID NO:32) indirectly attached to the carboxy of the lysine via the amino terminus of the Gly-Gly-Pro-Ser-Ser-Gly-Ala-Pro-Pro-Pro-Ser-CONH₂ (SEQ ID NO:32) residue; and
a norvaline amino acid residue attached indirectly to and between the Gly-Thr-Phe-Thr-Ser-Asp (SEQ ID NO:31) residue and the lysine residue.

In another aspect, the present disclosure relates to an incretin analog comprising:
a lysine residue comprising a group of formula (I) attached to the lysine ε-nitrogen, wherein formula (I) is 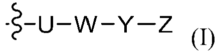 wherein:
   U is absent or represents -C(O)-CH₂-O-(CH₂)₂-O-(CH₂)₂-NH-}, wherein } is point of attachment to W;
   W represents

      -C(O)-CH₂-O-(CH₂)₂-O-(CH₂)₂-NH-],

      -C(O)-NH-(CH₂)₃₋₄-NH-], -C(O)-C(CH₃)₂-NH-],

      or 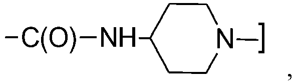 wherein ] is point of attachment to Y;
   Y is absent or represents -C(O)-(CH₂)₂-CH(CO₂H)NH--
      or -C(O)CH((CH₂)ₓCO₂H)NH--, wherein x is 1, 2 or 3, and -- is point of attachment to Z; and
   Z represents -C(O)-(CH₂)ₙ-COOH or -C(O)-(CH₂)ₙ-CH₃, wherein n is an integer from 14-20;
a peptide residue comprising the sequence Gly-Thr-Phe-Thr-Ser-Asp (SEQ ID NO:31) attached indirectly via its carboxy terminus to the lysine residue;
a peptide residue having the sequence Gly-Gly-Pro-Ser-Ser-Gly-Ala-Pro-Pro-Pro-Ser-NH₂ (SEQ ID NO:32) indirectly attached to the carboxy of the lysine via the amino terminus of the Gly-Gly-Pro-Ser-Ser-Gly-Ala-Pro-Pro-Pro-Ser-CONH₂ (SEQ ID NO:32) residue; and
a norvaline amino acid residue attached indirectly to and between the Gly-Thr-Phe-Thr-Ser-Asp (SEQ ID NO:32) residue and the lysine residue.

### DETAILED DESCRIPTION OF THE INVENTION

ABBREVIATIONS
Aib: 2-aminoisobutyric acid
DIPEA: *N,N*'-di-isopropylethylamine
HOBt: 1-hydroxybenztriazole
DIPC: *N,N*'-di-isopropylcarbodiimide
THF: tetrahydrofuran
DCM: dichloromethane
Fmoc: fluorenylmethyloxycarbonyl
HOSu: *N*-hydroxysuccinimide
DCC: dicyclohexyl carbodiimide
DMAc: dimethylacetamide
IBCF: isobutyl chloroformate
NMM: *N*-methylmorpholine
DIC: diisopropylcarbodiimide

### DEFINITIONS

"Pharmaceutically acceptable salts" according to the present disclosure include acid addition salts formed with either organic or inorganic acids. Suitable pharmaceutically acceptable salts of the compounds of the present disclosure include acid addition salts which may be salts of inorganic acids such as hydrochloric acid, hydrobromic acid, phosphoric acid, or the like, or of organic acids such as acetic acid, benzenesulfonic acid, methanesulfonic acid, benzoic acid, citric acid, lactic acid, fumaric acid, succinic acid, adipic acid, pimelic acid, suberic acid, azelaic acid, malic acid, tartaric acid, amino acids (e.g., glutamic acid or aspartic acid), or the like. The pharmaceutically acceptable acid addition salts of the compounds of the present disclosure include salts formed with the addition of one or more equivalents of acids, such as monohydrochloride or dihydrochloride salts. Salts can be prepared by any process under the purview of an ordinary person skilled in the art. (*See* Berge et al., J. Pharm. Sci., 1977, 66, 1-19; and "Handbook of Pharmaceutical Salts: Properties, Selection, and Use," edited by Stahl et al., Verlag Helv. Chim. Acta, Zurich, Switzerland, and Wiley-VCH, Weinheim, Germany, 2002.)

The term "effective amount or amount effective" as used herein refers to an amount of a compound which is sufficient, upon single or multiple dose administration(s) to a subject, in curing, alleviating, relieving, or partially addressing the clinical manifestation of a given disease or state and its complications beyond that expected in the absence of such treatment. Thus, the result can be reduction and/or alleviation of the signs, symptoms, or causes of a disease, or any other desired alteration of a biological system. It is understood that "a therapeutically effective amount" can vary from subject to subject depending on age, weight, general condition of the subject, the condition being treated, the severity of the condition being treated, and the judgment of the prescribing physician.

The amino acid "Aib" as used herein can be represented by structure: 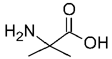 and can also be defined by the chemical name of "2-aminoisobutyric acid."

The amino acid "S(OMe)" or "Ser(OMe)" as used herein can be represented by structure: 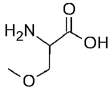 and can also be defined by the chemical name of "serine methyl ether." The terms L-Ser(OMe) and D-Ser(OMe) refer to "L" and "D" isomers of Ser(OMe), respectively.

The amino acid "nor-V", "nor-Val" or "norvaline" as used herein can be represented by structure: 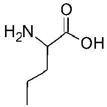 and can also be defined by the chemical name of "2-aminopentanoic acid." The terms L-norvaline and D-norvaline refer to "L" and "D" isomers of norvaline, respectively.

The amino acid "nor-L", "nor-Leu" or "norleucine" as used herein can be represented by structure: 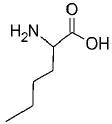 and can also be defined by the chemical name of "2-aminohexanoic acid." The terms L-norleucine and D-norleucine refer to "L" and "D" isomers of norleucine, respectively.

The amino acid "αMe-L", "αMe-Leu" or "αMe-leucine" as used herein can be represented by structure: 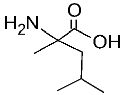 and can also be defined by the chemical name of "2-amino-2,4-dimethylpentanoic acid." The terms L-α-Me-Leucine and D-α-Me-Leucine refer to "L" and "D" isomers of α-Me-Leucine, respectively.

As described herein, the present disclosure provides stable, long-acting GLP-1 mono, GLP-1/GIP dual, GLP-1/GCG dual, and/or GLP-1/GIP/GCG triple receptor agonists which may be useful for treating T2DM, hyperlipidemia/dyslipidemia, metabolic syndromes, metabolic dysfunction-associated steatotic liver disease (MASLD), metabolic dysfunction-associated steatohepatitis (MASH), neurodegenerative disorders, fibrosis, and/or obesity, and reducing cardiovascular risks.

In one aspect, the present disclosure relates to a polypeptide or a pharmaceutically acceptable salt thereof comprising an amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 1)
 wherein:
X1 is Y;
X2 is Aib;
X3 is Q or N;
X10 is Y;
X12 is I;
X13 is L or D isomer of an amino acid of the formula 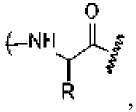 wherein " 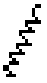 " represents the point of attachment to Leu, and wherein R is selected from C₁-C₆ alkyl, C₃-C₆ cycloalkylmethyl and C₃-C₆ cycloalkyl;
X16 is K;
X17 is I or K;
X18 is A;
X19 is Q;
X20 is, K, Aib, or L or D isomer of an amino acid of the formula 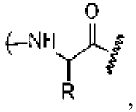 wherein " 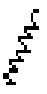 " represents the point of attachment to Leu, and wherein R is selected from C₁-C₆ alkyl, C₃-C₆ cycloalkylmethyl and C₃-C₆ cycloalkyl;
X21 is A;
X23 is V or I;
X24 is Q or E;
X25 is W or Y;
X27 is I or L;
X28 is A or E;
X29 is G;
X30 is G;
X31 is P;
X32 is S;
X33 is S;
X34 is G;
X35 is A;
X36 is P;
X37 is P;
X38 is P; and
X39 is S;

wherein the acid group of the C-terminal amino acid is a free carboxylic acid group or is amidated as C-terminal primary amide; and
with a proviso that at least one of X17 and X20 is K and that at least one of that said K is conjugated to a C₁₆-C₂₂ fatty acid.

In one embodiment, the polypeptide of SEQ ID NO: 1 can have K conjugated to a C₁₆-C₂₂ fatty acid via a linker.

In certain embodiments, the linker is selected from a group consisting of aminoethoxyethoxyacetic acid, glutamic acid, diaminobutane, Aib, and any combinations thereof.

In a preferred embodiment, glutamic acid is γ- glutamic acid.

In another aspect, the present disclosure relates to a polypeptide or a pharmaceutically acceptable salt thereof comprising an amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 2)
 wherein:
X1 is Y;
X2 is Aib;
X3 is Q or N;
X10 is Y;
X12 is I;
X13 is αMe-L;
X16 is K;
X17 is I or K;
X18 is A;
X19 is Q;
X20 is K, Aib, or L or D isomer of an amino acid of the formula 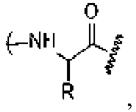 wherein " 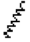 " represents the point of attachment to Leu, and wherein R is selected from C₁-C₆ alkyl, C₃-C₆ cycloalkylmethyl and C₃-C₆ cycloalkyl;
X21 is A;
X23 is V or I;
X24 is Q or E;
X25 is W or Y;
X27 is I or L;
X28 is A or E;
X29 is G;
X30 is G;
X31 is P;
X32 is S;
X33 is S;
X34 is G;
X35 is A;
X36 is P;
X37 is P;
X38 is P; and
X39 is S;

wherein the acid group of the C-terminal amino acid is a free carboxylic acid group or is amidated as C-terminal primary amide; and
with a proviso that at least one of X17 and X20 is K, and further provided that at least one of that said K comprises a side chain amino (ε amino) group acylated with a moiety of the formula selected from:
   aminoethoxyethoxyacetic acid-Aib-Glu-C₁₆-C₂₂ fatty acid chain;
   aminoethoxyethoxyacetic acid-C(O)-diaminobutane-Glu-C₁₆-C₂₂ fatty acid chain;
   Glu-C₁₆-C₂₂ fatty acid chain;
   aminoethoxyethoxyacetic acid- aminoethoxyethoxyacetic acid-Glu-C₁₆-C₂₂ fatty acid chain; and
   aminoethoxyethoxyacetic acid-Glu-C₁₆-C₂₂ fatty acid chain.
   with a proviso that when X20 is Aib, the side chain amino group is not acylated with aminoethoxyethoxyacetic acid-aminoethoxyethoxyacetic acid-Glu-C₁₆-C₂₂ fatty acid chain or aminoethoxyethoxyacetic acid-Glu-C₁₆-C₂₂ fatty acid chain.

In another aspect, the present disclosure relates to a polypeptide or a pharmaceutically acceptable salt thereof comprising the amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 3)
 wherein:
X1 is Y;
X2 is Aib;
X3 is Q or N;
X10 is Y;
X12 is I;
X13 is Aib, Ser(OMe), nor-V, nor-L, or αMe-L;
X16 is K;
X17 is I or K;
X18 is A;
X19 is Q;
X20 is, K, Aib, Ser(OMe), nor-V, or nor-L;
X21 is A;
X23 is V or I;
X24 is Q or E;
X25 is W or Y;
X27 is E, I or L;
X28 is A or E;
X29 is G;
X30 is G;
X31 is P;
X32 is S;
X33 is S;
X34 is G;
X35 is A;
X36 is P;
X37 is P;
X38 is P; and
X39 is S;
wherein the acid group of the C-terminal amino acid is a free carboxylic acid group or is amidated as C-terminal primary amide; and
with a proviso that at least one of X17 and X20 is K, and further provided that at least one of that said K has the side chain amino (ε amino) group acylated with a moiety of the formula selected from:

| | |
|---|---|
| Moiety A | 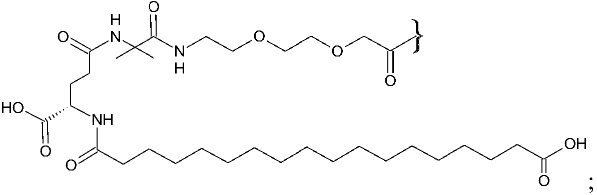 |
| Moiety B | 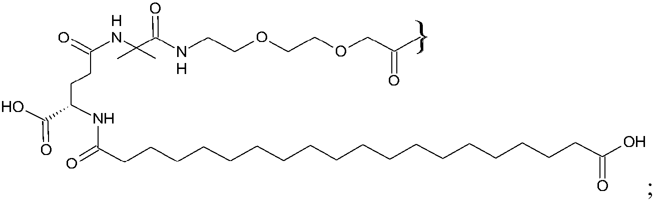 |
| Moiety C | 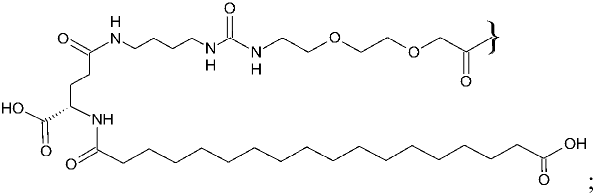 |
| Moiety D | 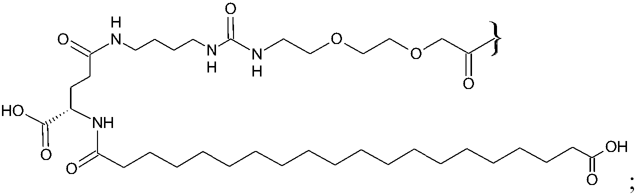 |
| Moiety E | 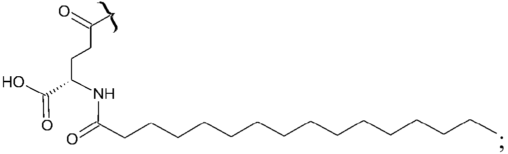 |
| Moiety F | 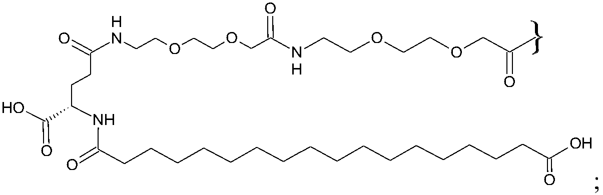 |
| Moiety G | 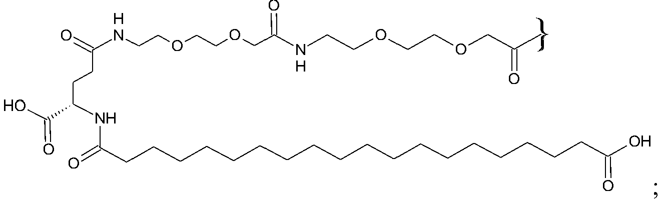 |
| Moiety H | 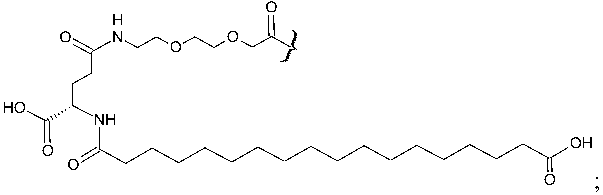 and |
| Moiety I | 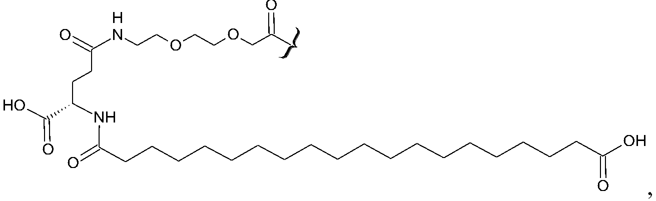 |

wherein the polypeptide is not SEQ ID NO: 7, SEQ ID NO: 23 or SEQ ID NO: 30.

In one embodiment, the polypeptide according to SEQ ID NO: 3 may comprise that:
X1 is Y;
X2 is Aib;
X3 is Q or N;
X10 is Y;
X12 is I;
X13 is nor-V, nor-L or αMe-L;
X16 is K;
X17 is I or K;
X18 is A;
X19 is Q;
X20 is K, Aib, nor-V, or nor-L;
X21 is A;
X23 is V or I;
X24 is Q or E;
X25 is W or Y;
X27 is I or L;
X28 is A or E;
X29 is G;
X30 is G;
X31 is P;
X32 is S;
X33 is S;
X34 is G;
X35 is A;
each of X36, X37 and X38 is independently P; and
X39 is S;
wherein the side chain amino (ε amino) group of K at position X17 is acylated with a moiety of the formula selected from:

| | |
|---|---|
| Moiety A | 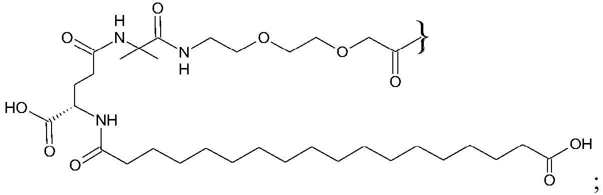 |
| Moiety B | 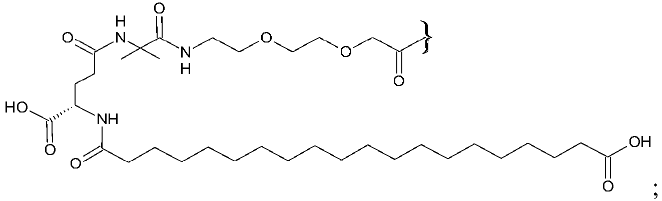 |
| Moiety C | 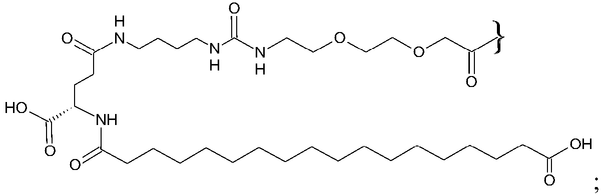 |
| Moiety D | 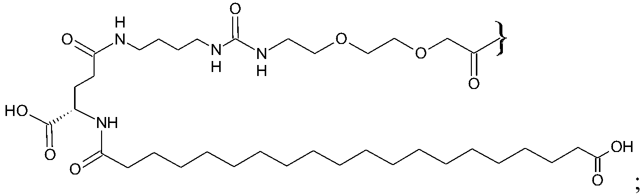 |
| Moiety H | 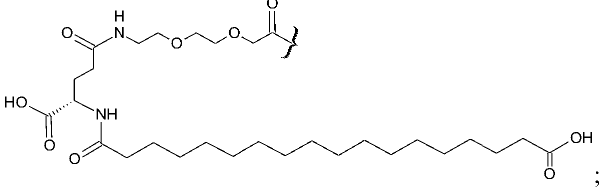 and |
| Moiety I | 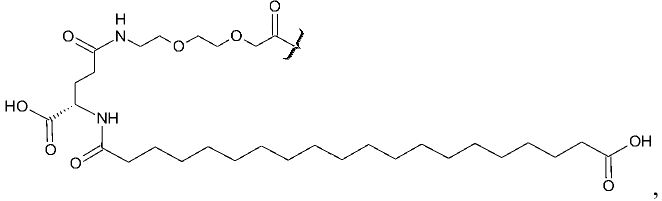 |

wherein the polypeptide is not SEQ ID NO: 7.

In another embodiment, the polypeptide according to SEQ ID NO: 3 may comprise that:
X1 is Y;
X2 is Aib;
X3 is Q or N;
X10 is Y;
X12 is I;
X13 is nor-V, nor-L or αMe-L;
X16 is K;
X17 is K;
X18 is A;
X19 is Q;
X20 is Aib, nor-V or nor-L;
X21 is A;
X23 is I;
X24 is E;
X25 is Y;
X27 is L;
X28 is E;
X29 is G;
X30 is G;
X31 is P;
X32 is S;
X33 is S;
X34 is G;
X35 is A;
each of X36, X37 and X38 is independently P; and
X39 is S;
wherein the side chain amino (ε amino) group of K at position X17 is acylated with a moiety of the formula selected from:

| | |
|---|---|
| Moiety A | 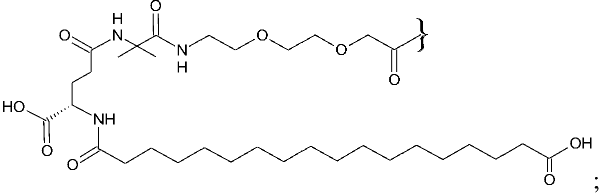 |
| Moiety B | 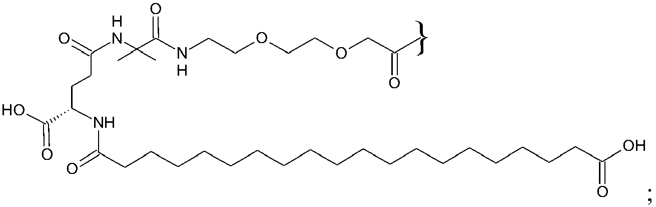 |
| Moiety C | 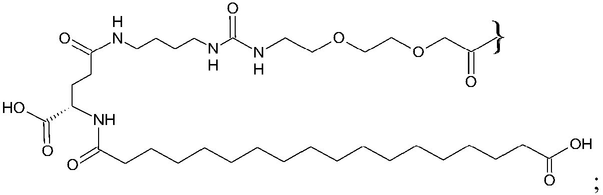 |
| Moiety D | 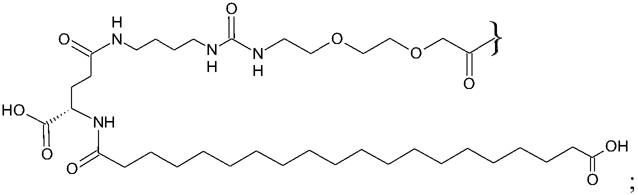 |
| Moiety H | 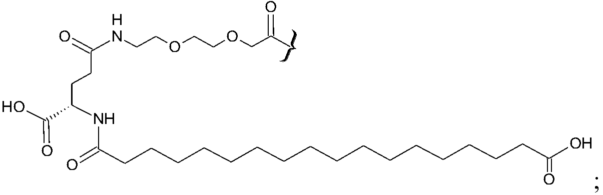 and |
| Moiety I | 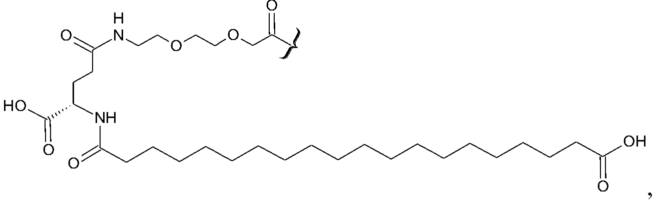 |

wherein the polypeptide is not SEQ ID NO: 7.

In another aspect, the present disclosure relates to a polypeptide or a pharmaceutically acceptable salt thereof comprising an amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 4)
 wherein:
X1 is Y;
X2 is Aib;
X3 is Q or N;
X10 is Y;
X12 is I;
X13 is nor-V, nor-L or αMe-L;
X16 is K;
X17 is I or K;
X18 is A;
X19 is Q;
X20 is K or Aib;
X21 is A;
X23 is V or I;
X24 is Q or E;
X25 is W or Y;
X27 is I or L;
X28 is A or E;
X29 is G;
X30 is G;
X31 is P;
X32 is S;
X33 is S;
X34 is G;
X35 is A;
each of X36, X37 and X38 is independently P; and
X39 is S;
with a proviso that at least one of X17 and X20 is K, and further provided that said K comprises a side chain amino (ε amino) group acylated with a moiety of the formula selected from:

| | |
|---|---|
| Moiety A | 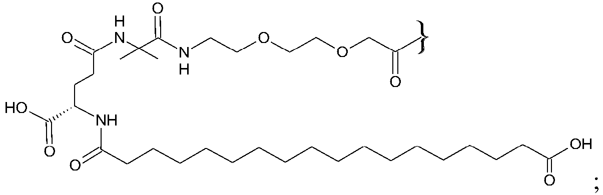 |
| Moiety B | 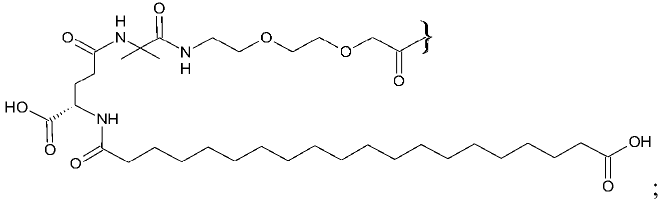 |
| Moiety C | 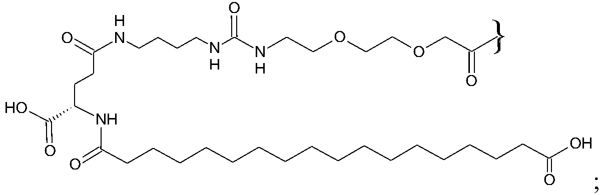 and |
| Moiety D | 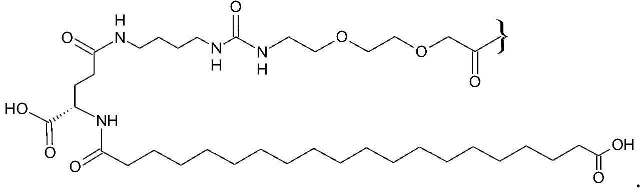 |

In another aspect, the present disclosure relates to a polypeptide or a pharmaceutically acceptable salt thereof comprising an amino acid sequence: 
Y-Aib-X3-G-T-F-T-S-D-Y-S-I-X13-L-D-K-X17-A-Q-X20-A-F-X23-X24-X25-L-X27-
X28-G-G-P-S-S-G-A-P-P-P-S (SEQ ID NO: 5)
 wherein:
X3 is Q or N;
X13 is nor-V, nor-L or αMe-L;
X17 is I or K;
X20 is K or Aib;
X23 is V or I;
X24 is Q or E;
X25 is W or Y;
X27 is I or L; and
X28 is A or E;
wherein the acid group of the C-terminal amino acid is a free carboxylic acid group or is amidated as C-terminal primary amide; and
with a proviso that at least one of X17 and X20 is K, and further provided that said K comprises a side chain amino (ε amino) group acylated with a moiety of the formula selected from:

| | |
|---|---|
| Moiety A | 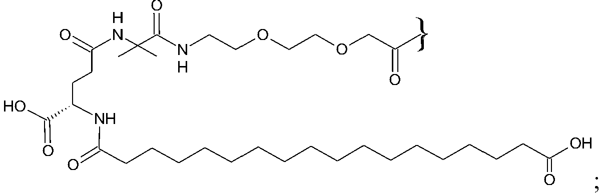 |
| Moiety B | 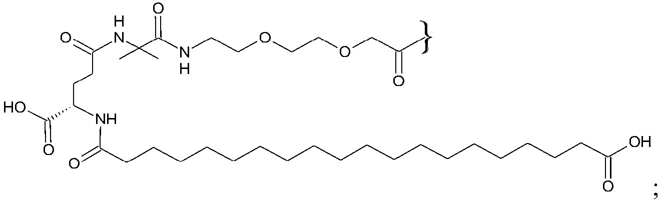 |
| Moiety C | 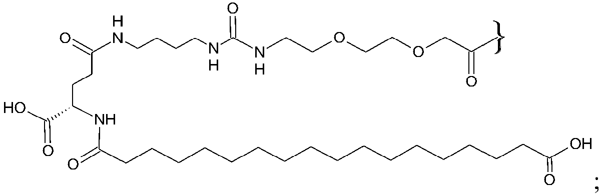 |
| Moiety D | 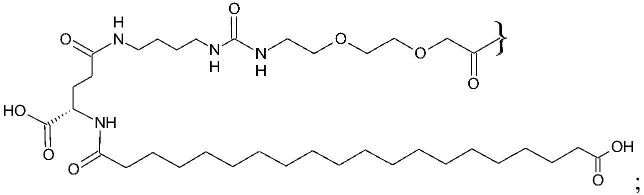 |
| Moiety G | 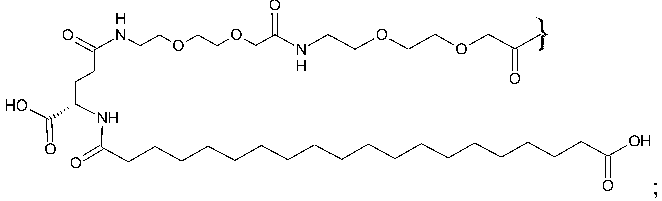 |
| Moiety H | 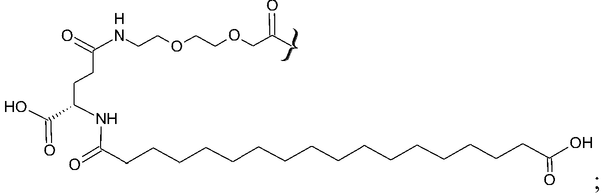 and |
| Moiety I | 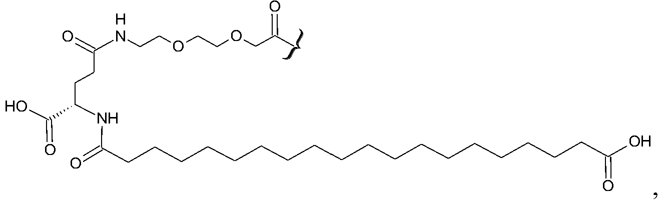 |

wherein the polypeptide is not SEQ ID NO: 7.

In one embodiment, the polypeptide according to SEQ ID NO: 5 may comprise that:
X3 is Q;
X13 is αMe-L;
X17 is K;
X20 is Aib;
X23 is I;
X24 is E;
X25 is Y;
X27 is L; and
X28 is E;
wherein the side chain amino (ε amino) group of K at position X17 is acylated with moiety of the formula selected from:

| | |
|---|---|
| Moiety A | 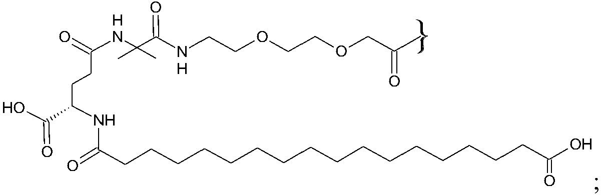 |
| Moiety B | 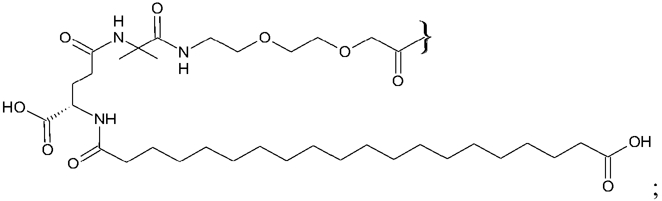 |
| Moiety C | 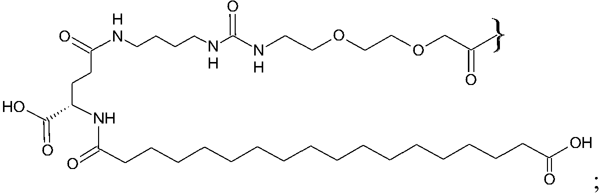 and |
| Moiety D | 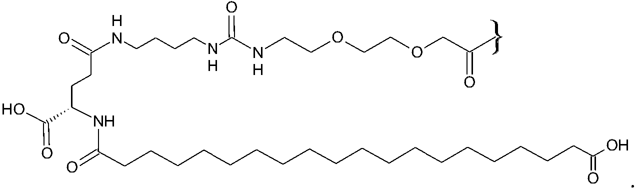 |

In another embodiment, the polypeptide according to SEQ ID NO: 5 may comprise that:
X3 is Q;
X13 is nor-V;
X17 is K;
X20 is Aib;
X23 is I;
X24 is E;
X25 is Y;
X27 is L; and
X28 is E;
wherein the side chain amino (ε amino) group of K at position X17 is acylated with moiety of the formula selected from:

| | |
|---|---|
| Moiety A | 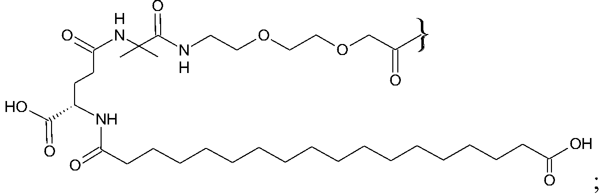 |
| Moiety B | 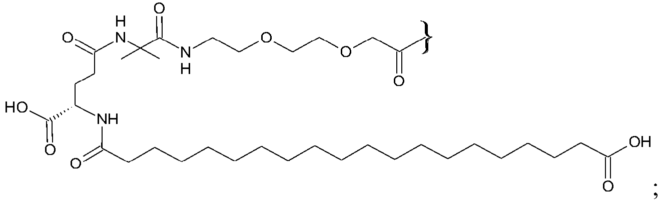 and |
| Moiety G | 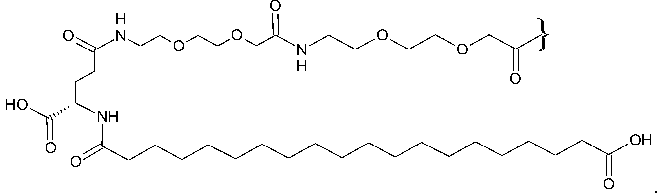 |

In another embodiment, the polypeptide according to SEQ ID NO: 5 may comprise that:
X3 is Q;
X13 is nor-L;
X17 is K;
X20 is Aib;
X23 is I;
X24 is E;
X25 is Y;
X27 is L; and
X28 is E;
wherein the side chain amino (ε amino) group of K at position X17 is acylated with a moiety of the formula selected from:

| | |
|---|---|
| Moiety A | 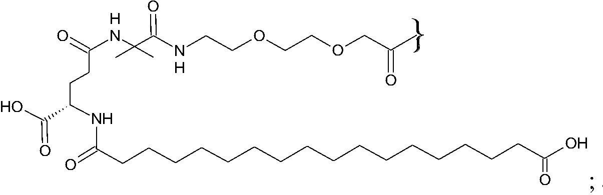 and |
| Moiety B | 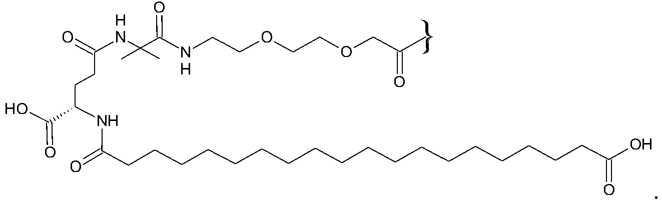 |

In another embodiment, the polypeptide according to SEQ ID NO: 5 may comprise that:
X3 is Q;
X13 is nor-V;
X17 is I;
X20 is K;
X23 is V;
X24 is Q;
X25 is W;
X27 is I; and
X28 is A;
wherein the side chain amino (ε amino) group of K at position X20 is acylated with moiety of the formula selected from:

| | |
|---|---|
| Moiety A | 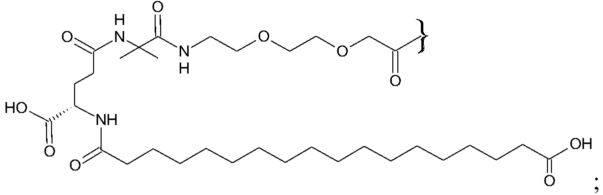 and |
| Moiety B | 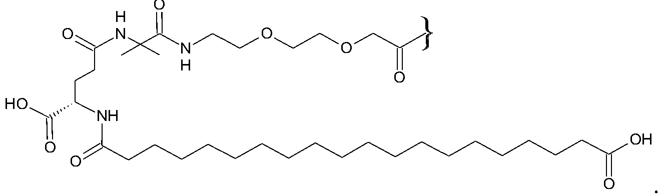 |

In another embodiment, the polypeptide according to SEQ ID NO: 5 may comprise that:
X3 is N;
X13 is nor-V;
X17 is I;
X20 is K;
X23 is V;
X24 is Q;
X25 is W;
X27 is I; and
X28 is A;
wherein the side chain amino (ε amino) group of K at position X20 is acylated with moiety of the formula selected from:

| | |
|---|---|
| Moiety A | 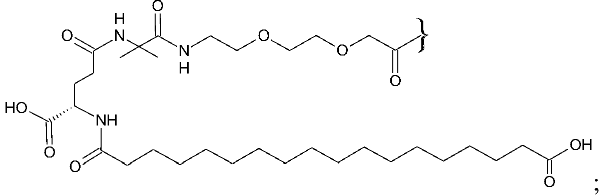 and |
| Moiety B | 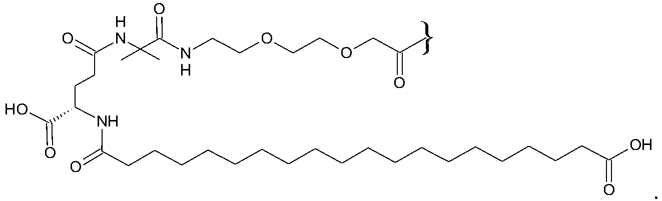 |

In another aspect, the present disclosure relates to a polypeptide or a pharmaceutically acceptable salt thereof comprising an amino acid sequence: 
Y-Aib-X3-G-T-F-T-S-D-Y-S-I-X13-L-D-K-X17-A-Q-X20-A-F-I-E-Y-L-L-E-G-G-P-S-
S-G-A-P-P-P-S (SEQ ID NO: 6)
 Y-Aib-X3-G-T-F-T-S-D-Y-S-I-X13-L-D-K-X17-A-Q-X20-A-F-I-E-Y-L-L-E-G-G-P-S-S-G-A-P-P-P-S (SEQ ID NO: 6 wherein:
X3 is Q or N;
X13 is nor-V, nor-L or αMe-L;
X17 is K; and
X20 is Aib, nor-L or nor-V;
wherein the acid group of the C-terminal amino acid is a free carboxylic acid group or is amidated as C-terminal primary amide; and
wherein the side chain amino (ε amino) group of K at position X17 is acylated with a moiety of the formula selected from:

| | |
|---|---|
| Moiety A | 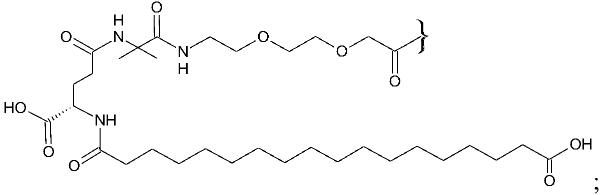 |
| Moiety H | 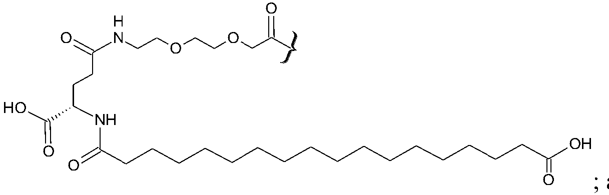 and |
| Moiety I | 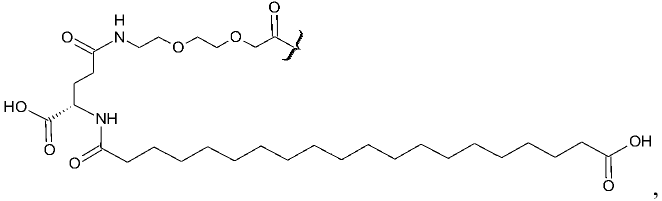 |

wherein the polypeptide is not SEQ ID NO: 7.

In one embodiment, the polypeptide according to SEQ ID NO: 6 may comprise that:
X13 is nor-V or nor-L; and
X20 is Aib;
wherein the side chain amino (ε amino) group of K at position X17 is acylated with moiety of the formula selected from:

| | |
|---|---|
| Moiety H | 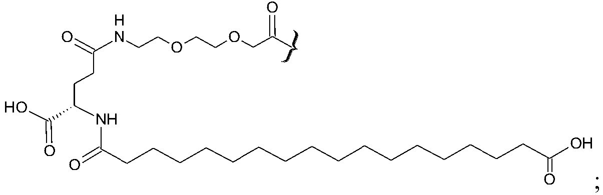 and |
| Moiety I | 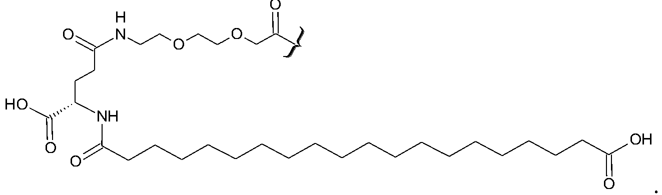 |

In another embodiment, the polypeptide according to SEQ ID NO: 6 may comprise that:
X13 is αMe-L; and
X20 is nor-L or nor-V;
wherein the side chain amino (ε amino) group of K at position X17 is acylated with a moiety of the formula selected from:

| | |
|---|---|
| Moiety A | 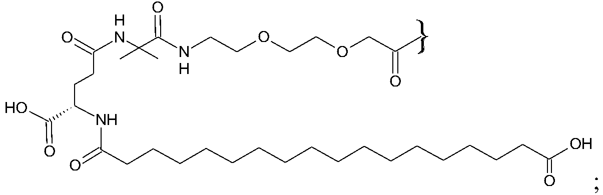 and |
| Moiety I | 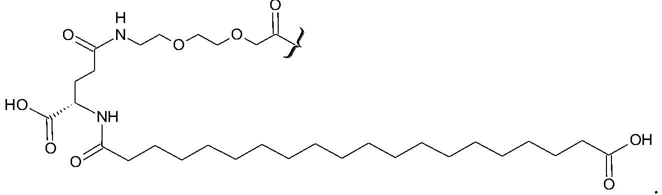 |

In another aspect, the present disclosure relates to an incretin analog polypeptide comprising:
a lysine residue comprising a fatty acid protracting group attached to the lysine ε-nitrogen;
a peptide residue comprising the sequence Gly-Thr-Phe-Thr-Ser-Asp (SEQ ID NO:31) attached indirectly via its carboxy terminus to the lysine residue;
a peptide residue having the sequence Gly-Gly-Pro-Ser-Ser-Gly-Ala-Pro-Pro-Pro-Ser-CONH₂ (SEQ ID NO:32) indirectly attached to the carboxy of the lysine via the amino terminus of the Gly-Gly-Pro-Ser-Ser-Gly-Ala-Pro-Pro-Pro-Ser-CONH₂ (SEQ ID NO:32) residue; and
a norvaline amino acid residue attached indirectly to and between the Gly-Thr-Phe-Thr-Ser-Asp (SEQ ID NO:31) residue and the lysine residue.

In another aspect, the present disclosure relates to an incretin analog comprising:
a lysine residue comprising a group of formula (I) attached to the lysine ε-nitrogen, wherein formula (I) is 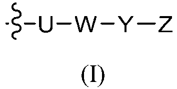 wherein:
   U is absent or represents -C(O)-CH₂-O-(CH₂)₂-O-(CH₂)₂-NH-}, wherein } is point of attachment to W;
   W represents

      -C(O)-CH₂-O-(CH₂)₂-O-(CH₂)₂-NH-],

      -C(O)-NH-(CH₂)₃₋₄-NH-], -C(O)-C(CH₃)₂-NH-],

      or 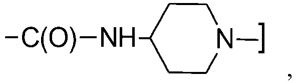 wherein ] is point of attachment to Y;
   Y is absent or represents -C(O)-(CH₂)₂-CH(CO₂H)NH--
      or -C(O)CH((CH₂)ₓCO₂H)NH--, wherein x is 1, 2 or 3, and -- is point of attachment to Z; and
   Z represents -C(O)-(CH₂)ₙ-COOH or -C(O)-(CH₂)ₙ-CH₃, wherein n is an integer from 14-20;
a peptide residue comprising the sequence Gly-Thr-Phe-Thr-Ser-Asp (SEQ ID NO:31) attached indirectly via its carboxy terminus to the lysine residue;
a peptide residue having the sequence Gly-Gly-Pro-Ser-Ser-Gly-Ala-Pro-Pro-Pro-Ser-NH₂ (SEQ ID NO:32) indirectly attached to the carboxy of the lysine via the amino terminus of the Gly-Gly-Pro-Ser-Ser-Gly-Ala-Pro-Pro-Pro-Ser-CONH₂ (SEQ ID NO:32) residue; and
a norvaline amino acid residue attached indirectly to and between the Gly-Thr-Phe-Thr-Ser-Asp (SEQ ID NO:31) residue and the lysine residue.

In one embodiment, in the incretin analog, the lysine is attached to the Gly-Thr-Phe-Thr-Ser-Asp (SEQ ID NO:31) residue by a peptide residue comprising 10 amino acids.

In another embodiment, in the incretin analog, the lysine is attached to the Gly-Gly-Pro-Ser-Ser-Gly-Ala-Pro-Pro-Pro-Ser-CONH₂ (SEQ ID NO:32) residue by a peptide residue comprising 11 amino acids.

In another aspect, the present disclosure relates to a pharmaceutical composition comprising a pharmaceutically acceptable carrier and a polypeptide or an incretin analog as described herein.

In another aspect, the present disclosure relates to a method of treating obesity, Type 2 diabetes mellitus (T2DM), metabolic syndrome, metabolic dysfunction-associated steatotic liver disease (MASLD), metabolic dysfunction-associated steatohepatitis (MASH), neurodegenerative disorders, fibrosis, hyperlipidemia/dyslipidemia, obesity and reducing cardiovascular risks, the method comprising administering to a patient in need of such treatment a polypeptide or an incretin analog as described herein.

In another aspect, the present disclosure relates to a polypeptide or pharmaceutically acceptable salt thereof comprising an amino acid sequence selected from the group consisting of:
i.) Y-Aib-QGTFTSDYSI-(αMe-L)-LDKK*AQ-Aib-AFIEYLLEGGPSSGAPPPS-NH₂ (SEQ ID NO: 8);
ii.) Y-Aib-QGTFTSDYSI-(Norvaline)-LDKK*AQ-Aib-AFIEYLLEGGPSSGAPPPS-NH₂ (SEQ ID NO: 9);
iii.) Y-Aib-NGTFTSDYSI-(Norvaline)-LDKK*AQ-Aib-AFIEYLLEGGPSSGAPPPS-NH₂ (SEQ ID NO: 10);
iv.) Y-Aib-QGTFTSDYSI-(Norleucine)-LDKK*AQ-Aib-AFIEYLLEGGPSSGAPPPS-NH₂ (SEQ ID NO: 11);
v.) Y-Aib-QGTFTSDYSI-(αMe-L)-LDKK*AQ-(nor-V)-AFIEYLLEGGPSSGAPPPS-NH₂ (SEQ ID NO: 12);
vi.) Y-Aib-NGTFTSDYSI-(αMe-L)-LDKK*AQ-(nor-V)-AFIEYLLEGGPSSGAPPPS-NH₂ (SEQ ID NO: 13);
vii.) Y-Aib-QGTFTSDYSI-(αMe-L)-LDKK*AQ-(nor-L)-AFIEYLLEGGPSSGAPPPS-NH₂ (SEQ ID NO: 14);
viii.) Y-Aib-QGTFTSDYSI-(Norvaline)-LDKIAQK*AFVQWLIAGGPSSGAPPPS-NH₂ (SEQ ID NO: 15); and
ix.) Y-Aib-NGTFTSDYSI-(Norvaline)-LDKIAQK*AFVQWLIAGGPSSGAPPPS-NH₂ (SEQ ID NO: 16),
wherein the side chain amino (ε amino) group of K* is acylated with a moiety of the formula selected from:

| | |
|---|---|
| Moiety A | 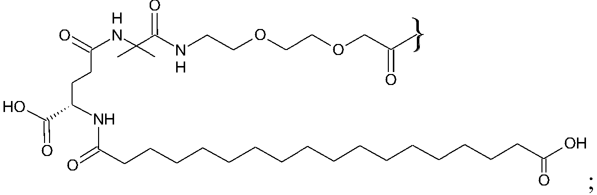 |
| Moiety B | 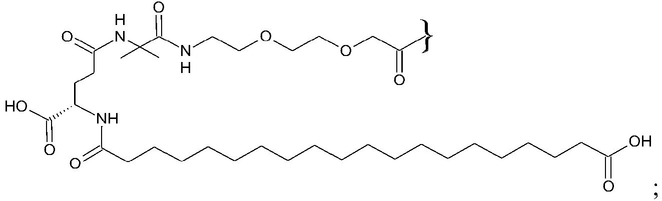 |
| Moiety C | 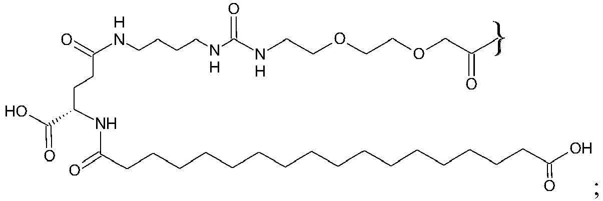 |
| Moiety D | 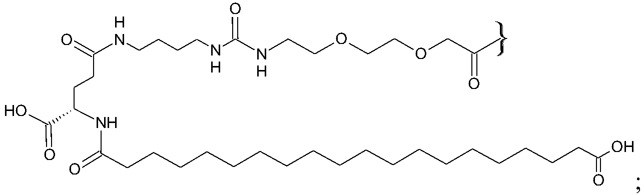 |
| Moiety E | 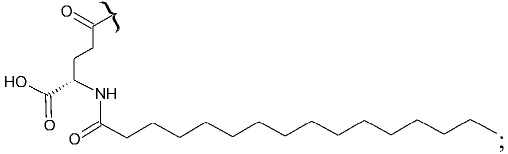 |
| Moiety F | 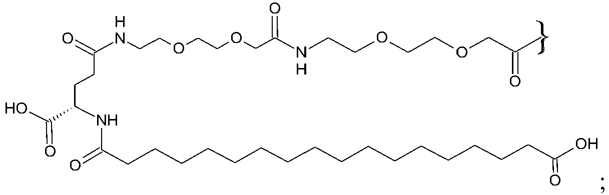 |
| Moiety G | 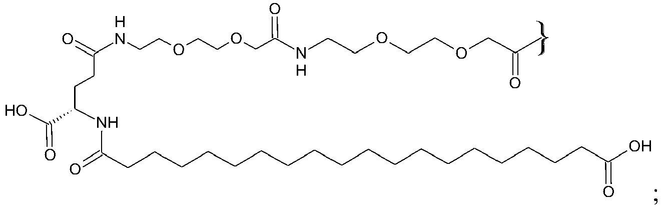 |
| Moiety H | 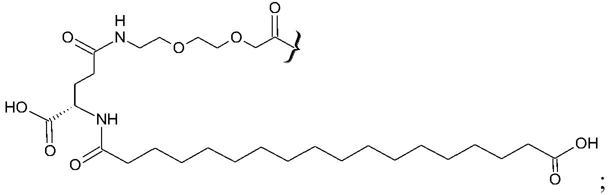 and |
| Moiety I | 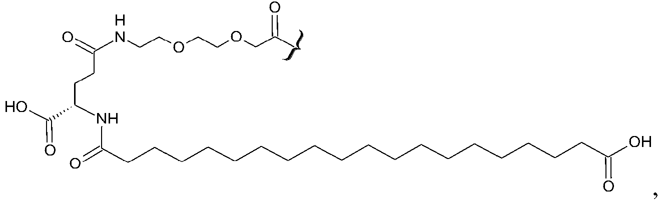 |

wherein the polypeptide is not SEQ ID NO: 7, SEQ ID NO: 23 or SEQ ID NO: 30.

The sequences of the polypeptides as described herein are represented by either the single-letter code or the three-letter code of the amino acids as approved by the International Union of Pure and Applied Chemistry (IUPAC).

Unless stated otherwise, the present disclosure intends to cover both L and D isomers of the amino acids in the sequences as described herein. However, in certain preferred embodiments, all the amino acids are in the "L" configuration unless indicated otherwise.

In another aspect, the present disclosure relates to a polypeptide or pharmaceutically acceptable salt thereof selected from one of the representative compounds in Table 1.

**Table 1. Representative Polypeptide Compounds**

| Compound No. | Structure | SEQ ID NO |
|---|---|---|
| 12 | 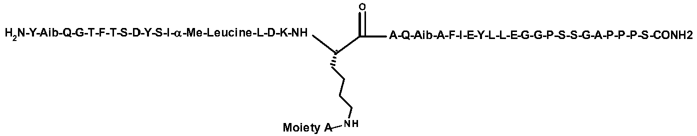 | 17 |
| 13 | 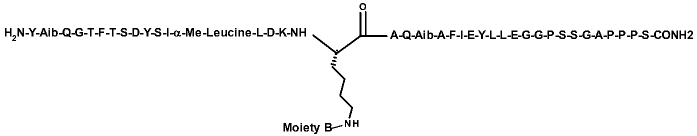 | 18 |
| 14 | 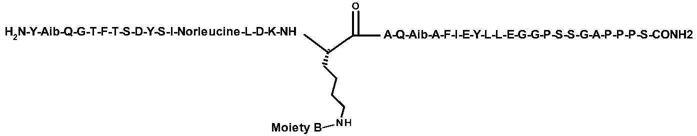 | 19 |
| 15 | 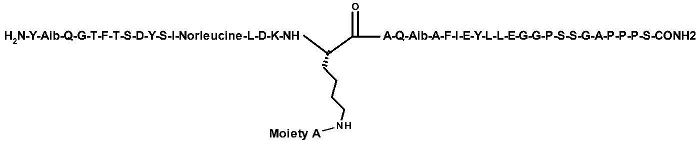 | 20 |
| 16 | 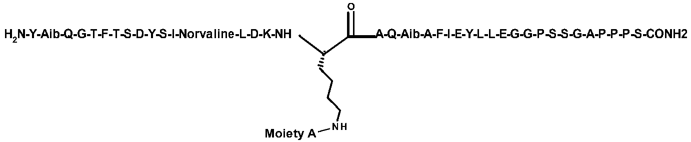 | 21 |
| 17 | 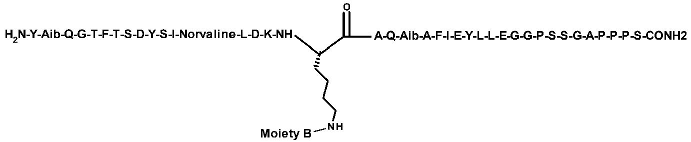 | 22 |
| 18 | 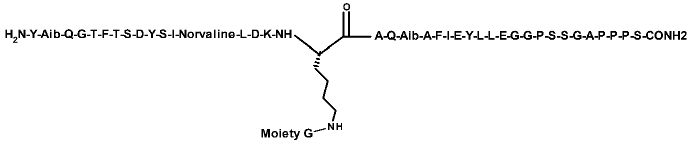 | 23 |
| 19 | 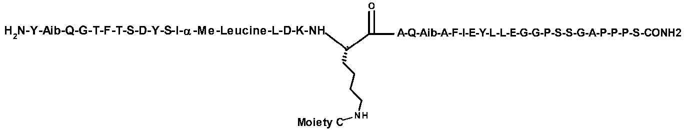 | 24 |
| 20 | 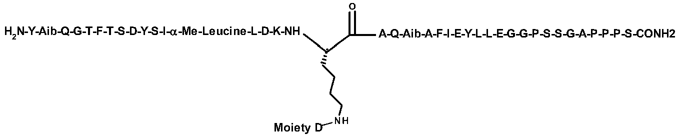 | 25 |
| 21 | 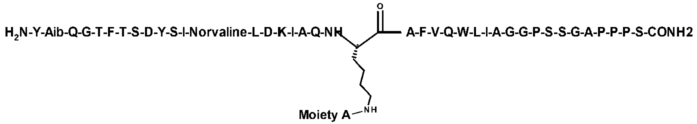 | 26 |
| 22 | 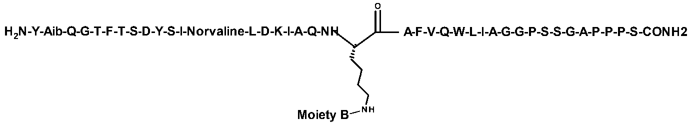 | 27 |
| 23 | 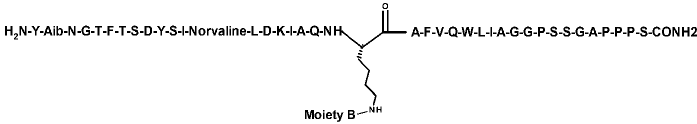 | 28 |
| 24 | 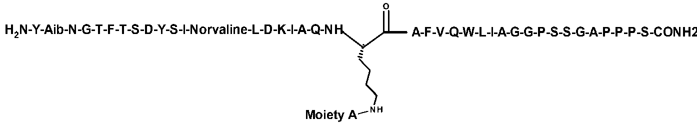 | 29 |
| 26 | 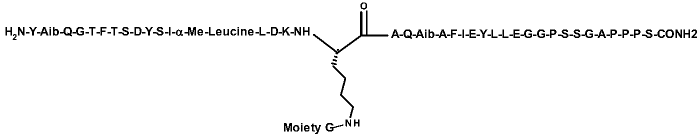 | 30 |

| | | |
|---|---|---|
| *Unless stated otherwise, all the amino acids mentioned above in Table 1 are in the "L" configuration. | | |

**Table 2. Structure of Moiety A, Moiety B, Moiety C, Moiety D, Moiety E, Moiety F, Moiety G, Moiety H, and Moiety I**

| | |
|---|---|
| Moiety A | 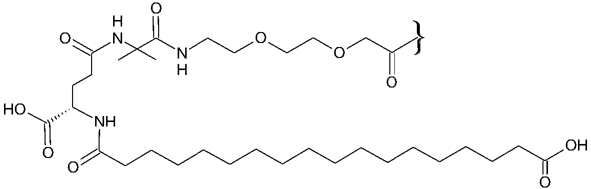 |
| Moiety B | 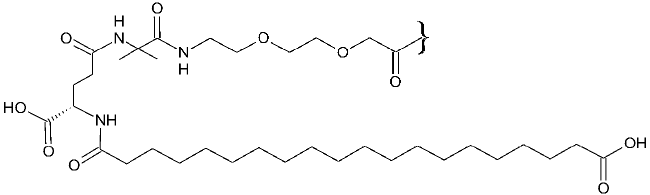 |
| Moiety C | 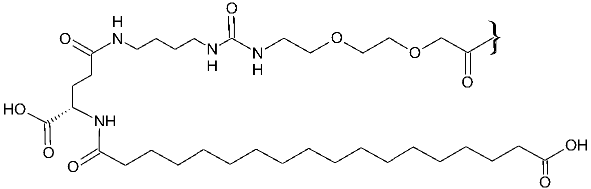 |
| Moiety D | 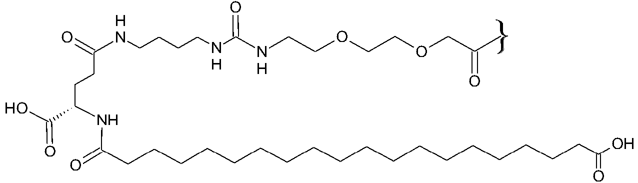 |
| Moiety E | 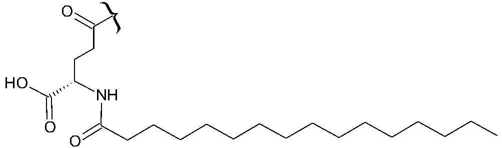 |
| Moiety F | 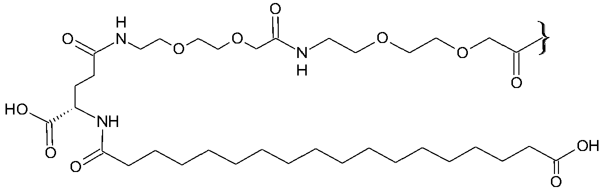 |
| Moiety G | 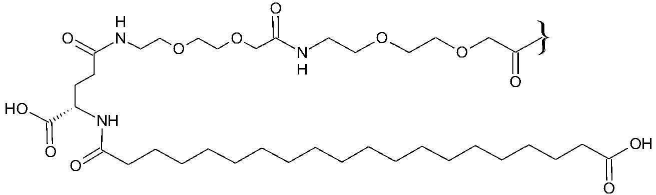 |
| Moiety H | 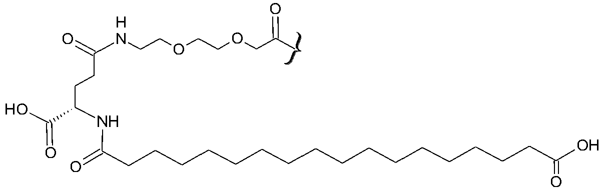 |
| Moiety I | 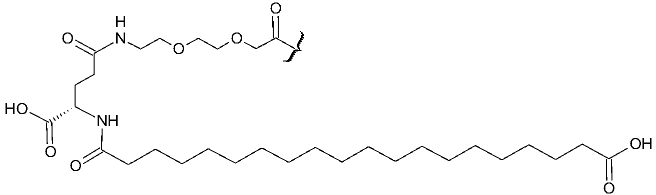 |

In another aspect, the present disclosure relates to a method of treating or preventing Type 2 diabetes mellitus (T2DM).

In another aspect, the present disclosure relates to a method of treating or preventing hyperlipidemia/dyslipidemia.

In another aspect, the present disclosure relates to a method of treating or preventing obesity.

In another aspect, the present disclosure relates to a method of treating or preventing metabolic syndromes, non-alcoholic fatty liver diseases (NAFLD), non-alcoholic steatohepatitis (NASH), neurodegenerative disorders, fibrosis, and/or cardiovascular risks.

In one embodiment, the method of treatment comprises administering to a patient in need thereof an effective amount of a polypeptide as described herein or a pharmaceutically acceptable salt thereof.

In another aspect, the present disclosure relates to a method of treatment of Type 2 diabetes mellitus (T2DM), the method comprising administering to a patient in need of such treatment an effective amount of a polypeptide as described herein or a pharmaceutically acceptable salt thereof.

In another aspect, the present disclosure relates to a method of treatment of obesity, the method comprising administering to a patient in need of such treatment an effective amount of a polypeptide as described herein or a pharmaceutically acceptable salt thereof.

In another aspect, the present disclosure relates to a method of treatment of hyperlipidemia/dyslipidemia, the method comprising administering to a patient in need of such treatment an effective amount of a polypeptide as described herein or a pharmaceutically acceptable salt thereof.

In another aspect, the present disclosure relates to a pharmaceutical composition comprising a polypeptide as described herein or a pharmaceutically acceptable salt thereof with one or more of a pharmaceutically acceptable carrier, diluent, or excipient.

The compounds of the invention are preferably formulated as pharmaceutical compositions administered by parenteral routes (e.g., subcutaneous, intravenous, intraperitoneal, intramuscular, or transdermal). Such pharmaceutical compositions and processes for preparing the same are well known in the art. (*See,* e.g., "Remington: The Science and 50 Practice of Pharmacy, " edited by D. B. Troy, 21st Edition, Lippincott, Williams & Wilkins, 2006).

In another aspect, the present disclosure relates to the polypeptides as described herein or the pharmaceutically acceptable salts thereof for use as a medicament.

In another aspect, the present disclosure relates to the polypeptides as described herein or the pharmaceutically acceptable salts thereof for use in the treatment or prevention of Type 2 diabetes mellitus (T2DM).

In another aspect, the present disclosure relates to the polypeptides as described herein or the pharmaceutically acceptable salts thereof for use in the treatment or prevention of hyperlipidemia/dyslipidemia.

In another aspect, the present disclosure relates to the polypeptides as described herein or the pharmaceutically acceptable salts thereof for use in the treatment or prevention of obesity.

In another aspect, the present disclosure relates to the polypeptides as described herein or the pharmaceutically acceptable salts thereof for use in the treatment or prevention of a disease selected from the group consisting of metabolic syndromes, non-alcoholic fatty liver diseases (NAFLD), non-alcoholic steatohepatitis (NASH), neurodegenerative disorders, fibrosis, and cardiovascular risks.

In another aspect, the polypeptide as described herein or the pharmaceutically acceptable salts thereof may be administered simultaneously, separately or sequentially in combination with an effective amount of one or more additional therapeutic agents.

In another aspect, the pharmaceutical composition according to the present disclosure comprises a polypeptide as described herein or a pharmaceutically acceptable salt thereof for use as a medicament.

In another aspect, the pharmaceutical composition according to the present disclosure comprises a polypeptide as described herein or a pharmaceutically acceptable salt thereof for use in the treatment or prevention of Type 2 diabetes mellitus (T2DM).

In another aspect, the pharmaceutical composition according to the present disclosure comprises a polypeptide as described herein or a pharmaceutically acceptable salt thereof for use in the treatment or prevention of hyperlipidemia/dyslipidemia.

In another aspect, the pharmaceutical composition according to the present disclosure comprises a polypeptide as described herein or a pharmaceutically acceptable salt thereof for use in the treatment or prevention of obesity.

In another aspect, the pharmaceutical composition according to the present disclosure comprises a polypeptide as described herein or a pharmaceutically acceptable salt thereof for use in the treatment or prevention of a disease selected from the group consisting of metabolic syndromes, non-alcoholic fatty liver diseases (NAFLD), non-alcoholic steatohepatitis (NASH), neurodegenerative disorders, fibrosis, and cardiovascular risks.

In another aspect, the pharmaceutical composition according to the present disclosure comprises a polypeptide as described herein or a pharmaceutically acceptable salt thereof which is provided simultaneously, separately or sequentially in combination with an effective amount of one or more additional therapeutic agents.

The present disclosure may involve one or more embodiments as described herein. It is to be understood that the claims as described herein are illustrative of the present disclosure and are not intended to limit the embodiments to the specific embodiments exemplified. It is also to be understood that the embodiments as defined herein may be used independently or in conjunction with any definition or any other embodiment as defined herein. Thus, the present disclosure may contemplate all possible combinations and permutations of various independently described embodiments.

Other features of the present disclosure will become apparent to the skilled artisan based on the following examples. Generally speaking, the present disclosure may extend to any novel feature as described herein, including the accompanying claims and drawings. Thus, features, integers, characteristics, compounds, or chemical moieties described in conjunction with a particular aspect, embodiment or example of the present disclosure are to be understood to be applicable to any other aspect, embodiment or example as described herein, unless incompatible therewith.

Moreover, unless stated otherwise, any features as disclosed herein may be replaced by an alternative feature serving the same or a similar purpose.

### EXAMPLES

**Instruments and analytical methods.** Instruments used for characterization and analysis of the compounds as described herein include High Performance Liquid Chromatograph (HPLC) (Waters e2695 Alliance; Detector Waters (2489 UV/Visible)).

Mass instruments: HPLC: Waters e2695 Alliance; and Detector: Acquity-QDa.

The compounds as described herein were purified by preparative HPLC procedures as outlined below.

Preparative HPLC: WATERS 2555 Quaternary gradient module (Max Total Flow: 300 mL/min, Max Pressure: 3000 psi) or Shimadzu LC-8A (Max Total Flow: 150 mL, Max Pressure: 30 Mpa), Column: Phenyl, 10µ Flow: 75 mL/min

Mobile Phases:

| | For first purification | For second purification |
|---|---|---|
| Mobile Phase A | pH 8.0 phosphate buffer | 1% acetic acid in water |
| Mobile Phase B | Acetonitrile | 1% acetic acid in acetonitrile:n-propanol (50:50) |
| Gradient | 15 to 45% Mobile Phase B in 300 min | 20 to 50 % Mobile Phase B in 250 min |

The purity of the compounds as described herein were analyzed by one of the RP-HPLC methods as outlined below.

### HPLC Method A

Column: Xbridge Peptide BEH C18 (4.6 mm x 250 mm, 3.5µ)
Eluent:
Mobile Phase A: buffer: acetonitrile = 900:100
Mobile Phase B: buffer: acetonitrile = 300:700
Buffer: potassium dihydrogen orthophosphate in water and pH is adjusted to 3.0±0.1 with orthophosphoric acid
Flow rate: 0.8 mL/min
Detection: UV detection at 210 nm
Column Temperature: 65 °C
Sample Tray Temperature: 5 °C
Run Time: 90 min

| Time | Mobile Phase A % | Mobile Phase B % |
|---|---|---|
| 0 | 55 | 45 |
| 3 | 55 | 45 |
| 5 | 40 | 60 |
| 60 | 39 | 61 |
| 65 | 0 | 100 |
| 75 | 0 | 100 |
| 75.01 | 55 | 45 |
| 90 | 55 | 45 |

### HPLC Method B

Column: YMC Pack Pro C18 (4.6 mm x 250 mm, 3.0µ)
Eluent:
   Mobile Phase A: buffer: acetonitrile = 900:100
   Mobile Phase B: buffer: acetonitrile = 300:700
   Buffer: potassium dihydrogen orthophosphate in water and pH is adjusted to 3.0±0.1 with orthophosphoric acid
Flow rate: 1.0 mL/min
Detection: UV detection at 210 nm
Column Temperature: 50 °C
Sample Tray Temperature: 5 °C
Run Time: 38 min

| Time | Mobile Phase A % | Mobile Phase B % |
|---|---|---|
| 0 | 100 | 0 |
| 5 | 100 | 0 |
| 30 | 0 | 100 |
| 32 | 0 | 100 |
| 32.1 | 100 | 0 |
| 38 | 100 | 0 |

### HPLC Method C

Column: X-Select CSH C18, 130A°, 2.5 µm, (4.6 X 150)mm
Eluent:
Mobile Phase A: buffer: acetonitrile = 900:100
Mobile Phase B: buffer: acetonitrile = 300:700
Buffer: potassium dihydrogen orthophosphate in water; a trimethylamine is added; and pH is adjusted to 2.5±0.1 with orthophosphoric acid
Flow rate: 0.5 mL/min
Detection: UV detection at 214 nm
Column Temperature: 60°C
Sample Tray Temperature: 5 °C
Run Time: 90 min

| Time | Mobile Phase A % | Mobile Phase B % |
|---|---|---|
| 0 | 55 | 45 |
| 6 | 55 | 45 |
| 10 | 40 | 60 |
| 80 | 39 | 61 |
| 80.1 | 0 | 100 |
| 85 | 0 | 100 |
| 85.1 | 55 | 45 |
| 90 | 55 | 45 |

### HPLC Method D

Column: X-Select CSH C18, 130A°, 2.5 µm, (4.6 X 150)mm
Eluent:
   Mobile Phase A: buffer: acetonitrile = 900:100
   Mobile Phase B: buffer: acetonitrile = 300:700
   Buffer: potassium dihydrogen orthophosphate in water; trimethylamine is added; and pH is adjusted to 2.5±0.1 with orthophosphoric acid
Flow rate: 0.8 mL/min
Detection: UV detection at 210 nm
Column Temperature: 60°C
Sample Tray Temperature: 5 °C
Run Time: 33 min

| Time | Mobile Phase A % | Mobile Phase B % |
|---|---|---|
| 0 | 60 | 40 |
| 5 | 55 | 45 |
| 25 | 20 | 80 |
| 25.1 | 60 | 40 |
| 33.0 | 60 | 40 |

### METHOD OF PREPARATION

### Example A: Preparation of Moiety A-di-tert-butyl ester

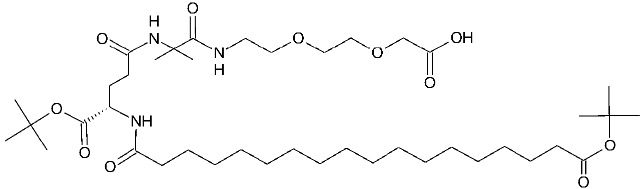

### Moiety A-di-tert-butyl ester

Moiety A-di-*tert*-butyl ester was prepared using solid phase synthesis. 2-[2-(2-Fmoc-aminoethoxy)ethoxy]acetic acid was attached to 2-chlorotrityl chloride resin in the presence of DIPEA to yield 2-[2-(2-Fmoc-aminoethoxy)ethoxy]acetic acid-2-chlorotrityl-resin. The Fmoc protecting group was removed by selective de-blocking of amino group using piperidine followed by coupling with Fmoc-Aib-OH in THF using DIPC and HOBt which yielded 2-[2-[2-[(2-Fmoc-amino-2-methyl-propanoyl)amino]ethoxy]ethoxy]acetic acid-2-chlorotrityl-resin. The Fmoc group was removed by selective de-blocking using piperidine and the free amino group was coupled with Fmoc-Glu-OtBu using HOBt and DIPC to yield 2-[2-[2-[[2-[[(4*S*)-4-Fmoc-amino-5-*tert*-butoxy-5-oxo-pentanoyl]amino]-2-methylpropanoyl] amino]ethoxy]ethoxy]acetic acid-2-chlorotrityl-resin. The Fmoc group of the resultant compound was selectively de-blocked using piperidine, and the free amino group was then coupled with octadecanedioic acid mono *tert* butyl ester to give 2-[2-[2-[[2-[[(4*S*)-5-*tert*-butoxy-4-[(18-*tert*-butoxy-18-oxo-octadecanoyl)amino]-5-oxo-pentanoyl]amino]-2-methyl-propanoyl]- amino]ethoxy]ethoxy]acetic acid-2-chlorotrityl-resin. The intermediate was then cleaved from 2-chlorotrityl-resin using trifluoroethanol:DCM (1:1) to obtain 2-[2-[2-[[2-[[(4*S*)-5-*tert*-butoxy-4-[(18-*tert*-butoxy-18-oxo-octadecanoyl)amino]-5-oxo-pentanoyl]amino]-2-methyl-propanoyl]amino]ethoxy]ethoxy]acetic acid (Moiety A-di-*tert-*butyl ester). LCMS= m/z: 786.39 (M+H⁺).

### Preparation of Moiety A-OSu

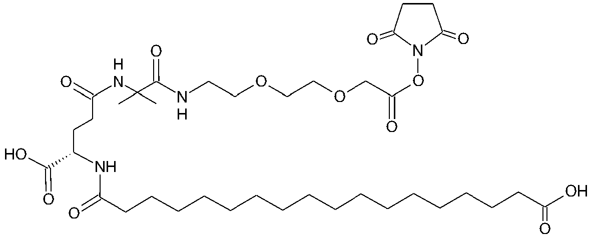

### Moiety A-OSu

The resultant Moiety A-di-*tert*-butyl ester was then reacted with HOSu in the presence of dicyclohexyl carbodiimide (DCC) to yield succinimide protected intermediate, which was de-protected with trifluoroacetic acid to yield the title compound Moiety A-OSu.

### Example B: Preparation of Moiety B-di-tert-butyl ester

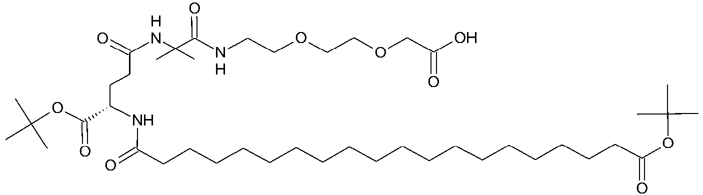

### Moiety B-di-tert-butyl ester

Moiety B-di-*tert*-butyl ester was prepared using the analogous process given in Example A, wherein 20-(*tert*-butoxy)-20-oxoicosanoic acid was used instead of octadecanedioic acid mono *tert* butyl ester to give 2-[2-[2-[[2-[[(4*S*)-5-*tert*-butoxy-4-[(20-*tert-*butoxy-20-oxo-icosanoyl)amino]-5-oxo-pentanoyl]amino]-2-methylpropanoyl]amino]ethoxy]ethoxy]acetic acid-2-chlorotrityl-resin. This intermediate was then cleaved from 2-chlorotrityl-resin using trifluoroethanol:DCM (1:1) to obtain 2-[2-[2-[[2-[[(4*S*)-5-*tert*-butoxy-4-[(20-*tert*-butoxy-20-oxo-icosanoyl)amino]-5-oxo-pentanoyl]amino]-2-methyl-propanoyl]amino]ethoxy]ethoxy]acetic acid (Moiety B-di-*tert*-butyl ester). LCMS= m/z: 814.10 (M+H⁺).

### Preparation of Moiety B-OSu

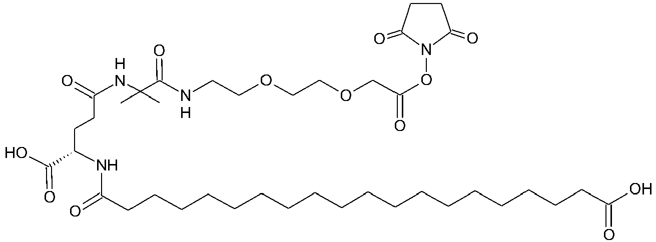

### Moiety B-OSu

The resultant Moiety B-di-*tert*-butyl ester was then reacted with HOSu in the presence of dicyclohexyl carbodiimide (DCC) to yield succinimide protected intermediate, which was de-protected with trifluoroacetic acid to yield the title compound Moiety B-OSu.

### Example C: Preparation of Moiety C-di-tert-butyl ester

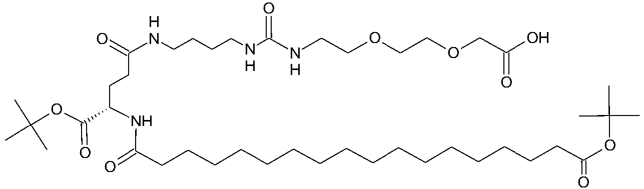

### Moiety C-di-tert-butyl ester

Moiety C-di-*tert*-butyl ester was prepared using solid phase synthesis. 2-[2-(2-Fmoc-aminoethoxy)ethoxy]acetic acid was attached to 2-chlorotrityl chloride resin in the presence of DIPEA to yield 2-[2-(2-Fmoc-aminoethoxy)ethoxy]acetic acid-2-chlorotrityl-resin. The Fmoc protecting group was removed by selective de-blocking of amino group using piperidine, and the free amino group was then activated using *p-*nitrophenylchlroformate in THF and DIPEA followed by reaction with Fmoc-amino butylamine hydrochloride salt in THF:DMAc and DIPEA, which yielded 2-[2-[2-(4-Fmoc-aminobutylcarbamoylamino)ethoxy]ethoxy]acetic acid-2-chlorotrityl-resin. The Fmoc group was removed by selective de-blocking using piperidine, and the free amino group was then coupled to Fmoc-Glu-OtBu using of HOBt and DIPC, which yielded 2-[2-[2-[4-[[(4*S*)-4-Fmoc-amino-5-*tert*-butoxy-5-oxo-pentanoyl]amino]butylcarbamoylamino] ethoxy]ethoxy]acetic acid-2-chlorotrityl-resin. The resultant 2-[2-[2-[4-[[(4S)-4-Fmoc-amino-5-*tert*-butoxy-5-oxo-pentanoyl]amino]-butylcarbamoylamino]ethoxy] ethoxy]acetic acid-2-chlorotrityl-resin was selectively deblocked using piperidine, and then coupled with octadecanedioic acid mono *tert*-butyl ester to give intermediate 2-[2-[2-[4-[[(4*S*)-5-*tert-*butoxy-4-[(18-tert-butoxy-18-oxo-octadecanoyl)amino]-5-oxo-pentanoyl] amino]butylcarbamoylamino]ethoxy]ethoxy]acetic acid-2-chlorotrityl-resin. The intermediate was then cleaved from 2-chlorotrityl-resin using trifluoroethanol:DCM (1:1) to obtain 2-[2-[2-[4-[[(4*S*)-5-*tert*-butoxy-4-[(18-*tert*-butoxy-18-oxo-octadecanoyl)amino]-5-oxo-pentanoyl] amino]butylcarbamoylamino]ethoxy]ethoxy]acetic acid (Moiety C-di-*tert*-butyl ester). LCMS= m/z: 814.56 (M+H⁺).

### Preparation of Moiety C-OSu

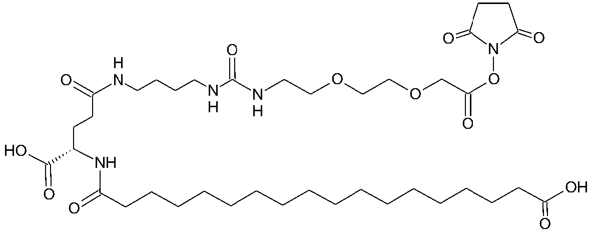

### Moiety C-OSu

The resultant Moiety C-di-*tert*-butyl ester was then reacted with HOSu in the presence of dicyclohexyl carbodiimide (DCC) to yield succinimide protected intermediate, which was de-protected with trifluoroacetic acid to yield the title compound Moiety C-OSu.

### Example D: Preparation of Moiety D-di-tert-butyl ester

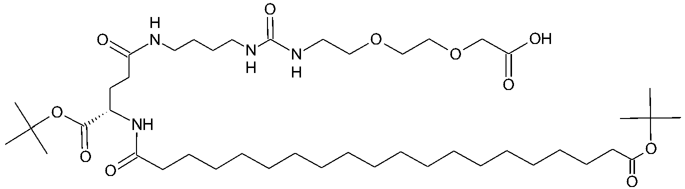

### Moiety D di-tert-butyl-ester

Moiety B-di-*tert*-butyl ester was prepared using the analogous process given in Example C, wherein 20-(*tert*-butoxy)-20-oxoicosanoic acid was used instead of octadecanedioic acid mono *tert* butyl ester to give intermediate 2-[2-[2-[4-[[5-*tert*-butoxy-4-[(20-*tert*-butoxy-20-oxo-icosanoyl)amino]-5-oxo-pentanoyl]amino]butylcarbamoylamino]ethoxy]ethoxy]acetic acid-2-chlorotrityl-resin. The intermediate was then cleaved from 2-chlorotrityl-resin using trifluoroethanol:DCM (1:1) to obtain 2-[2-[2-[4-[[5-*tert*-butoxy-4-[(20-*tert*-butoxy-20-oxo-icosanoyl)amino]-5-oxo-pentanoyl]amino]butylcarbamoylamino]ethoxy]ethoxy]acetic acid (Moiety D-di-tert-butyl ester). LCMS= m/z: 843.14 (M+H⁺).

### Preparation of Moiety D-OSu

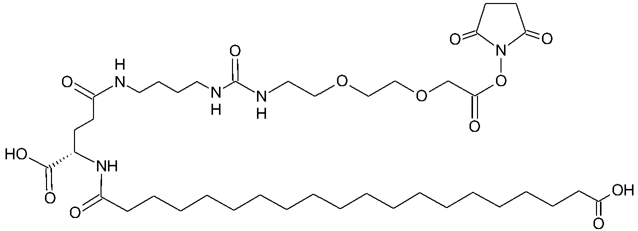

### Moiety D-OSu

The resultant Moiety D-di-*tert*-butyl ester was then reacted with HOSu in the presence of dicyclohexyl carbodiimide (DCC) to yield succinimide protected intermediate, which was de-protected with trifluoroacetic acid to yield the title compound Moiety D-OSu.

### Example E: Preparation of Moiety E-OSu

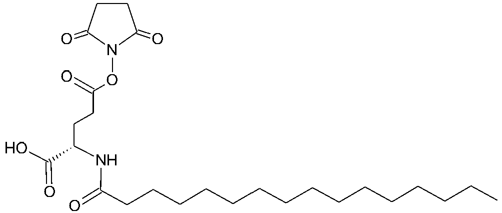

### Moiety E-OSu

L-Glutamic acid alpha-tert-butyl ester (H-Glu-OtBu) was reacted with palmitic acid in the presence of IBCF and NMM to yield CH₃-(CH₂)₁₄-C(O)-Glu-OtBu, which was then reacted with HOSu in the presence of IBCF and NMM to yield CH₃-(CH₂)₁₄-C(O)-Glu(OSu)-OtBu, which was then de-protected with trifluoroacetic acid to yield Moiety E-OSu.

### Example F: Preparation of Moiety F-di-tert-butyl ester

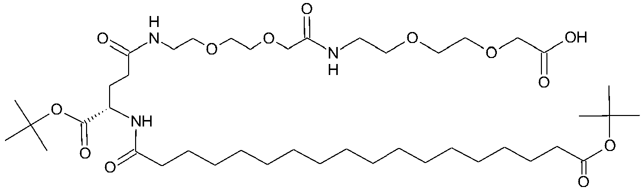

### Moiety F-di-tert-butyl ester

Moiety F-di-*tert*-butyl ester was prepared using solid phase synthesis. 2-[2-(2-Fmoc-aminoethoxy)ethoxy]acetic acid was attached to 2-chlorotrityl chloride resin in the presence of DIPEA to yield 2-[2-(2-Fmoc-aminoethoxy)ethoxy]acetic acid-2-chlorotrityl-resin. The Fmoc protecting group was removed by selective de-blocking of amino group using piperidine followed by coupling with 2-[2-(2-Fmoc-aminoethoxy)ethoxy]acetic acid in THF using DIPC and HOBt, which yielded {(Fmoc-amino-ethoxy)-ethoxy}-acetyl-{(-amino-ethoxy)-ethoxy}-acetic acid-2-chlorotrityl-resin. The Fmoc group was removed by selective de-blocking using piperidine, and the free amino group was coupled with Fmoc-Glu-OtBu using HOBt and DIPC to yield Fmoc-Glu({(amino-ethoxy)-ethoxy}-acetyl-{(-amino-ethoxy)-ethoxy}-acetic acid-2-chlorotrityl-resin)-OtBu. The Fmoc group of the resultant compound was selectively de-blocked using piperidine and the free amino group was then coupled with octadecanedioic acid mono *tert* butyl ester to give 2-[2-[2-[[2-[2-[2-[[5-*tert*-butoxy-4-[(18-*tert*-butoxy-18-oxo-octadecanoyl)amino]-5-oxo-pentanoyl]amino]ethoxy]ethoxy]acetyl]amino]ethoxy]ethoxy]acetic acid-2-chlorotrityl-resin. The intermediate was then cleaved from 2-chlorotrityl-resin using trifluoroethanol:DCM (1:1) to obtain 2-[2-[2-[[2-[2-[2-[[5-tert-butoxy-4-[(18-*tert*-butoxy-18-oxo-octadecanoyl)amino]-5-oxo-pentanoyl]amino]ethoxy]ethoxy]acetyl]amino]ethoxy]ethoxy]acetic acid (Moiety F-di-*tert*-butyl ester). LCMS= m/z: 846.10 (M+H⁺).

### Preparation of Moiety F-OSu

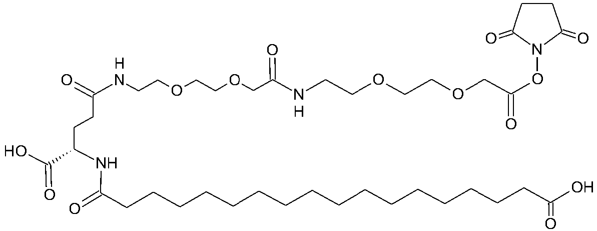

### Moiety F-OSu

The resultant Moiety F-di-*tert*-butyl ester was then reacted with HOSu in the presence of dicyclohexyl carbodiimide (DCC) to yield succinimide protected intermediate, which was de-protected with trifluoroacetic acid to yield the title compound Moiety F-OSu.

### Example G: Preparation Moiety G

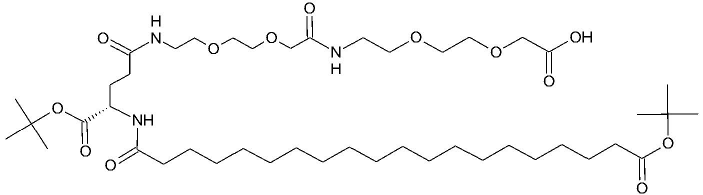

### Moiety G-di-tert-butyl ester

Moiety G-di-*tert*-butyl ester was prepared using the analogous process given in Example F, wherein 20-(*tert*-butoxy)-20-oxoicosanoic acid was used instead of octadecanedioic acid mono *tert* butyl ester to give intermediate 2-[2-[2-[[2-[2-[2-[[5-tert-butoxy-4-[(20-tert-butoxy-20-oxo-icosanoyl)amino]-5-oxo-pentanoyl]amino]ethoxy]ethoxy]acetyl]amino]ethoxy]ethoxy]acetic acid-2-chlorotrityl-resin. The intermediate was then cleaved from 2-chlorotrityl-resin using trifluoroethanol:DCM (1:1) to obtain 2-[2-[2-[[2-[2-[2-[[5-tert-butoxy-4-[(20-tert-butoxy-20-oxo-icosanoyl)amino]-5-oxo-pentanoyl]amino]ethoxy]ethoxy]acetyl]amino]ethoxy]ethoxy]acetic acid (Moiety G-di-*tert*-butyl ester). LCMS= m/z: 874.15 (M+H⁺).

### Preparation of Moiety G-OSu

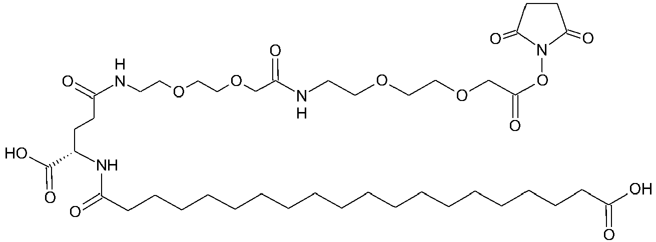

### Moiety G-OSu

The resultant Moiety G-di-*tert*-butyl ester was then reacted with HOSu in the presence of dicyclohexyl carbodiimide (DCC) to yield succinimide protected intermediate, which was de-protected with trifluoroacetic acid to yield the title compound Moiety G-OSu.

### Example H: Preparation Moiety H-di-tert-butyl ester

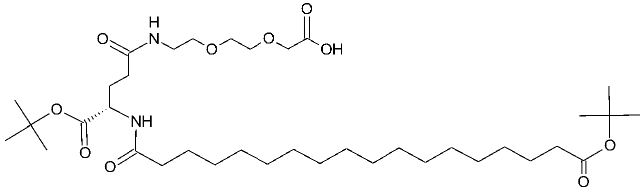

### Moiety H-di-tert-butyl ester

Moiety H-di-*tert*-butyl ester was prepared using solid phase synthesis using 2-chlorotrityl chloride resin. 2-[2-(2-Fmoc-aminoethoxy)ethoxy]acetic acid was attached to 2-chlorotrityl chloride resin in the presence of DIPEA to yield 2-[2-(2-Fmoc-aminoethoxy)ethoxy]acetic acid-2-chlorotrityl-resin. The Fmoc protecting group was removed by selective de-blocking of amino group using piperidine followed by coupling with Fmoc-Glu-OtBu using HOBt and DIPC to yield 2-[2-[2-[[(4S)-5-*tert*-butoxy-4-(9*H*-fluoren-9-ylmethoxycarbonylamino)-5-oxo-pentanoyl]amino]ethoxy]ethoxy]acetic acid-2-chlorotrityl-resin. The Fmoc group of the resultant compound was selectively de-blocked using piperidine and the free amino group was then coupled with octadecanedioic acid mono *tert* butyl ester to give 2-[2-[2-[[(4S)-5-*tert*-butoxy-4-[(18-tert-butoxy-18-oxo-octadecanoyl)amino]-5-oxo-pentanoyl]amino]ethoxy]ethoxy]acetic acid 2-chlorotrityl-resin. The intermediate was then cleaved from 2-chlorotrityl-resin using trifluoroethanol:DCM (1:1) to obtain 2-[2-[2-[[(4S)-5-tert-butoxy-4-[(18-tert-butoxy-18-oxo-octadecanoyl)amino]-5-oxo-pentanoyl]amino]ethoxy]ethoxy]acetic acid (Moiety H-di-*tert*-butyl ester). LCMS= m/z: 700.94 (M+H⁺).

### Preparation of Moiety H-OSu

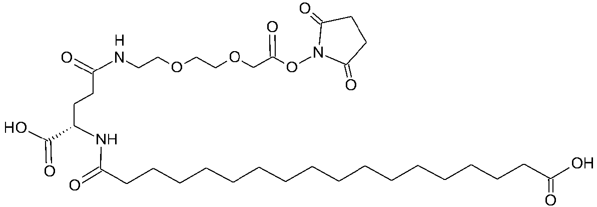

### Moiety H-OSu

The resultant Moiety H-di-*tert*-butyl ester was then reacted with HOSu in the presence of dicyclohexyl carbodiimide (DCC) to yield succinimide protected intermediate, which was de-protected with trifluoroacetic acid to yield the title compound Moiety H-OSu.

### Example I: Preparation Moiety I

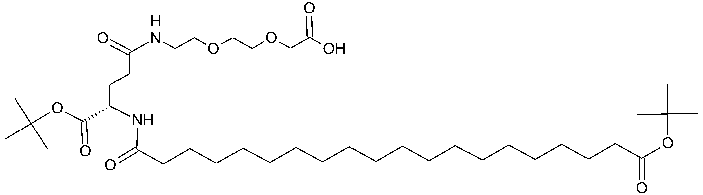

### Moiety I-di-tert-butyl ester

Moiety I-di-*tert*-butyl ester was prepared using the analogous process given in Example H, wherein 20-(*tert*-butoxy)-20-oxoicosanoic acid was used instead of octadecanedioic acid mono *tert* butyl ester to give intermediate 2-[2-[2-[[(4S)-5-tert-butoxy-4-[(20-*tert*-butoxy-20-oxo-icosanoyl)amino]-5-oxo-pentanoyl]amino]ethoxy]ethoxy]acetic acid 2-chlorotrityl-resin. The intermediate was then cleaved from 2-chlorotrityl-resin using trifluoroethanol:DCM (1:1) to obtain 2-[2-[2-[[(4S)-5-tert-butoxy-4-[(20-*tert*-butoxy-20-oxo-icosanoyl)amino]-5-oxo-pentanoyl]amino]ethoxy]ethoxy]acetic acid (Moiety I-di-*tert*-butyl ester). LCMS= m/z: 728.99 (M+H⁺).

### Preparation of Moiety I-OSu

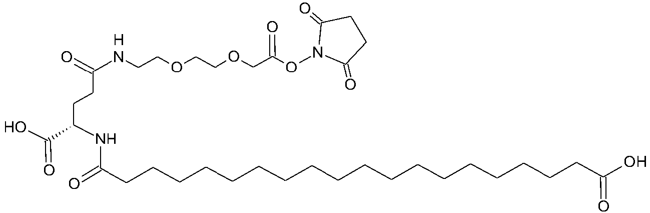

### Moiety I-OSu

The resultant Moiety I-di-*tert*-butyl ester was then reacted with HOSu in the presence of dicyclohexyl carbodiimide (DCC) to yield succinimide protected intermediate, which was de-protected with trifluoroacetic acid to yield the title compound Moiety I-OSu.

### Example 12: Synthesis of Compound 12

The parent peptide was synthesized by solid-phase method. The starting resin used for synthesis was Fmoc-Rink amide resin. Selectively de-blocking of Fmoc protected amino group of Rink amide resin using piperidine followed by coupling of Fmoc-Ser(tBu)-OH with the Rink amide resin. The coupling was performed by using diisopropylcarbodiimide, *N*-hydroxybenzotriazole (DIPC-HOBt) as coupling reagent to yield Fmoc-Ser(tBu)-Rink amide Resin, which completes the first cycle. Acetic anhydride and diisopropylethyl amine was used to terminate/cap the uncoupled amino groups at every amino acid coupling. Selective de-blocking of amino group of Fmoc-Ser(tBu)-Rink amide Resin using piperidine. Then coupling with Fmoc-Pro-OH using HOBt and DIPC yield Fmoc-Pro-Ser(tBu)-Rink amide Resin, which completes the second cycle.

The above 3 steps: selective capping, deblocking of Fmoc- protection of amino acid attached to the resin and coupling of next amino acid residue in sequence with Fmoc-protected amino group were repeated for remaining 36 amino acid residues. The side chain of the Fmoc-protected amino acids were protected orthogonally (e.g., hydroxyl group of Serine, Tyrosine or Threonine were protected with tert-butyl(-tBu) group, amino group of Lysine was protected with tert-butyloxycarbonyl (-Boc) and (4,4-dimethyl-2,6-dioxocyclohex-1-ylidene)-3-methylbutyl (IVDde) group, respectively, and carboxylic acid groups of aspartic acid or glutamic acid were protected with (-tBu) group and amide group of glutamine was protected with trityl (-Trt) group). The above mentioned three steps, i.e., selective capping, deblocking and then coupling with next Fmoc protected amino acid were performed to get Fmoc-Aib-Gln(Trt)-Gly-Thr(tBu)-Phe-Thr(tBu)-Ser(tBu)-Asp(OtBu)-Tyr(tBu)-Ser(tBu)-Ile-(αMethyl-Leu)-Leu-Asp(OtBu)-Lys(Boc)-Lys(IVDde)-Ala-Gln(Trt)-Aib-Ala-Phe-Ile-Glu(OtBu)-Tyr(tBu)-Leu-Leu-Glu(OtBu)-Gly-Gly-Pro-Ser(tBu)-Ser(tBu)-Gly-Ala-Pro-Pro-Pro-Ser(tBu)-resin.

De-blocking of Fmoc-Aib-Gln(Trt)-Gly-Thr(tBu)-Phe-Thr(tBu)-Ser(tBu)-Asp(OtBu)-Tyr(tBu)-Ser(tBu)-Ile-(αMethyl-Leu)-Leu-Asp(OtBu)-Lys(Boc)-Lys(IVDde)-Ala-Gln(Trt)-Alb-Ala-Phe-Ile-Glu(OtBu)-Tyr(tBu)-Leu-Leu-Glu(OtBu)-Gly-Gly-Pro-Ser(tBu)-Ser(tBu)-Gly-Ala-Pro-Pro-Pro-Ser(tBu)-resin using piperidine followed by coupling of Boc-Tyr(tBu)-OH was performed by using diisopropylcarbodiimide, *N-*hydroxybenzotriazole (DIPC-HOBt) as coupling reagent to yield Boc-Tyr(tBu)-Aib-Gln(Trt)-Gly-Thr(tBu)-Phe-Thr(tBu)-Ser(tBu)-Asp(OtBu)-Tyr(tBu)-Ser(tBu)-Ile-(αMethyl-Leu)-Leu-Asp(OtBu)-Lys(Boc)-Lys(IVDde)-Ala-Gln(Trt)-Aib-Ala-Phe-Ile-Glu(OtBu)-Tyr(tBu)-Leu-Leu-Glu(OtBu)-Gly-Gly-Pro-Ser(tBu)-Ser(tBu)-Gly-Ala-Pro-Pro-Pro-Ser(tBu)-resin. De-protection of IVDde group of peptide resin using hydrazine hydrate followed by coupling of Moiety A-di-*tert*-butyl ester was performed by using diisopropylcarbodiimide, *N-*hydroxybenzotriazole (DIPC-HOBt) as coupling reagent in the presence of which yield Compound 12 on resin. Cleavage and de-protection from resin using trifluoroacetic acid with ethane-1,2-dithiol, triisopropylsilane followed by purification through preparative HPLC resulted in purified Compound 12.

Mass (LCMS): m/z = 1197.92 (MH4 4+); Calculated Mass = 4787.64; HPLC Purity (Method C): 97.4%.

### Example 13: Synthesis of Compound 13

Compound 13 was prepared by solid phase method as per the analogous process given for Example 12, wherein IVDde de-protection was followed by coupling of Moiety B-di-tert-butyl ester, instead of Moiety A-di-tert-butyl ester coupling.

Mass (LCMS): m/z = 1204.89 (MH4 4+); Calculated Mass = 4815.52; HPLC Purity (Method C): 97.2%.

### Example 14: Synthesis of Compound 14

Compound 14 was prepared by solid phase method as per the analogous process given for Example 12, wherein (i) Fmoc-norleucine-OH was used instead of Fmoc-αMe-Leucine-OH at position 13, and (ii) IVDde de-protection was followed by coupling of Moiety B-di-*tert*-butyl ester, instead of Moiety A-di-tert-butyl ester coupling.

Mass (LCMS): m/z = 1201.39 ((MH4 4+); Calculated Mass: 4801.53; HPLC Purity (Method C): 98.09%.

### Example 15: Synthesis of Compound 15

Compound 15 was prepared by solid phase method as per the analogous process given for Example 14, wherein IVDde de-protection was followed by coupling of Moiety A-di-tert-butyl ester, instead of Moiety B-di-*tert*-butyl ester coupling.

Mass (LCMS): m/z = 1194.39 (MH4 4+); Calculated Mass: 4773.53; HPLC Purity (Method C): 96.4%.

### Example 16: Synthesis of Compound 16

Compound 16 was prepared by solid phase method as per the analogous process given for Example 12, wherein Fmoc-norvaline-OH was used instead of Fmoc-αMe-Leucine-OH at position 13.

Mass (LCMS): m/z =1191.32 (MH4 4+) and Calculated Mass= 4761.25.

### Example 17: Synthesis of Compound 17

Compound 17 was prepared by solid phase method as per the analogous process given for Example 16, wherein IVDde de-protection was followed by coupling of Moiety B-di-tert-butyl ester, instead of Moiety A-di-*tert*-butyl ester coupling.

### Example 18: Synthesis of Compound 18

Compound 18 was prepared by solid phase method as per the analogous process given for Example 16, wherein IVDde de-protection was followed by coupling of Moiety G-di-tert-butyl ester, instead of Moiety A-di-*tert*-butyl ester coupling.

### Example 19: Synthesis of Compound 19

Compound 19 was prepared by solid phase method as per the analogous process given for Example 12, wherein IVDde de-protection was followed by coupling of Moiety C-di-tert-butyl ester, instead of Moiety A-di-*tert*-butyl ester coupling.

Mass (LCMS) m/z: 1205.64 (MH4 4+)and Calculated Mass: 4818.53.

### Example 20: Synthesis of Compound 20

Compound 20 was prepared by solid phase method as per the analogous process given for Example 12, wherein IVDde de-protection was followed by coupling of Moiety D-di-*tert*-butyl ester, instead of Moiety A-di-*tert*-butyl ester coupling.

Mass (LCMS) m/z: 1212.64 (MH4 4+) and Calculated Mass: 4846.53.

### Example 21: Synthesis of Compound 21

The parent peptide was synthesized by solid-phase method. The starting resin used for synthesis was Fmoc-Rink amide resin. Selectively de-blocking of Fmoc protected amino group of Rink amide resin using piperidine followed by coupling of Fmoc-Ser(tBu)-OH with the Rink amide resin. The coupling was performed by using diisopropylcarbodiimide, *N-*hydroxybenzotriazole (DIPC-HOBt) as coupling reagent to yield Fmoc-Ser(tBu)-Rink amide Resin, which completes the first cycle. Acetic anhydride and diisopropylethyl amine was used to terminate/cap the uncoupled amino groups at every amino acid coupling. Selective de-blocking of amino group of Fmoc-Ser(tBu)-Rink amide Resin using piperidine. Then coupling with Fmoc-Pro-OH using HOBt and DIPC yield Fmoc-Pro-Ser(tBu)-Rink amide Resin, which completes the second cycle. Acetic anhydride and diisopropylethyl amine was used to terminate the uncoupled amino groups after every amino acid coupling.

The above 3 steps: selective capping, deblocking of Fmoc- protection of amino acid attached to the resin and coupling of next amino acid residue in sequence with Fmoc-protected amino group were repeated for remaining 36 amino acid residues. The side chain of the Fmoc-protected amino acids were protected orthogonally (e.g., hydroxyl group of Serine, Tyrosine or Threonine were protected with *tert*-butyl(-tBu) group, amino group of Lysine was protected with *tert*-butyloxycarbonyl (-Boc) and (4,4-dimethyl-2,6-dioxocyclohex-1-ylidene)-3-methylbutyl (IVDde) group, respectively, and carboxylic acid groups of aspartic acid or glutamic acid were protected with (-tBu) group and amide group of glutamine was protected with trityl (-Trt) group). The above mentioned three steps: selective capping, deblocking and then coupling with next Fmoc protected amino acid were performed to get Fmoc-Aib-Gln(Trt)-Gly-Thr(tBu)-Phe-Thr(tBu)-Ser(tBu)-Asp(OtBu)-Tyr(tBu)-Ser(tBu)-Ile-norvaline-Leu-Asp(OtBu)-Lys(Boc)-Ile-Ala-Gln(Trt)-Lys(IVDde)-Ala-Phe-Val-Gln(Trt)-Trp-Leu-Ile-Ala-Gly-Gly-Pro-Ser(tBu)-Ser(tBu)-Gly-Ala-Pro-Pro-Pro-Ser(tBu)-resin.

De-blocking of Fmoc-Aib-Gln(Trt)-Gly-Thr(tBu)-Phe-Thr(tBu)-Ser(tBu)-Asp(OtBu)-Tyr(tBu)-Ser(tBu)-Ile-norvaline-Leu-Asp(OtBu)-Lys(Boc)-Ile-Ala-Gln(Trt)-Lys(IVDde)-Ala-Phe-Val-Gln(Trt)-Trp-Leu-Ile-Ala-Gly-Gly-Pro-Ser(tBu)-Ser(tBu)-Gly-Ala-Pro-Pro-Pro-Ser(tBu)-resin using piperidine followed by coupling of Boc-Tyr(tBu)-OH was performed by using diisopropylcarbodiimide, *N*-hydroxybenzotriazole (DIPC-HOBt) as coupling reagent to yield Boc-Tyr(tBu)-Aib-Gln(Trt)-Gly-Thr(tBu)-Phe-Thr(tBu)-Ser(tBu)-Asp(OtBu)-Tyr(tBu)-Ser(tBu)-Ile-norvaline-Leu-Asp(OtBu)-Lys(Boc)-Ile-Ala-Gln(Trt)-Lys(IVDde)-Ala-Phe-Val-Gln(Trt)-Trp-Leu-Ile-Ala-Gly-Gly-Pro-Ser(tBu)-Ser(tBu)-Gly-Ala-Pro-Pro-Pro-Ser(tBu)-resin. De-protection of IVDde group of peptide resin using hydrazine hydrate followed by coupling of Moiety A-di-*tert*-butyl ester was performed by using diisopropylcarbodiimide, N-hydroxybenzotriazole (DIPC-HOBt) as coupling reagent to yield Compound 21 on resin. Cleavage and de-protection from resin using trifluoroacetic acid with ethane-1,2-dithiol, triisopropylsilane followed by purification through preparative HPLC resulted in purified Compound 21.

Mass (LCMS) m/z: 1185.39 (MH4 4+); Calculated Mass: 4737.53; HPLC Purity (Method C): 98.5%.

### Example 22: Synthesis of Compound 22

Compound 22 was prepared by solid phase method as per the analogous process given for Example 21, wherein IVDde de-protection was followed by coupling of Moiety B-di-tert-butyl ester, instead of Moiety A-di-*tert*-butyl ester coupling.

Mass (LCMS): m/z = 1192.41 (MH4 4+); Calculated Mass: 4765.60; HPLC Purity (Method C): 97.6%.

### Example 23: Synthesis of Compound 23

Compound 23 was prepared by solid phase method as per the analogous process given for Example 22, wherein Fmoc-Asn(Trt)-OH was used instead of Fmoc-Gln(Trt)-OH at position 3.

Mass (LCMS): m/z =1189.20 (MH4 4+) and Calculated Mass: 4752.77.

### Example 24: Synthesis of Compound 24

Compound 24 was prepared by solid phase method as per the analogous process given for Example 21, wherein wherein Fmoc-Asn(Trt)-OH was used instead of Fmoc-Gln(Trt)-OH at position 3.

Mass (LCMS): m/z =1182.22 (MH4 4+) and Calculated Mass: 4724.85.

### Example 26: Synthesis of Compound 26

Compound 26 was prepared by solid phase method as per the analogous process given for Example 12, wherein IVDde de-protection was followed by coupling of Moiety G-di-tert-butyl ester, instead of Moiety A-di-tert-butyl ester coupling.

Mass (LCMS): m/z = 1219.95 (MH4 4+) and Calculated Mass: 4875.76; HPLC Purity (Method C): 96.3%.

### BIOLOGICAL STUDIES

### Example 1: Efficacy Study in db/db Mice at 10nM/kg Dose

The effect of the compounds as described herein on blood glucose, food intake and body weight were studied in mice. This study was performed in Type 2 diabetes mouse (db/db) model. The animals were divided into 9 treatment groups (n=6): a diabetic control group, Compound 12 (10 nM/kg), Compound 13 (10 nM/kg), Compound 26 (10 nM/kg), Compound 14 (10 nM/kg), Compound 15 (10 nM/kg), Compound 21 (10 nM/kg), Compound 22 (10 nM/kg), and Tirzepatide (10 nM/kg). Baseline blood glucose was measured from all the animals. All the animals were administered with test item subcutaneously. Blood glucose was measured at 4 hr, 8 hr, 12 hr, 24 hr, 48 hr, 72 hr, and 96 hr post treatment. Delta blood glucose (mM) was calculated. Body weight changes and cumulative food consumption were measured at 48 hr and 96 hr post treatment.

**Table 3. Effect on Blood Glucose**

| | Delta Blood Glucose (mM) | | | | | | | |
|---|---|---|---|---|---|---|---|---|
| Treatment (n=6) | | 4 hr | 8 hr | 12 hr | 24 hr | 48 hr | 72 hr | 96 hr |
| Diabetic Control | Mean | 0.7 | -0.1 | 0.5 | 0.5 | -0.2 | 0.7 | 0.5 |
| | SD | 0.9 | 2.8 | 2.3 | 3.3 | 3.8 | 2.8 | 3.3 |
| Compound 12, 10 nM/kg/s.c/single dose | Mean | -3.2 | -8.9* | -13.2*** | -13.7*** | -11.4*** | -9.2** | -6.4 |
| | SD | 2.2 | 4.5 | 4.5 | 2.5 | 4.5 | 4.6 | 3.5 |
| Compound 13, 10 nM/kg/s.c/single dose | Mean | -7.4* | -5.7 | -9.4** | -14.4*** | -13.9*** | -12.1*** | -5.6 |
| | SD | 3.1 | 3.6 | 2.7 | 4.3 | 5 | 4.5 | 5.4 |
| Compound 26, 10 nM/kg/s.c/single dose | Mean | -5.5 | -1.9 | -5.6 | -8.1* | -7.3 | -4.7 | 2.1 |
| | SD | 2 | 3.5 | 5.7 | 3.3 | 4.1 | 4.2 | 3.9 |
| Compound 14, 10 nM/kg/s.c/single dose | Mean | -3.8 | -3.1 | -6.2 | -9.2** | -9.3* | -7 | -2.6 |
| | SD | 3.5 | 4.8 | 4.5 | 6.1 | 7 | 6.5 | 3.4 |
| Compound 15, 10 nM/kg/s.c/single dose | Mean | -2.7 | -5.1 | -9.1** | -11.9*** | -8.8* | -6.4 | -1.7 |
| | SD | 2.8 | 1.8 | 2 | 3 | 3.5 | 4.3 | 1.3 |
| Compound 21, 10 nM/kg/s.c/single dose | Mean | -4.5 | -5.2 | -7.7* | -10.2** | 1 | 5.2 | 5.4 |
| | SD | 3.8 | 2.8 | 4.4 | 5.9 | 7.9 | 6.4 | 5.8 |
| Compound 22, 10 nM/kg/s.c/single dose | Mean | -10.6*** | -9.3** | -11.3*** | -14.7*** | -9.6** | -2.8 | -3.9 |
| | SD | 7.7 | 3.8 | 2.6 | 4.2 | 4.7 | 2.8 | 5.6 |
| Tirzepatide, 10 nM/kg/s.c/single dose | Mean | -9.2** | -12.3*** | -12.9*** | -14.4*** | -12.2*** | -8.2* | -4.7 |
| | SD | 7.1 | 8.7 | 6.6 | 7.9 | 8.7 | 14.2 | 12.8 |

| | | | | | | | | |
|---|---|---|---|---|---|---|---|---|
| *p<0.05, **p<0.01, ***p<0.001 vs. Diabetic Control, two-way ANOVA followed by Bonferroni's post test | | | | | | | | |

**Table 4. Effect on Body Weight**

| Groups (n=6) | Body Weight Change (%) 48 hr | | Body Weight Change (%) 96 hr | |
|---|---|---|---|---|
| | Mean | SD | Mean | SD |
| Diabetic Control | 2.2 | 0.6 | 3.2 | 1.8 |
| Compound 12, 10 nM/kg/s.c/single dose | -8.0*** | 2.4 | -3.7*** | 1.7 |
| Compound 13, 10 nM/kg/s.c/single dose | -10.3*** | 1.6 | -6.3*** | 1.6 |
| Compound 14, 10 nM/kg/s.c/single dose | -8.8*** | 1.3 | -7.1*** | 1.6 |
| Compound 15, 10 nM/kg/s.c/single dose | -7.9*** | 2.1 | -5.0*** | 4.5 |
| Compound 21, 10 nM/kg/s.c/single dose | -2.5*** | 1.1 | -1.2* | 1.5 |
| Compound 22, 10 nM/kg/s.c/single dose | -5.4*** | 1.4 | -2.8*** | 1.0 |
| Tirzepatide, 10 nM/kg/s.c/single dose | -4.6*** | 1.7 | -1.8** | 1.0 |

| | | | | |
|---|---|---|---|---|
| *p<0.05, **p<0.01, ***p<0.001 vs. Diabetic Control; One-way ANOVA followed by Dunnett's post test | | | | |

**Table 5. Effect on Food Consumption**

| Groups (n=6) | Cumulative Food Consumption (g) 0-48 hr | | Cumulative Food Consumption (g) 0-96 hr | |
|---|---|---|---|---|
| | Mean | SD | Mean | SD |
| Diabetic Control | 15.7 | 0.3 | 25.7 | 2.1 |
| Compound 12, 10 nM/kg/s.c/single dose | 3.1*** | 0.8 | 10.9*** | 3.6 |
| Compound 13, 10 nM/kg/s.c/single dose | 4.1*** | 1.9 | 11.2*** | 1.0 |
| Compound 14, 10 nM/kg/s.c/single dose | 3.0*** | 0.8 | 8.3*** | 2.9 |
| Compound 15, 10 nM/kg/s.c/single dose | 3.6*** | 1.6 | 15.6** | 7.1 |
| Compound 21, 10 nM/kg/s.c/single dose | 6.4*** | 0.5 | 19.6 | 0.5 |
| Compound 22, 10 nM/kg/s.c/single dose | 4.8*** | 1.8 | 13.6*** | 7.8 |
| Tirzepatide, 10 nM/kg/s.c/single dose | 2.4*** | 2.6 | 9.6*** | 6.8 |

| | | | | |
|---|---|---|---|---|
| *p<0.05, **p<0.01, ***p<0.001 vs. Diabetic Control; One-way ANOVA followed by Dunnett's post test | | | | |

The results presented above demonstrate that the compounds can be effective in treatment of Type 2 diabetes, diabetes with obesity, obesity, and hyperlipidemia.

### Example 2: Oral Glucose Tolerance Test (OGTT) in Rats; Single Injection; 30 nM/kg dose

Animals were divided into 6 groups (n=4/group): a placebo control group, Compound 17, (30 nM/kg), Compound 18 (30 nM/kg), Compound 19 (30 nM/kg), Compound 20 (30 nM/kg), and Retatrutide (30 nM/kg). Animals were fasted for 12 hours before initiation of OGTT. After 24 hours of subcutaneous injection of tests or Retatrutide, blood glucose was measured with blood glucometer (time 0 measurement). All the animals were given 2 g/kg of glucose solution orally. Blood glucose was measured at 10, 20, 40, 60, 90, and 120 min following glucose challenge. Body weight and food consumption were recorded at 12 hr, 48 hr and 72 hr.

**Table 6. Change in Blood Glucose AUC₍₀₋₁₂₀ₘᵢₙ₎ 24 hr Post Subcutaneous Injection**

| Treatment | Mean | SD | % Change in Blood glucose AUC(₀₋₁₂₀ₘᵢₙ₎ vs placebo control |
|---|---|---|---|
| Placebo Control | 9672.5 | 450.9 | - |
| Compound 17, 30 nM/kg, s.c | 4771.3*** | 788.7 | -50.67 |
| Compound 18, 30 nM/kg, s.c | 4176.3*** | 545.8 | -56.82 |
| Compound 19, 30 nM/kg, s.c | 3848.8*** | 1207.9 | -60.20 |
| Compound 20, 30 nM/kg, s.c | 4472.5*** | 681.1 | -53.76 |
| Retatrutide, 30 nM/kg, s.c | 3792.5*** | 832.9 | -60.79 |

| | | | |
|---|---|---|---|
| *p<0.05, **p<0.01, ***p<0.001 vs Placebo Control; One-way ANOVA followed by Bonferroni's posttests | | | |

**Table 7. Body Weight Reduction (%)**

| | 24 hr | | 48 hr | | 72 hr | |
|---|---|---|---|---|---|---|
| Treatment (n=4) | Mean | SD | Mean | SD | Mean | SD |
| Placebo Control | -0.8 | 2.0 | 2.4 | 2.1 | 5.9 | 2.8 |
| Compound 17, 30 nM/kg, s.c | -6.8*** | 1.8 | -5.0*** | 1.9 | -3.3*** | 2.0 |
| Compound 18, 30 nM/kg, s.c | -6.5*** | 1.0 | -4.6*** | 0.9 | -2.6*** | 0.7 |
| Compound 19, 30 nM/kg, s.c | -6.3*** | 1.7 | -4.9*** | 1.1 | -2.6*** | 1.2 |
| Compound 20, 30 nM/kg, s.c | -6.0*** | 1.4 | -4.6*** | 1.1 | -2.7*** | 1.5 |
| Retatrutide, 30 nM/kg, s.c | -6.0*** | 0.9 | -4.5*** | 1.5 | -2.5*** | 1.5 |

| | | | | | | |
|---|---|---|---|---|---|---|
| *p<0.05, **p<0.01, ***p<0.001 vs Placebo Control and #p<0.05, ##p<0.01, and ###p<0.001 vs Retatrutide; One-way ANOVA followed by Bonferroni's posttests. | | | | | | |

**Table 8. Food Consumption (g)**

| | 0-12 hr | | 24-48 hr | | 24-72 hr | |
|---|---|---|---|---|---|---|
| Treatment (n=4) | Mean | SD | Mean | SD | Mean | SD |
| Placebo Control | 10.7 | 2.7 | 33.4 | 4.8 | 59.3 | 5.2 |
| Compound 17, 30 nM/kg, s.c | 1.4*** | 0.6 | 15.8*** | 1.9 | 31.3*** | 6.0 |
| Compound 18, 30 nM/kg, s.c | 3.0*** | 0.6 | 5.8*** | 1.0 | 16.8*** | 3.8 |
| Compound 19, 30 nM/kg, s.c | 1.3*** | 0.3 | 7.2*** | 2.4 | 17.0*** | 1.7 |
| Compound 20, 30 nM/kg, s.c | 1.6*** | 1.3 | 12.8*** | 3.1 | 33.1*** | 2.7 |
| Retatrutide, 30 nM/kg, s.c | 0.7*** | 0.6 | 8.6*** | 1.1 | 23.8*** | 3.8 |

| | | | | | | |
|---|---|---|---|---|---|---|
| *p<0.05, **p<0.01, ***p<0.001 vs Placebo Control and #p<0.05, ##p<0.01, and ###p<0.001 vs Retatrutide; One-way ANOVA followed by Bonferroni's posttests. | | | | | | |

Surprisingly, the present inventors have found that the compounds showed comparable weight reduction to Retatrutide but without dramatic food intake reduction. This may be because of the lesser potency of the compounds on GCGR (verses GLP-1R) as compared to Retatrutide.

The variation in individual receptor agonism in GLP-1/GCG dual receptor agonists and GLP-1/GIP/GCG triple receptor agonists might lead to various outcomes in terms of therapeutic efficacy and adverse effects.

To attain optimum efficacy and reduce side effects, a delicate balance must be achieved between GLP-1/GIP/GCG receptor agonism.

It is a challenge to devise molecules which show enhanced therapeutic effect over the existing therapies and lower side effects. The weight reduction caused by Compounds 17, 18, 19, and 20 without causing acute reduction in food consumption might be related to improved potency of these compounds towards GLP-1R compared to GCGR, which can be derived from Example 7.

### Example 3: In-Vitro Assays

Stably expressing GLP-1R, GIPR or GCGR cell lines were used to determine *in-vitro* potency of the compounds as described herein. Signaling by the GLP-1R, GIP-R, and GCG-R involves activation of adenylate cyclase and cAMP production. Hit Hunter^{®} cAMP assays monitor the activation of GLP-1R, GIPR or GCGR via Gi and Gs secondary messenger signaling using a technology developed by DiscoverX which is called Enzyme Fragment Complementation (EFC) with β-galactosidase (β-Gal) as the functional reporter. The enzyme is split into two complementary portions: EA for Enzyme Acceptor and ED for Enzyme Donor. ED is fused to cAMP and in the assay competes with cAMP generated by cells for binding to a cAMP-specific antibody. Active β-Gal is formed by complementation of exogenous EA to any unbound ED cAMP. Active enzyme can then convert a chemiluminescent substrate, generating an output signal detectable on a standard microplate reader.

Three different assays were performed using cells expressing either of the three receptors. cAMP Hunter cell lines were expanded from freezer stocks according to standard procedures. Cells were seeded in a total volume of 20 µL into white walled, 384-well microplates and incubated at 37 °C for the appropriate time prior to testing. Media was aspirated and cells were then treated with 15 uL of cAMP conjugated antibody and 5 uL of test compound. After appropriate compound incubation, assay signal was generated through incubation with 20 µL cAMP-ED cell lysis cocktail for one hour followed by incubation with 20 µL cAMP-EA reagent for three hours at room temperature. Free cAMP-ED available in the system compliment with the free cAMP-EA to form Active β-Gal that reacts with the substrate to give chemiluminescent signal. Microplates were read following signal generation with a PerkinElmer EnvisionTM instrument for chemiluminescent signal detection. The amount of signal is directly proportional to the concentration of cAMP generated due to response. Different concentrations of the sample was used (different for different compound) to generate log Concentration to %Effect curve. Four parametric logistic curve was generated and EC50 was determined. Appropriate assay reference was used (Exendin-4 for GLP-1R, GIP for GIPR and Glucagon for GCGR) for each assay.

Cellular cAMP Assay of Retatrutide, Compound 12, Compound 13, Compound 14, Compound 17, Compound 18, Compound 19, and Compound 20 was performed and the half effective concentrations on GLP-1R-expressing cells and GIPR-expressing cells was as mentioned in Table 9.

**Table 9.**

| | | EC50 value | | |
|---|---|---|---|---|
| Compound | Concentration | GCG | GIP | GLP-1 |
| Compound 12 | (nM) | 6.99 | 12.04 | 11.96 |
| Compound 13 | (nM) | >1.8 | >1.5 | >2.4 |
| Compound 14 | (nM) | 20.89 | 24.03 | 8.54 |
| Compound 17 | (pM) | 13.33 | 11.48 | 8.7 |
| Compound 18 | (pM) | 193.2 | 22.68 | 17.33 |
| Compound 19 | (pM) | 109.1 | 13.79 | 11.61 |
| Compound 20 | (pM) | 139.8 | 18.1 | 14.37 |
| Retatrutide | (pM) | 9.63 | 5.99 | 8.3 |

A lower affinity for GCG receptor in comparison to GLP-1 receptor might reduce several side effects caused by GCGR agonism.

Glucagon agonism is diabetogenic and causes increased heart rate, catabolism of amino acids and proteins leading to loss of lean body mass. Therefore the compounds of the present invention offer several benefits along with reduced side effects profile.

### EMBODIMENTS

Embodiment 1. A polypeptide or a pharmaceutically acceptable salt thereof comprising an amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 1)
 wherein:
X1 is Y;
X2 is Aib;
X3 is Q or N;
X10 is Y;
X12 is I;
X13 is L or D isomer of an amino acid of the formula 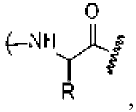 wherein " 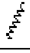 " represents the point of attachment to Leu, and wherein R is selected from C₁-C₆ alkyl, C₃-C₆ cycloalkylmethyl and C₃-C₆ cycloalkyl;
X16 is K;
X17 is I or K;
X18 is A;
X19 is Q;
X20 is K, Aib, or L or D isomer of an amino acid of the formula 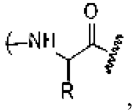 wherein " 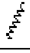 " represents the point of attachment to Leu, and wherein R is selected from C₁-C₆ alkyl, C₃-C₆ cycloalkylmethyl and C₃-C₆ cycloalkyl;
X21 is A;
X23 is V or I;
X24 is Q or E;
X25 is W or Y;
X27 is I or L;
X28 is A, or E;
X29 is G;
X30 is G;
X31 is P;
X32 is S;
X33 is S;
X34 is G;
X35 is A;
X36 is P;
X37 is P;
X38 is P; and
X39 is S;

wherein the acid group of the C-terminal amino acid is a free carboxylic acid group or is amidated as C-terminal primary amide; and
with a proviso that at least one of X17 and X20 is K and that at least one of that said K is conjugated to a C₁₆-C₂₂ fatty acid.
Embodiment 2. The polypeptide according to embodiment 1, wherein K is conjugated to a C₁₆-C₂₂ fatty acid via a linker.
Embodiment 3. The polypeptide according to embodiment 2, wherein the linker is selected from a group consisting of aminoethoxyethoxyacetic acid, glutamic acid, diaminobutane, Aib, and any combinations thereof.
Embodiment 4. The polypeptide according to embodiment 3, wherein the glutamic acid is γ- glutamic acid.
Embodiment 5. A polypeptide or a pharmaceutically acceptable salt thereof comprising an amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 2)
 wherein:
X1 is Y;
X2 is Aib;
X3 is Q or N;
X10 is Y;
X12 is I;
X13 is αMe-L;
X16 is K;
X17 is I or K;
X18 is A;
X19 is Q;
X20 is K, Aib, or L or D isomer of an amino acid of the formula 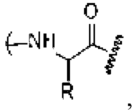 wherein " 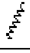 " represents the point of attachment to Leu, and wherein R is selected from C₁-C₆ alkyl C₃-C₆ cycloalkylmethyl and C₃-C₆ cycloalkyl;
X21 is A;
X23 is V or I;
X24 is Q or E;
X25 is W or Y;
X27 is I or L;
X28 is A, or E;
X29 is G;
X30 is G;
X31 is P;
X32 is S;
X33 is S;
X34 is G;
X35 is A;
X36 is P;
X37 is P;
X38 is P; and
X39 is S;

wherein the acid group of the C-terminal amino acid is a free carboxylic acid group or is amidated as C-terminal primary amide; and
with a proviso that at least one of X17 and X20 is K, and further provided that at least one of said K comprises a side chain amino (ε amino) group acylated with a moiety of the formula selected from:
   aminoethoxyethoxyacetic acid-Aib-Glu-C₁₆-C₂₂ fatty acid chain;
   aminoethoxyethoxyacetic acid-C(O)-diaminobutane-Glu-C₁₆-C₂₂ fatty acid chain; Glu-C₁₆-C₂₂ fatty acid chain;
   aminoethoxyethoxyacetic acid- aminoethoxyethoxyacetic acid-Glu-C₁₆-C₂₂ fatty acid chain; and
   aminoethoxyethoxyacetic acid-Glu-C₁₆-C₂₂ fatty acid chain.
   with a proviso that when X20 is Aib, the side chain amino group is not acylated with aminoethoxyethoxyacetic acid-aminoethoxyethoxyacetic acid-Glu-C₁₆-C₂₂ fatty acid chain or aminoethoxyethoxyacetic acid-Glu-C₁₆-C₂₂ fatty acid chain.
Embodiment 6. A polypeptide or a pharmaceutically acceptable salt thereof comprising the amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 3)
 wherein:
X1 is Y;
X2 is Aib;
X3 is Q or N;
X10 is Y;
X12 is I;
X13 is Aib, Ser(OMe), nor-V, nor-L, or αMe-L;
X16 is K;
X17 is I or K;
X18 is A;
X19 is Q;
X20 is K, Aib, Ser(OMe), nor-V, or nor-L;
X21 is A;
X23 is V or I;
X24 is Q or E;
X25 is W or Y;
X27 is E, I or L;
X28 is A, or E;
X29 is G;
X30 is G;
X31 is P;
X32 is S;
X33 is S;
X34 is G;
X35 is A;
X36 is P;
X37 is P;
X38 is P; and
X39 is S;
wherein the acid group of the C-terminal amino acid is a free carboxylic acid group or is amidated as C-terminal primary amide; and
with a proviso that at least one of, X17 and X20 is K, and further provided that at least one of said K comprises a side chain amino (ε amino) group acylated with a moiety of the formula selected from:

| | |
|---|---|
| Moiety A | 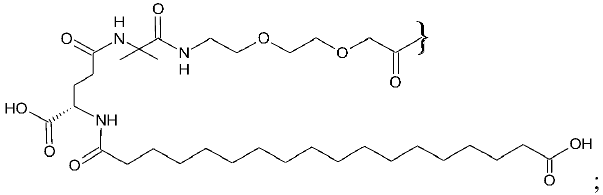 |
| Moiety B | 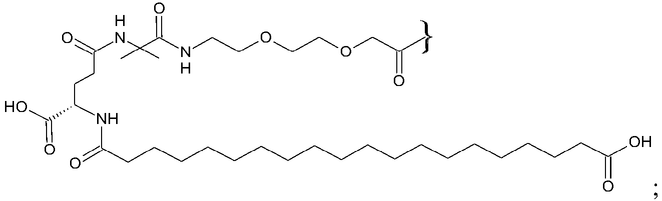 |
| Moiety C | 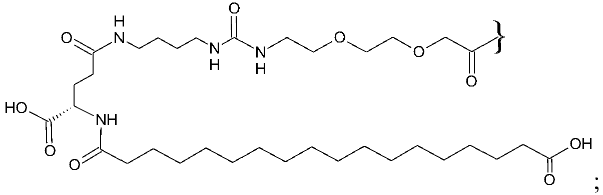 |
| Moiety D | 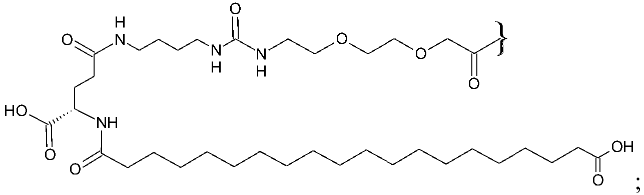 |
| Moiety E | 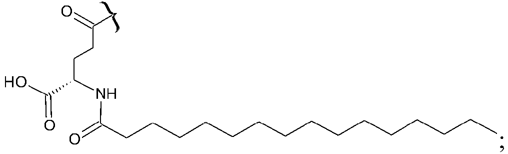 |
| Moiety F | 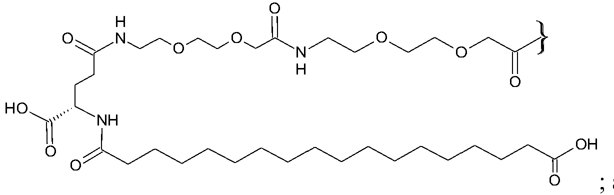 and |
| Moiety G | 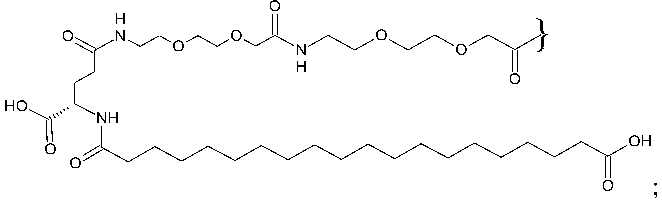 |
| Moiety H | 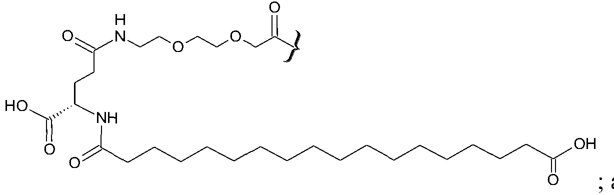 and |
| Moiety I | 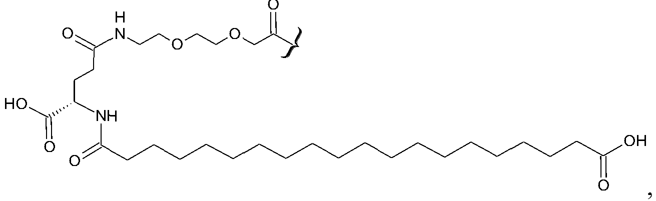 |

wherein the polypeptide is not SEQ ID NO: 7, SEQ ID NO: 23 or SEQ ID NO: 30.
Embodiment 7. The polypeptide according to embodiment 6, wherein:
X1 is Y;
X2 is Aib;
X3 is Q or N;
X10 is Y;
X12 is I;
X13 is nor-V, nor-L or αMe-L;
X16 is K;
X17 is I or K;
X18 is A;
X19 is Q;
X20 is K, Aib, nor-V or nor-L;
X21 is A;
X23 is V or I;
X24 is Q or E;
X25 is W or Y;
X27 is I or L;
X28 is A or E;
X29 is G;
X30 is G;
X31 is P;
X32 is S;
X33 is S;
X34 is G;
X35 is A;
each of X36, X37 and X38 is independently P; and
X39 is S;
wherein the side chain amino (ε amino) group of K at position X17 is acylated with a moiety of the formula selected from:

| | |
|---|---|
| Moiety A | 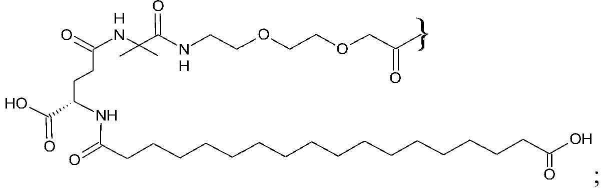 |
| Moiety B | 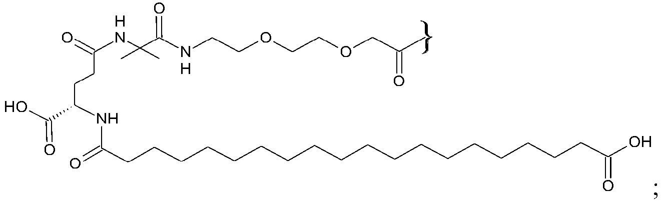 |
| Moiety C | 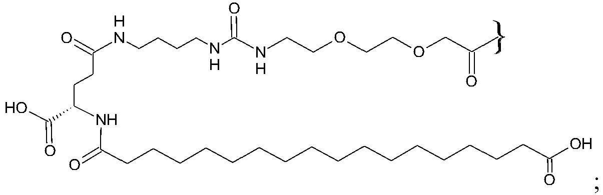 |
| Moiety D | 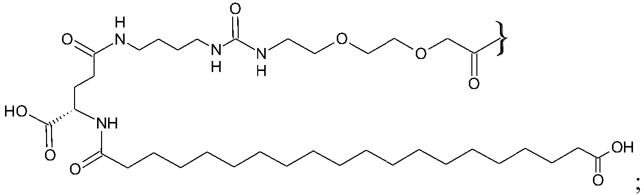 |
| Moiety H | 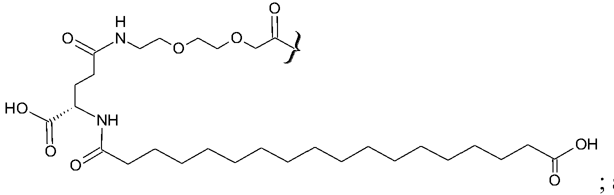 and |
| Moiety I | 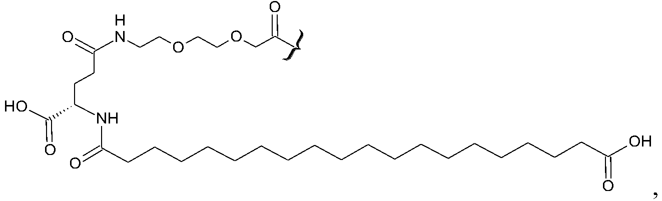 |

wherein the polypeptide is not SEQ ID NO: 7.
Embodiment 8. The polypeptide according to embodiment 6, wherein:
X1 is Y;
X2 is Aib;
X3 is Q or N;
X10 is Y;
X12 is I;
X13 is nor-V, nor-L or αMe-L;
X16 is K;
X17 is K;
X18 is A;
X19 is Q;
X20 is Aib, nor-V or nor-L;
X21 is A;
X23 is I;
X24 is E;
X25 is Y;
X27 is L;
X28 is E;
X29 is G;
X30 is G;
X31 is P;
X32 is S;
X33 is S;
X34 is G;
X35 is A;
each of X36, X37 and X38 is independently P; and
X39 is S;
wherein the side chain amino (ε amino) group of K at position X17 is acylated with a moiety of the formula selected from:

| | |
|---|---|
| Moiety A | 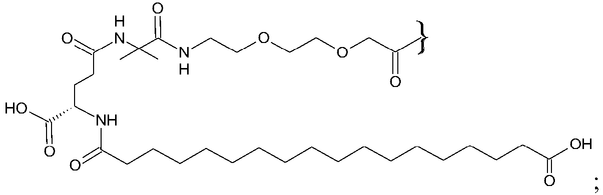 |
| Moiety B | 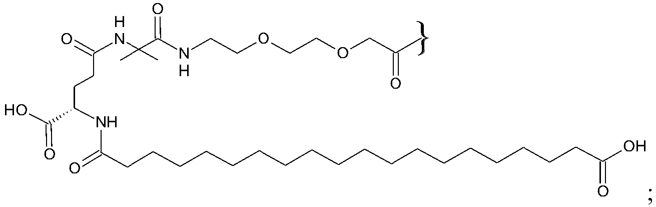 |
| Moiety C | 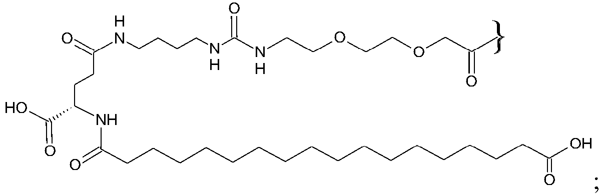 |
| Moiety D | 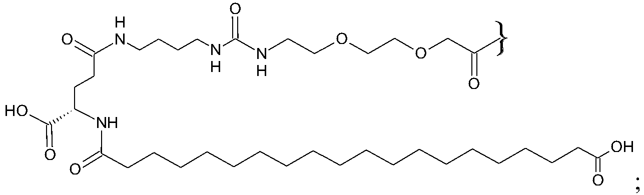 |
| Moiety H | 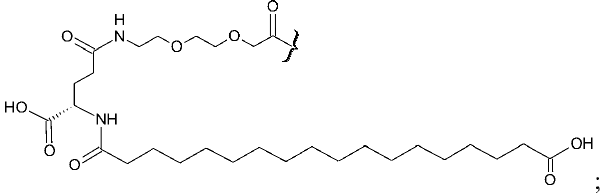 and |
| Moiety I | 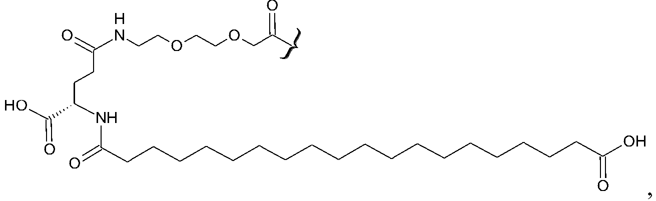 |

wherein the polypeptide is not SEQ ID NO: 7.
Embodiment 9. A polypeptide or a pharmaceutically acceptable salt thereof comprising an amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 4)
 wherein:
X1 is Y;
X2 is Aib;
X3 is Q or N;
X10 is Y;
X12 is I;
X13 is nor-V, nor-L or αMe-L;
X16 is K;
X17 is I or K;
X18 is A;
X19 is Q;
X20 is K or Aib;
X21 is A;
X23 is V or I;
X24 is Q or E;
X25 is W or Y;
X27 is I or L;
X28 is A or E;
X29 is G;
X30 is G;
X31 is P;
X32 is S;
X33 is S;
X34 is G;
X35 is A;
each of X36, X37 and X38 is independently P; and
X39 is S;
with a proviso that at least one of X17 and X20 is K, and further provided that said K comprises a side chain amino (ε amino) group acylated with a moiety of the formula selected from:

| | |
|---|---|
| Moiety A | 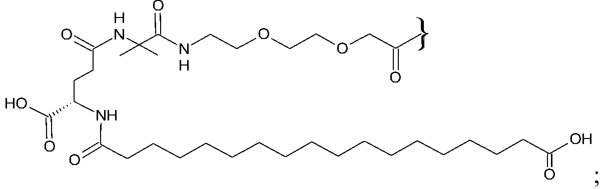 |
| Moiety B | 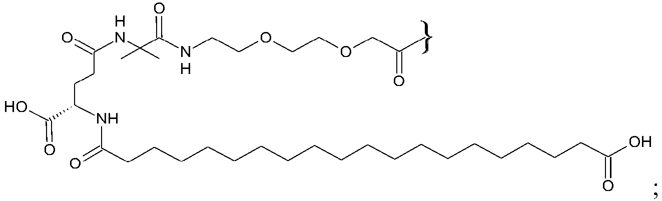 |
| Moiety C | 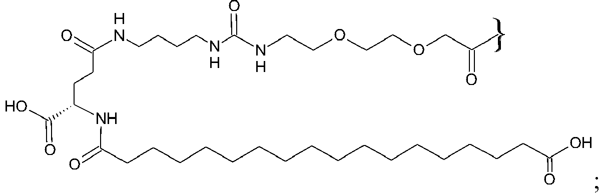 and |
| Moiety D | 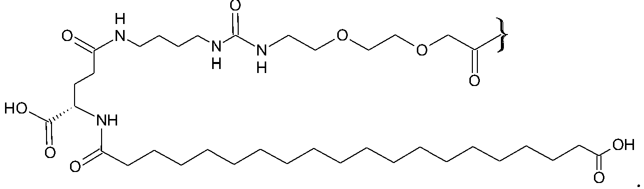 |

Embodiment 10. A polypeptide or a pharmaceutically acceptable salt thereof comprising an amino acid sequence: 
Y-Aib-X3-G-T-F-T-S-D-Y-S-I-X13-L-D-K-X17-A-Q-X20-A-F-X23-X24-X25-L-X27-
X28-G-G-P-S-S-G-A-P-P-P-S (SEQ ID NO: 5)
 wherein:
X3 is Q or N;
X13 is nor-V, nor-L or αMe-L;
X17 is I or K;
X20 is K or Aib;
X23 is V or I;
X24 is Q or E;
X25 is W or Y;
X27 is I or L; and
X28 is A or E;
wherein the acid group of the C-terminal amino acid is a free carboxylic acid group or is amidated as C-terminal primary amide; and
with a proviso that at least one of X17 and X20 is K, and further provided that said K comprises a side chain amino (ε amino) group acylated with a moiety of the formula selected from:

| | |
|---|---|
| Moiety A | 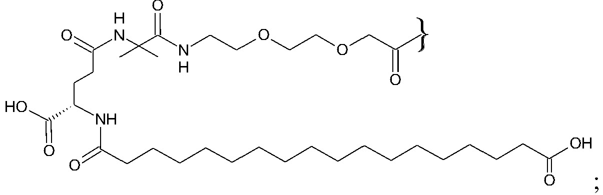 |
| Moiety B | 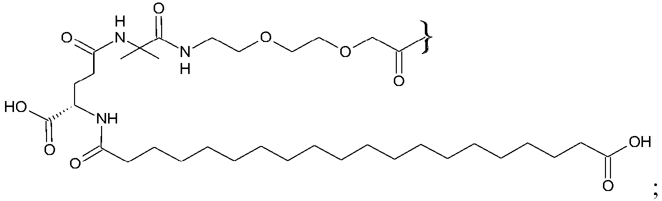 |
| Moiety C | 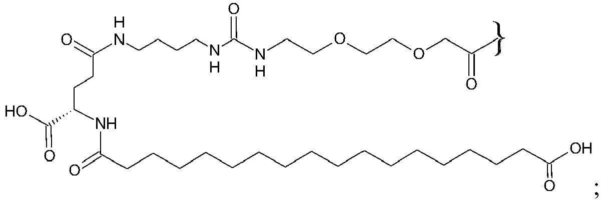 |
| Moiety D | 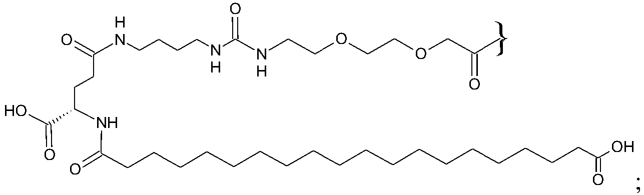 |
| Moiety G | 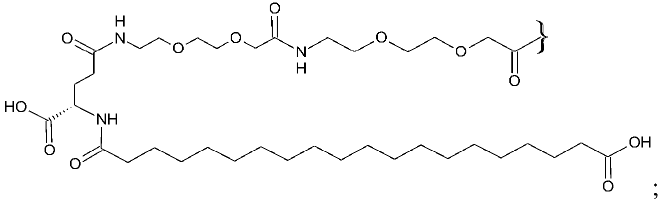 |
| Moiety H | 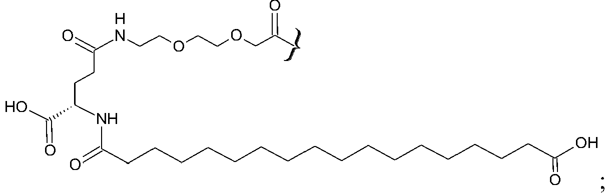 and |
| Moiety I | 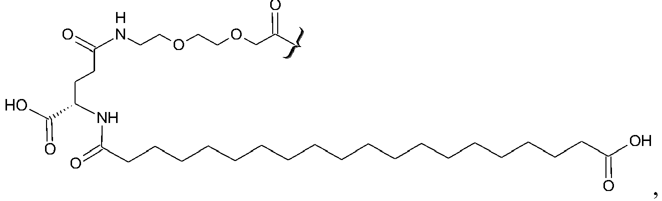 |

wherein the polypeptide is not SEQ ID NO: 7.
Embodiment 11. The polypeptide according to embodiment 10, wherein:
X3 is Q;
X13 is αMe-L;
X17 is K;
X20 is Aib;
X23 is I;
X24 is E;
X25 is Y;
X27 is L; and
X28 is E;
wherein the side chain amino (ε amino) group of K at position X17 is acylated with the moiety of formula selected from:

| | |
|---|---|
| Moiety A | 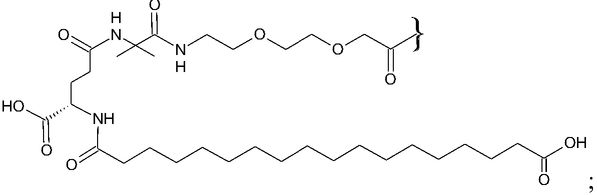 |
| Moiety B | 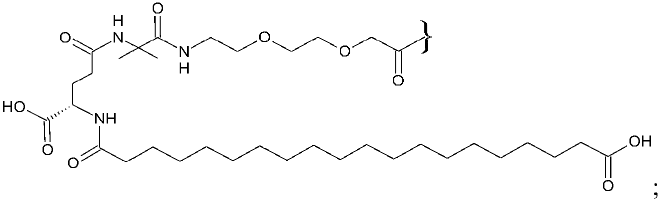 |
| Moiety C | 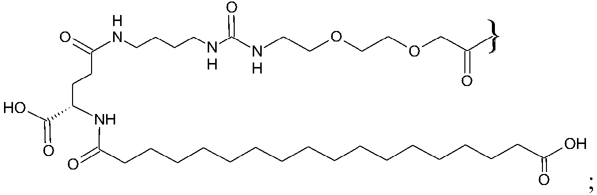 and |
| Moiety D | 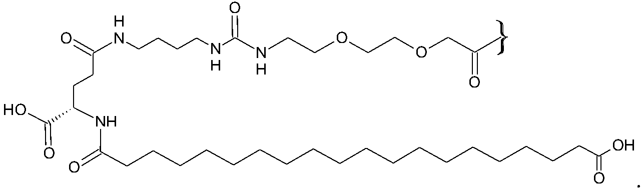 |

Embodiment 12. The polypeptide according to embodiment 10, wherein:
X3 is Q;
X13 is nor-V;
X17 is K;
X20 is Aib;
X23 is I;
X24 is E;
X25 is Y;
X27 is L; and
X28 is E;
wherein the side chain amino (ε amino) group of K at position X17 is acylated with the moiety of formula selected from:

| | |
|---|---|
| Moiety A | 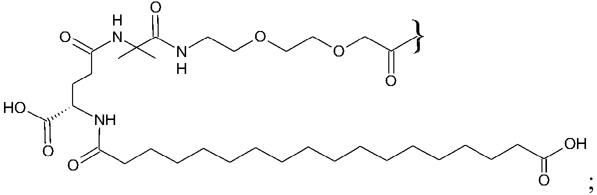 |
| Moiety B | 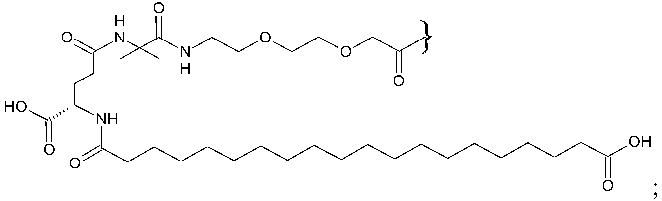 and |
| Moiety G | 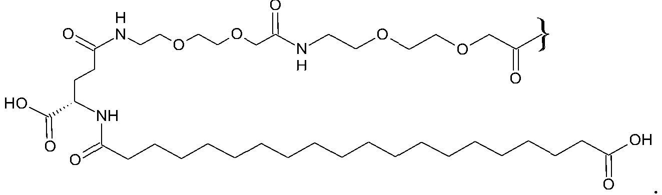 |

Embodiment 13. The polypeptide according to embodiment 10, wherein:
X3 is Q;
X13 is nor-L;
X17 is K;
X20 is Aib;
X23 is I;
X24 is E;
X25 is Y;
X27 is L; and
X28 is E;
wherein the side chain amino (ε amino) group of K at position X17 is acylated with a moiety of the formula selected from:

| | |
|---|---|
| Moiety A | 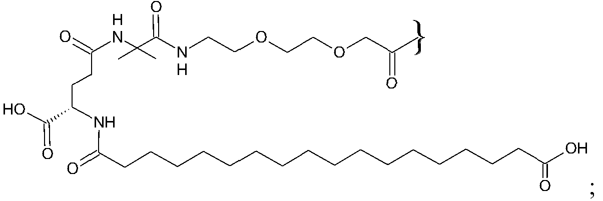 and |
| Moiety B | 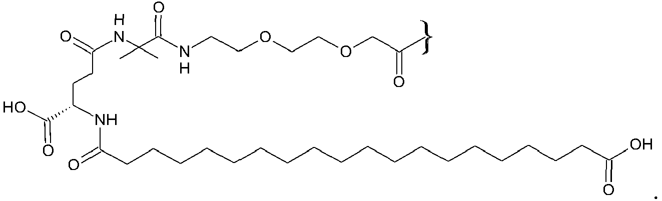 |

Embodiment 14. The polypeptide according to embodiment 10, wherein:
X3 is Q;
X13 is nor-V;
X17 is I;
X20 is K;
X23 is V;
X24 is Q;
X25 is W;
X27 is I; and
X28 is A;
wherein the side chain amino (ε amino) group of K at position X20 is acylated with a moiety of the formula selected from:

| | |
|---|---|
| Moiety A | 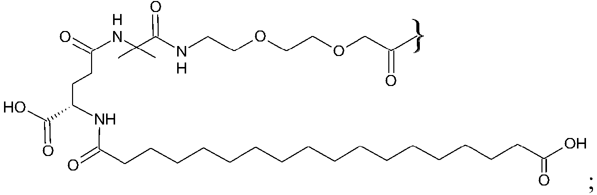 and |
| Moiety B | 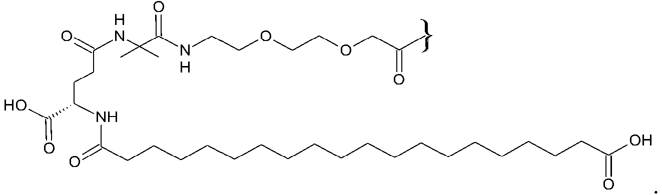 |

Embodiment 15. The polypeptide according to embodiment 10, wherein:
X3 is N;
X13 is nor-V;
X17 is I;
X20 is K;
X23 is V;
X24 is Q;
X25 is W;
X27 is I; and
X28 is A;
wherein the side chain amino (ε amino) group of K at position X20 is acylated with a moiety of the formula selected from:

| | |
|---|---|
| Moiety A | 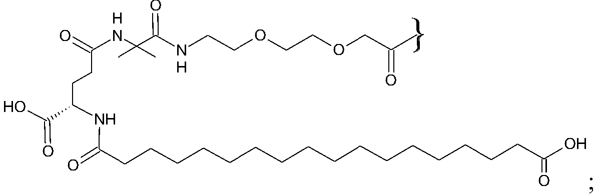 and |
| Moiety B | 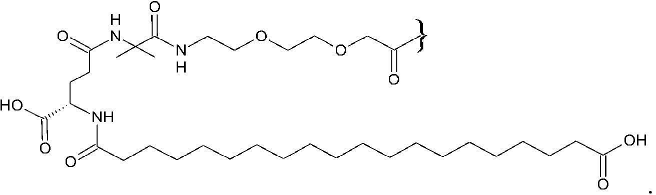 |

Embodiment 16. A polypeptide or a pharmaceutically acceptable salt thereof, comprising the amino acid sequence: 
Y-Aib-X3-G-T-F-T-S-D-Y-S-I-X13-L-D-K-X17-A-Q-X20-A-F-I-E-Y-L-L-E-G-G-P-S-
S-G-A-P-P-P-S (SEQ ID NO: 6)
 wherein:
X3 is Q or N;
X13 is nor-V, nor-L or αMe-L;
X17 is K; and
X20 is Aib, nor-L or nor-V;
wherein the acid group of the C-terminal amino acid is a free carboxylic acid group or is amidated as C-terminal primary amide; and
wherein the side chain amino (ε amino) group of K at position X17 is acylated with a moiety of the formula selected from:

| | |
|---|---|
| Moiety A | 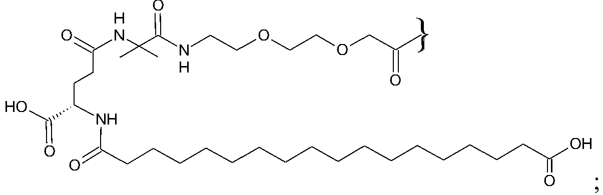 |
| Moiety H | 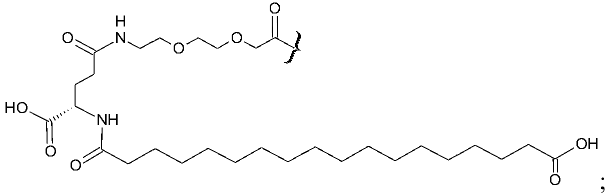 and |
| Moiety I | 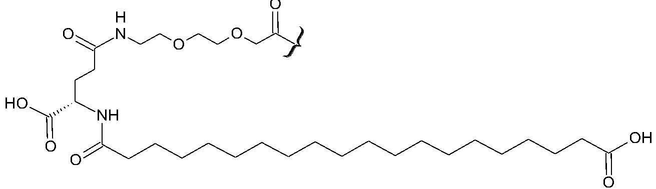 |

wherein the polypeptide is not SEQ ID NO: 7.
Embodiment 17. The polypeptide according to embodiment 16, wherein:
X13 is nor-V or nor-L; and
X20 is Aib;
wherein the side chain amino (ε amino) group of K at position X17 is acylated with a moiety of the formula selected from:

| | |
|---|---|
| Moiety H | 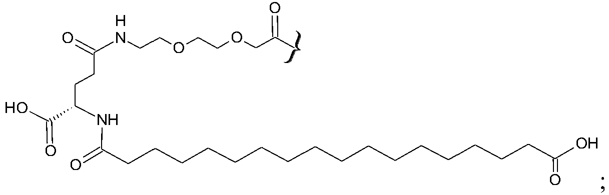 and |
| Moiety I | 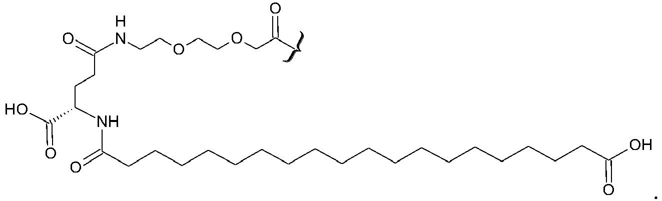 |

Embodiment 18. The polypeptide according to embodiment 16, wherein:
X13 is αMe-L; and
X20 is nor-L or nor-V;
wherein the side chain amino (ε amino) group of K at position X17 is acylated with a moiety of the formula selected from:

| | |
|---|---|
| Moiety A | 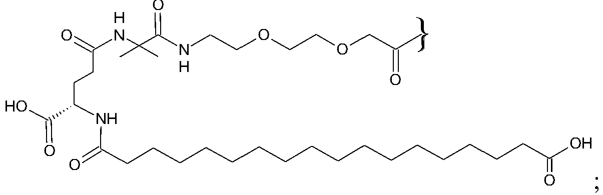 and |
| Moiety I | 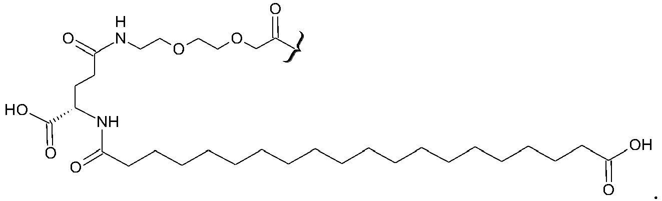 |

Embodiment 19. An incretin analog comprising:
a lysine residue comprising a fatty acid protracting group attached to the lysine ε-nitrogen;
a peptide residue comprising the sequence Gly-Thr-Phe-Thr-Ser-Asp (SEQ ID NO:31) attached indirectly via its carboxy terminus to the lysine residue;
a peptide residue having the sequence Gly-Gly-Pro-Ser-Ser-Gly-Ala-Pro-Pro-Pro-Ser-CONH₂ (SEQ ID NO:32) indirectly attached to the carboxy of the lysine via the amino terminus of the Gly-Gly-Pro-Ser-Ser-Gly-Ala-Pro-Pro-Pro-Ser-CONH₂ (SEQ ID NO:32) residue; and
a norvaline amino acid residue attached indirectly to and between the Gly-Thr-Phe-Thr-Ser-Asp (SEQ ID NO:31) residue and the lysine residue.
Embodiment 20. An incretin analog comprising
a lysine residue comprising a group of formula (I) attached to the lysine ε-nitrogen, wherein formula (I) is 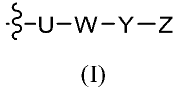 wherein
   U is absent or represents -C(O)-CH₂-O-(CH₂)₂-O-(CH₂)₂-NH-}, wherein } is point of attachment to W;
   W represents:

      -C(O)-CH₂-O-(CH₂)₂-O-(CH₂)₂-NH-],

      -C(O)-NH-(CH₂)₃₋₄-NH-], -C(O)-C(CH₃)₂-NH-],

      or 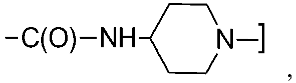 wherein ] is point of attachment to Y;
   Y is absent or represents -C(O)-(CH₂)₂-CH(CO₂H)NH--
      or -C(O)CH((CH₂)ₓCO₂H)NH--, wherein x is 1, 2 or 3, and -- is point of attachment to Z; and
   Z represents -C(O)-(CH₂)ₙ-COOH or -C(O)-(CH₂)ₙ-CH₃ wherein n is an integer from 14-20;
a peptide residue comprising the sequence Gly-Thr-Phe-Thr-Ser-Asp (SEQ ID NO:31) attached indirectly via its carboxy terminus to the lysine residue;
a peptide residue having the sequence Gly-Gly-Pro-Ser-Ser-Gly-Ala-Pro-Pro-Pro-Ser-NH₂ (SEQ ID NO:32) indirectly attached to the carboxy of the lysine via the amino terminus of the Gly-Gly-Pro-Ser-Ser-Gly-Ala-Pro-Pro-Pro-Ser-CONH₂ (SEQ ID NO:32) residue; and
a norvaline amino acid residue attached indirectly to and between the Gly-Thr-Phe-Thr-Ser-Asp (SEQ ID NO:31) residue and the lysine residue.
Embodiment 21. A polypeptide or an incretin analog selected from:

| Compound No. | Structure | SEQ ID NO |
|---|---|---|
| 12 | 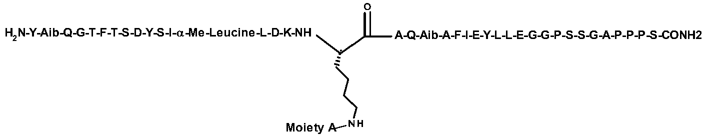 | 17 |
| 13 | 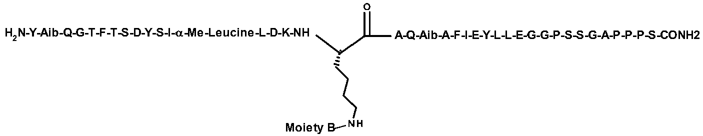 | 18 |
| 14 | 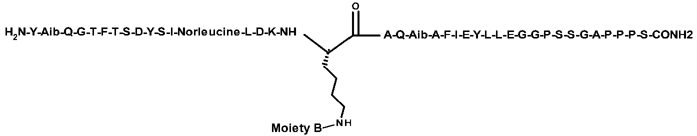 | 19 |
| 15 | 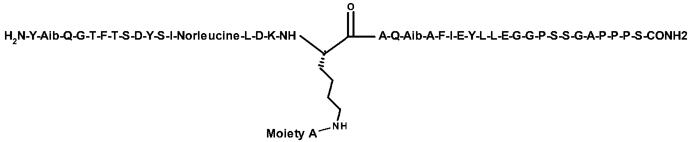 | 20 |
| 16 | 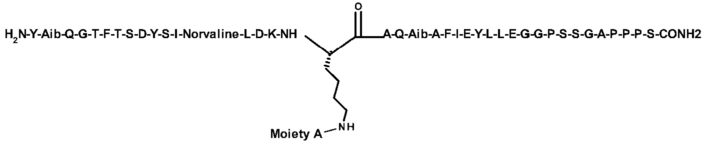 | 21 |
| 17 | 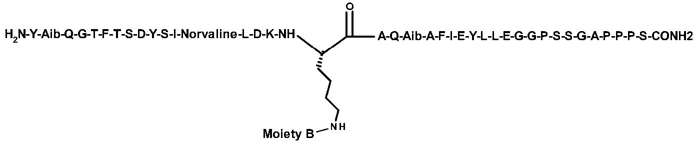 | 22 |
| 18 | 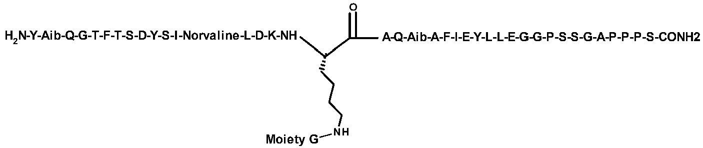 | 23 |
| 19 | 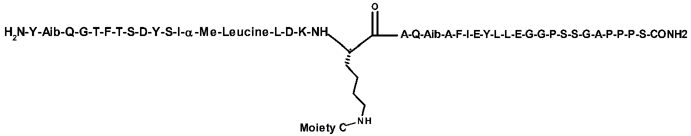 | 24 |
| 20 | 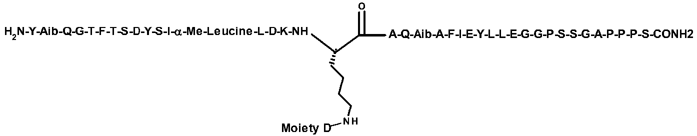 | 25 |
| 21 | 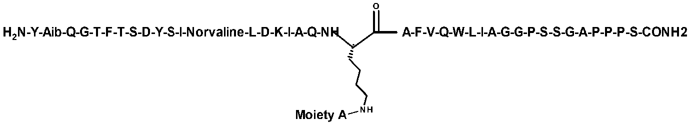 | 26 |
| 22 | 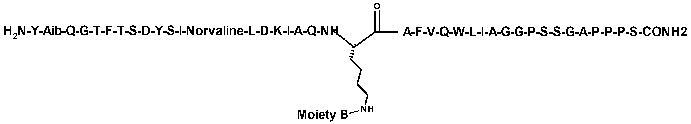 | 27 |
| 23 | 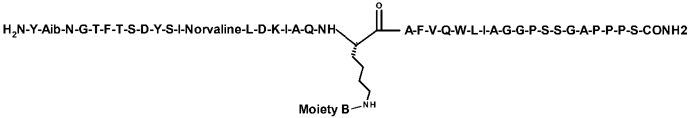 | 28 |
| 24 | 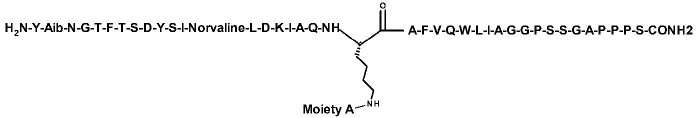 | 29 |

Embodiment 22. An incretin analog according to embodiment 19 or embodiment 20, wherein the lysine is attached to the Gly-Thr-Phe-Thr-Ser-Asp (SEQ ID NO:31) residue by a peptide residue comprising 10 amino acids.
Embodiment 23. An incretin analog according to embodiment 19 or embodiment 20, wherein the lysine is attached to the Gly-Gly-Pro-Ser-Ser-Gly-Ala-Pro-Pro-Pro-Ser-CONH₂ (SEQ ID NO:32) residue by a peptide residue comprising 11 amino acids.
Embodiment 24. A pharmaceutical composition comprising a pharmaceutically acceptable carrier and a polypeptide or incretin analog of any one of embodiments 1-22.
Embodiment 25. A method of treating obesity comprising administering to a patient in need of such treatment a polypeptide or incretin analog of any one of embodiments 1-22.
Embodiment 26. A method of treating type 2 diabetes mellitus (T2DM) comprising administering to a patient in need of such treatment a polypeptide or incretin analog of any one of embodiments 1-22.
Embodiment 27. A method of treating metabolic syndrome comprising administering to a patient in need of such treatment a polypeptide or incretin analog of any one of embodiments 1-22.
Embodiment 28. A method of treating metabolic dysfunction-associated steatotic liver disease (MASLD) comprising administering to a patient in need of such treatment a polypeptide or incretin analog of any one of embodiments 1-22.
Embodiment 29. A method of treating metabolic dysfunction-associated steatohepatitis (MASH) comprising administering to a patient in need of such treatment a polypeptide or incretin analog of any one of embodiments 1-22.
Embodiment 30. A method of treating neurodegenerative disorders comprising administering to a patient in need of such treatment a polypeptide or incretin analog of any one of embodiments 1-22.
Embodiment 31. A method of treating fibrosis comprising administering to a patient in need of such treatment a polypeptide or incretin analog of any one of embodiments 1-22.
Embodiment 32. A method of reducing cardiovascular risks comprising administering to a patient in need of such treatment a polypeptide or incretin analog of any one of embodiments 1-22.
Embodiment 33. A method of treating hyperlipidemia/dyslipidemia comprising administering to a patient in need of such treatment a polypeptide or incretin analog of any one of embodiments 1-22.

## Claims

1. A polypeptide or a pharmaceutically acceptable salt thereof comprising the amino acid sequence: 
X1-X2-X3-G-T-F-T-S-D-X10-S-X12-X13-L-D-X16-X17-X18-X19-X20-X21-F-X23-
X24-X25-L-X27-X28-X29-X30-X31-X32-X33-X34-X35-X36-X37-X38-X39 (SEQ ID
NO: 3)
 wherein:
X1 is Y;
X2 is Aib;
X3 is Q or N;
X10 is Y;
X12 is I;
X13 is Aib, Ser(OMe), nor-V, nor-L, or αMe-L;
X16 is K;
X17 is I or K;
X18 is A;
X19 is Q;
X20 is K, Aib, Ser(OMe), nor-V, or nor-L;
X21 is A;
X23 is V or I;
X24 is Q or E;
X25 is W or Y;
X27 is E, I or L;
X28 is A, or E;
X29 is G;
X30 is G;
X31 is P;
X32 is S;
X33 is S;
X34 is G;
X35 is A;
X36 is P;
X37 is P;
X38 is P; and
X39 is S;
wherein the acid group of the C-terminal amino acid is a free carboxylic acid group or is amidated as C-terminal primary amide; and
with a proviso that at least one of, X17 and X20 is K, and further provided that at least one of said K comprises a side chain amino (ε amino) group acylated with a moiety of the formula selected from:
| | |
|---|---|
| Moiety A | 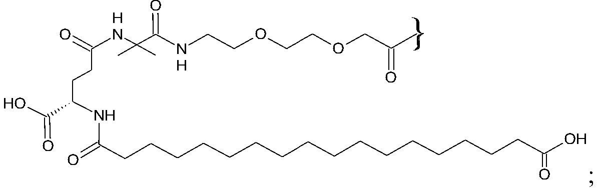 |
| Moiety B | 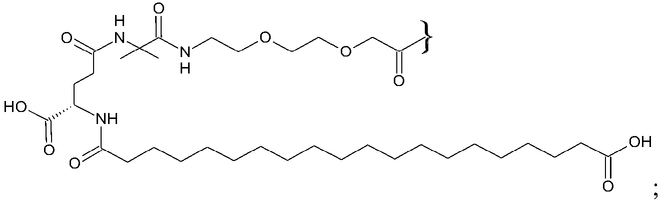 |
| Moiety C | 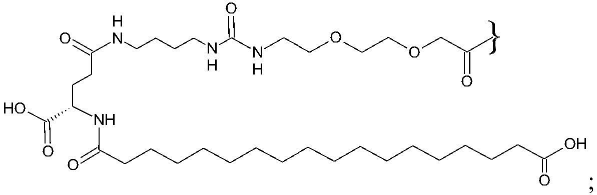 |
| Moiety D | 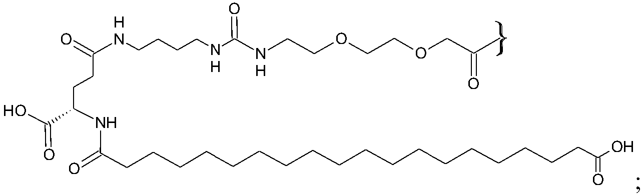 |
| Moiety F | 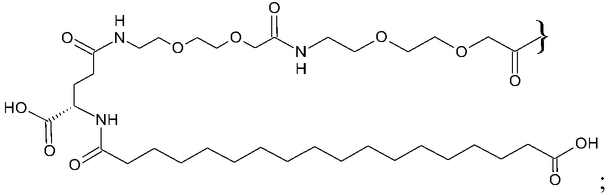 and |
| Moiety G | 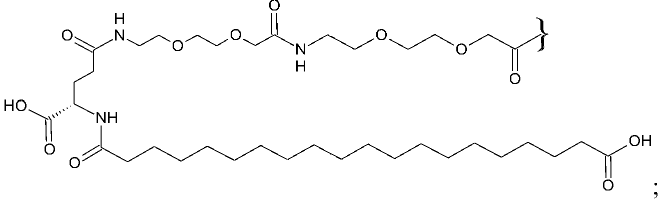 |
wherein the polypeptide is not SEQ ID NO: 30.

2. The polypeptide according to claim 1, wherein:
X1 is Y;
X2 is Aib;
X3 is Q or N;
X10 is Y;
X12 is I;
X13 is nor-V, nor-L or αMe-L;
X16 is K;
X17 is I or K;
X18 is A;
X19 is Q;
X20 is K, Aib, nor-V or nor-L;
X21 is A;
X23 is V or I;
X24 is Q or E;
X25 is W or Y;
X27 is I or L;
X28 is A or E;
X29 is G;
X30 is G;
X31 is P;
X32 is S;
X33 is S;
X34 is G;
X35 is A;
each of X36, X37 and X38 is independently P; and
X39 is S;
wherein the side chain amino (ε amino) group of K at position X17 is acylated with a moiety of the formula selected from:
| | |
|---|---|
| Moiety A | 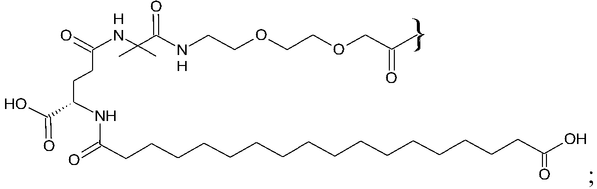 |
| Moiety B | 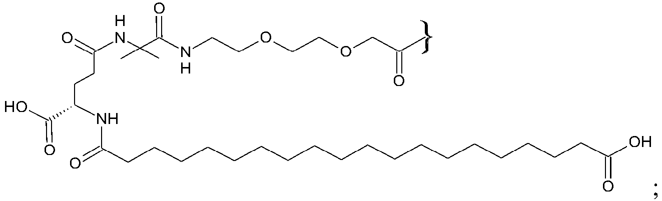 |
| Moiety C | 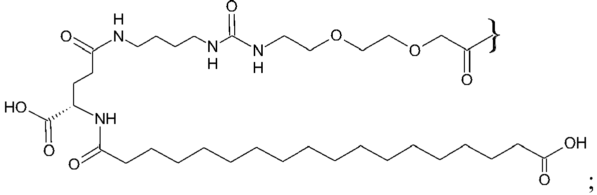 and |
| Moiety D | 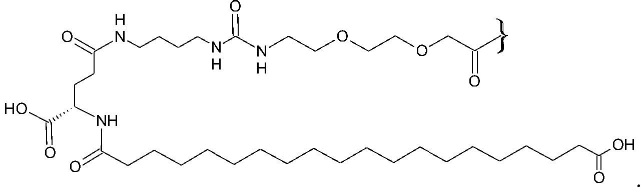 |

3. The polypeptide according to claim 1, wherein:
X1 is Y;
X2 is Aib;
X3 is Q or N;
X10 is Y;
X12 is I;
X13 is nor-V, nor-L or αMe-L;
X16 is K;
X17 is K;
X18 is A;
X19 is Q;
X20 is Aib, nor-V or nor-L;
X21 is A;
X23 is I;
X24 is E;
X25 is Y;
X27 is L;
X28 is E;
X29 is G;
X30 is G;
X31 is P;
X32 is S;
X33 is S;
X34 is G;
X35 is A;
each of X36, X37 and X38 is independently P; and
X39 is S;
wherein the side chain amino (ε amino) group of K at position X17 is acylated with a moiety of the formula selected from:
| | |
|---|---|
| Moiety A | 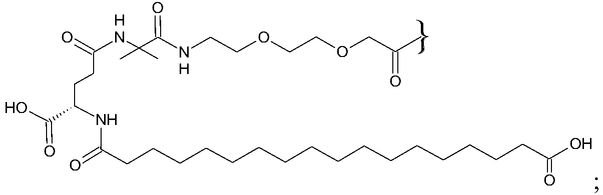 |
| Moiety B | 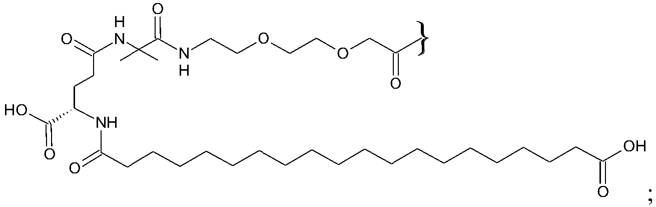 |
| Moiety C | 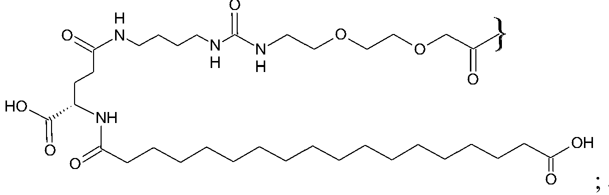 and |
| Moiety D | 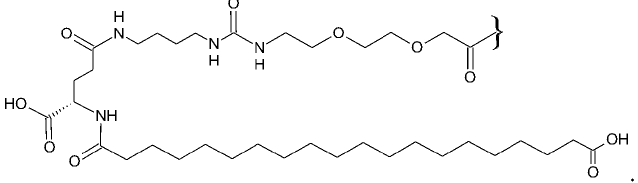 |

4. The polypeptide according to claim 1 comprising an amino acid sequence: 
Y-Aib-X3-G-T-F-T-S-D-Y-S-I-X13-L-D-K-X17-A-Q-X20-A-F-X23-X24-X25-L-X27-
X28-G-G-P-S-S-G-A-P-P-P-S (SEQ ID NO: 5),
 wherein:
X3 is Q or N;
X13 is nor-V, nor-L or αMe-L;
X17 is I or K;
X20 is K or Aib;
X23 is V or I;
X24 is Q or E;
X25 is W or Y;
X27 is I or L; and
X28 is A or E;
wherein the acid group of the C-terminal amino acid is a free carboxylic acid group or is amidated as C-terminal primary amide; and
with a proviso that at least one of X17 and X20 is K, and further provided that said K comprises a side chain amino (ε amino) group acylated with a moiety of the formula selected from:
| | |
|---|---|
| Moiety A | 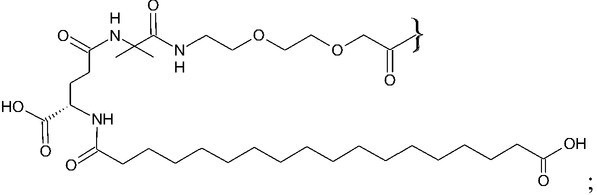 |
| Moiety B | 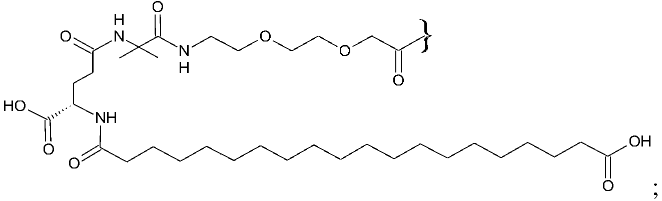 |
| Moiety C | 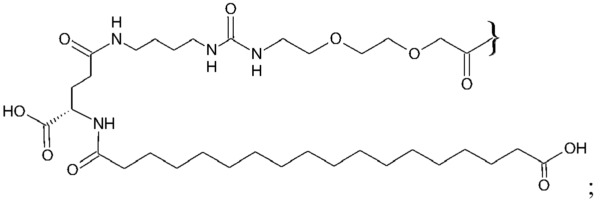 |
| Moiety D | 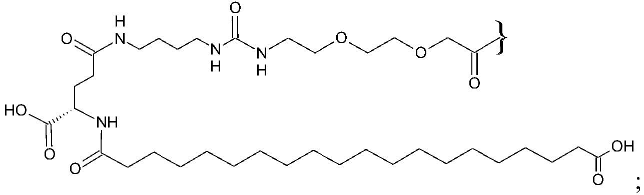 and |
| Moiety G | 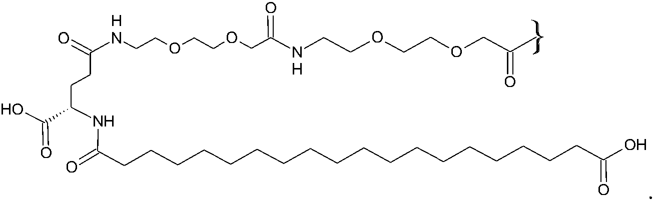 |

5. The polypeptide according to claim 4, wherein:
X3 is Q;
X13 is αMe-L;
X17 is K;
X20 is Aib;
X23 is I;
X24 is E;
X25 is Y;
X27 is L; and
X28 is E;
wherein the side chain amino (ε amino) group of K at position X17 is acylated with the moiety of formula selected from:
| | |
|---|---|
| Moiety A | 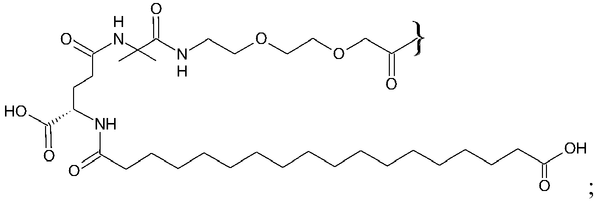 |
| Moiety B | 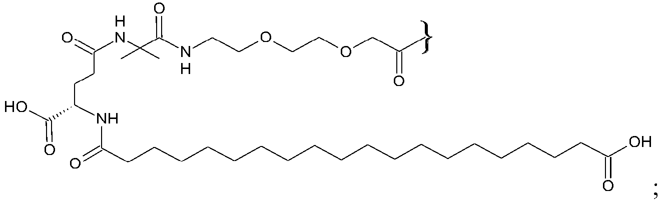 |
| Moiety C | 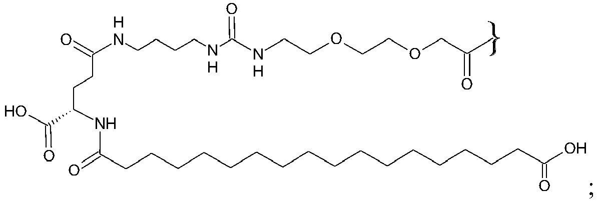 and |
| Moiety D | 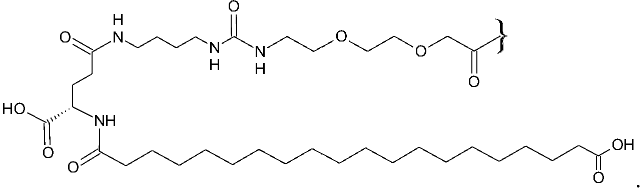 |

6. The polypeptide according to claim 4, wherein:
X3 is Q;
X13 is nor-V;
X17 is K;
X20 is Aib;
X23 is I;
X24 is E;
X25 is Y;
X27 is L; and
X28 is E;
wherein the side chain amino (ε amino) group of K at position X17 is acylated with the moiety of formula selected from:
| | |
|---|---|
| Moiety A | 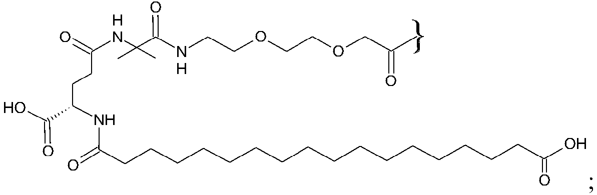 |
| Moiety B | 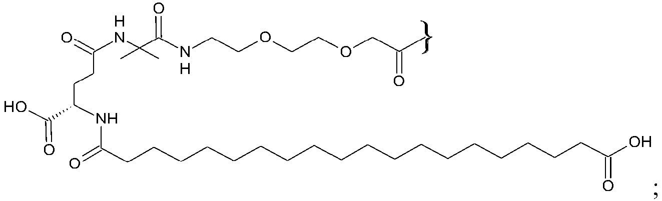 and |
| Moiety G | 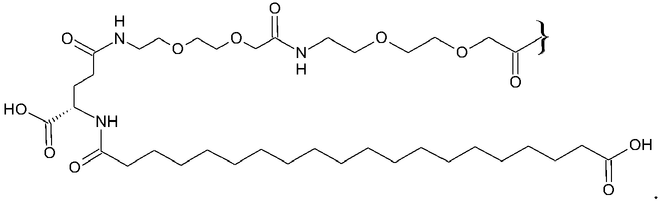 |

7. The polypeptide according to claim 4, wherein:
X3 is Q;
X13 is nor-L;
X17 is K;
X20 is Aib;
X23 is I;
X24 is E;
X25 is Y;
X27 is L; and
X28 is E;
wherein the side chain amino (ε amino) group of K at position X17 is acylated with a moiety of the formula selected from:
| | |
|---|---|
| Moiety A | 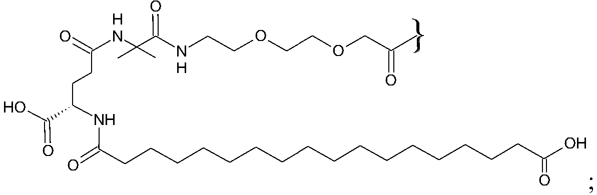 and |
| Moiety B | 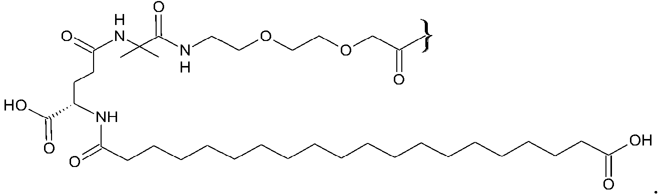 |

8. The polypeptide according to claim 4, wherein:
X3 is Q;
X13 is nor-V;
X17 is I;
X20 is K;
X23 is V;
X24 is Q;
X25 is W;
X27 is I; and
X28 is A;
wherein the side chain amino (ε amino) group of K at position X20 is acylated with a moiety of the formula selected from:
| | |
|---|---|
| Moiety A | 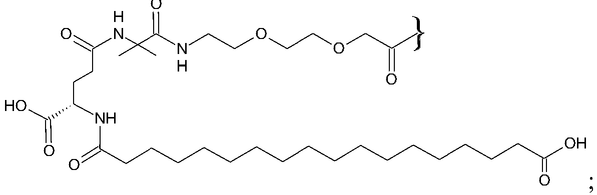 and |
| Moiety B | 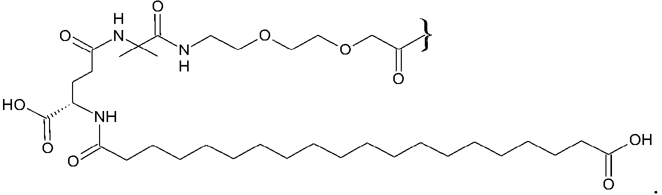 |

9. The polypeptide according to claim 4, wherein:
X3 is N;
X13 is nor-V;
X17 is I;
X20 is K;
X23 is V;
X24 is Q;
X25 is W;
X27 is I; and
X28 is A;
wherein the side chain amino (ε amino) group of K at position X20 is acylated with a moiety of the formula selected from:
| | |
|---|---|
| Moiety A | 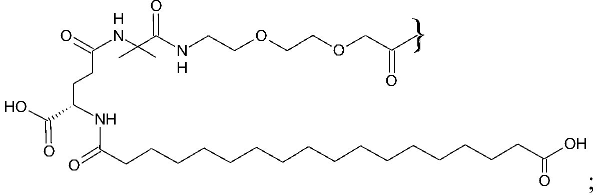 and |
| Moiety B | 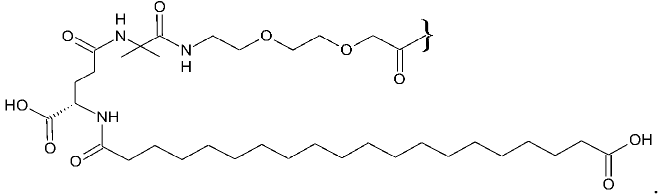 |

10. The polypeptide according to claim 1, comprising the amino acid sequence: 
Y-Aib-X3-G-T-F-T-S-D-Y-S-I-X13-L-D-K-X17-A-Q-X20-A-F-I-E-Y-L-L-E-G-G-P-S-
S-G-A-P-P-P-S (SEQ ID NO: 6)
 wherein:
X3 is Q or N;
X13 is nor-V, nor-L or αMe-L;
X17 is K; and
X20 is Aib, nor-L or nor-V;
wherein the acid group of the C-terminal amino acid is a free carboxylic acid group or is amidated as C-terminal primary amide; and
wherein the side chain amino (ε amino) group of K at position X17 is acylated with a moiety of the formula selected from:
| | |
|---|---|
| Moiety A | 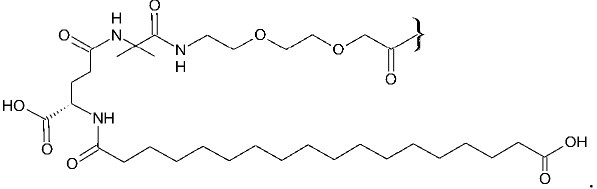 |

11. The polypeptide according to claim 10, wherein:
X13 is αMe-L; and
X20 is nor-L or nor-V;
wherein the side chain amino (ε amino) group of K at position X17 is acylated with a moiety of the formula selected from:
| | |
|---|---|
| Moiety A | 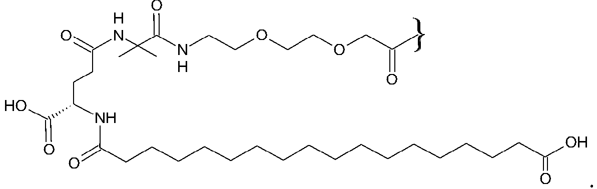 |

12. The polypeptide according to claim 1, selected from:
| Compound No. | Structure | SEQ ID NO |
|---|---|---|
| 12 | 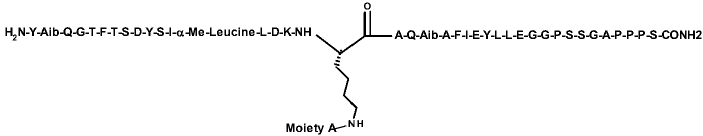 | 17 |
| 13 | 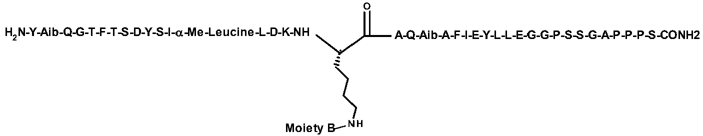 | 18 |
| 14 | 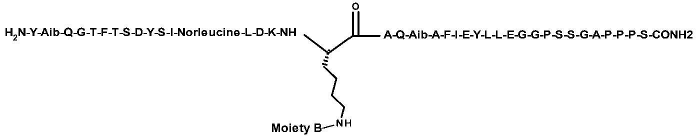 | 19 |
| 15 | 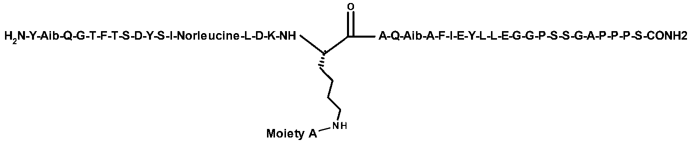 | 20 |
| 16 | 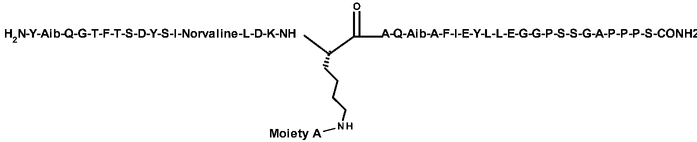 | 21 |
| 17 | 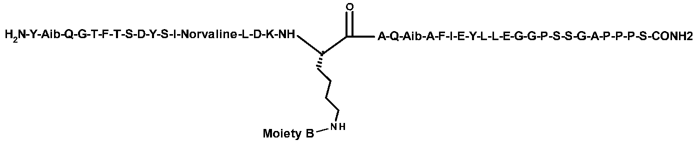 | 22 |
| 18 | 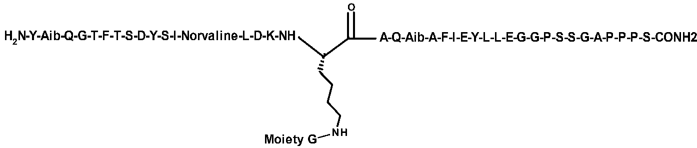 | 23 |
| 19 | 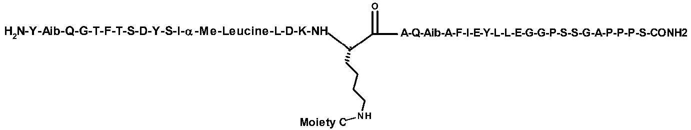 | 24 |
| 20 | 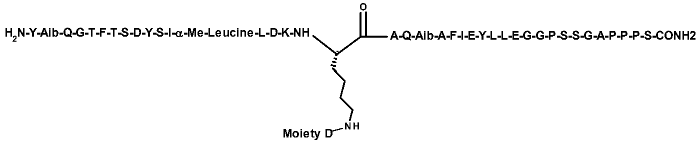 | 25 |
| 21 | 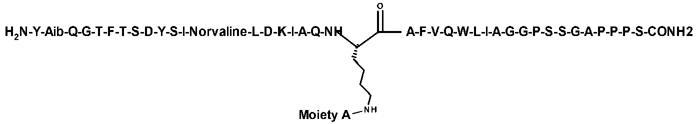 | 26 |
| 22 | 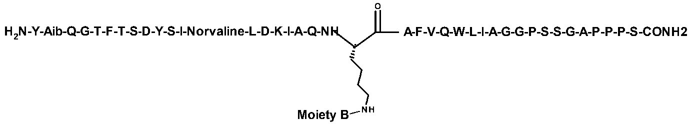 | 27 |
| 23 | 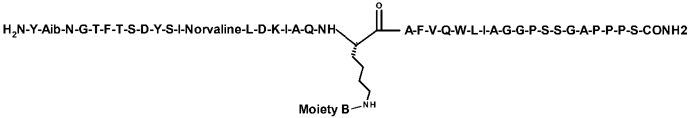 | 28 |
| 24 | 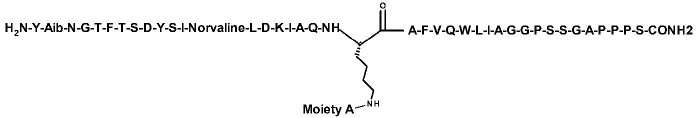 | 29 |

13. A pharmaceutical composition comprising a pharmaceutically acceptable carrier and the polypeptide of any one of claims 1-12.

14. A polypeptide according to any one of claims 1-12 or a pharmaceutical composition according to claim 13, for use in the treatment of obesity, type 2 diabetes mellitus (T2DM), metabolic syndrome, metabolic dysfunction-associated steatotic liver disease (MASLD), metabolic dysfunction-associated steatohepatitis (MASH), neurodegenerative disorders, fibrosis, cardiovascular risks or hyperlipidemia/dyslipidemia, wherein the method comprises administering to a patient in need of such treatment the polypeptide or a pharmaceutically acceptable salt thereof or the pharmaceutical composition.

15. A polypeptide according to any one of claims 1-12 or a pharmaceutical composition according to claim 13, for use as a medicament.
